# EUROPEAN PATENT APPLICATION

(11) **EP 4 361 271 A2**
(43) Date of publication of application: **01.05.2024**
(21) Application number: 24159628.7
(22) Date of filing: 14.12.2021
(51) Int. Cl.: C12N 15/115

(54) **APTAMER-SIRNA FUSIONS**

(30) Priority: 14.12.2020 GB 202019692
(62) Divisional of application: 21839437.7
(71) Applicant: Apterna Limited, London SW1P 2PN (GB)
(72) Inventor: HABIB, Nagy, London, W5 1TA (GB); VASCONCELOS, Luis, 4300-362 Porto (PT)
(74) Representative: Mewburn Ellis LLP

(57) **Abstract**

Provided herein are ribonucleic acid compounds comprising an aptamer capable of binding to transferrin receptor (TfR), and a CCAAT/enhancer-binding protein β (C/EBPβ) siRNA. Also provided are pharmaceutical compositions comprising the ribonucleic acid compounds. The compounds and 5 pharmaceutical compositions find use in treating or preventing diseases or disorders, in particular cancers. The compounds also find use in delivering C/EBPβ siRNA to a cell.

## Description

### Sequence Listing

The instant application contains a Sequence Listing which has been submitted electronically in ASCII format and is hereby incorporated by reference in its entirety. Said ASCII copy, created on December 13, 2021, is named 008124653_SL.txt and is 51,695 bytes in size.

### Field of the Invention

The present invention relates to aptamers conjugated to small interfering RNA (siRNAs) and particularly, although not exclusively, to a nucleic acid capable of binding to transferrin receptor (TfR) conjugated to a siRNA targeting CCAAT/enhancer-binding protein β (C/EBPβ) . The present invention also relates to the use of said nucleic acids in the treatment of cancer, and in particular pancreatic cancer.

### Background

Pancreatic ductal adenocarcinoma (PDAC) is one of the most aggressive malignant tumours with limited therapeutic efficacy and high mortality rates (Huguet et al 2009, Shaib et al 2006) . Currently, first approach to cure PDAC can be achieved through surgical resection, however, the majority of PDAC patients are diagnosed in metastatic stages which is surgically unresectable. This type of advanced PDAC is a main causality of death in PDAC. PDAC preferentially metastasizes to the liver, which is the main cause of mortality related to advanced pancreatic cancer (Houg & Bijlsma, 2018)
The current standard treatments of advanced PDAC are limited to mono-chemotherapy; gemcitabine or combinational chemotherapy; gemcitabine combined with other chemotherapeutic agents such as 5FU, erlotinib, cisplatin, capecitabine, docetaxel, and oxaliplatin (Mohammad, 2018) . However, combination chemotherapy does not show significantly statistical survival benefits for PDAC (Paulson et al 2013, hereby incorporated by reference in its entirety) , and induction of chemotherapy prior to treatment with radiation with dose escalation results in severe side effects and the quality of life was not improved (Ma et al, 2018, hereby incorporated by reference in its entirety) . To reduce side effects on healthy tissues, targeted delivery is preferred in the development of cancer therapeutics.

Targeted delivery of cancer therapeutics consists of two main approaches; passive targeting or active targeting. As passive targeting is solely depending on the enhanced permeability and retention (EPR) effects, less than 1 % accumulates in xenografted tumour (Rosenblum et al, 2018, hereby incorporated by reference in its entirety) . For specific homing and improving tumour localization by increasing targeting efficacy and retention at the target site throughout the uptake by target cells, the active targeting utilizes affinity ligands such as antibodies or aptamers.

Aptamers, sometimes described as chemical antibodies, are small single stranded RNA or DNA molecules that bind to their target through shape recognition (Stottenburg R et al., Biomol Eng. 2007 Oct;24(4) , hereby incorporated by reference in its entirety) :381-403) . Aptamers comprise unique three-dimensional structures that are capable of specific molecular recognition of their cognate targets, and they display a number of advantages over antibodies, including their size, production process, increased stability and lack of immunogenicity.

The transferrin receptor (TfR) is a membrane glycoprotein expressed on the cellular surface which mediates cellular uptake of iron from the plasma glycoprotein transferrin. Transferrin binds to iron to create transferrin-iron complexes (Crichton & Charloteaux-Wauters, Eur J Biochem 1987;164(3) :485-506) . These complexes bind to TfR and bound transferrin is internalised into cells via receptor-mediated endocytosis (Qian ZM et al., Pharmacol Rev. 2002, 54(4) :561-587, hereby incorporated by reference in its entirety) . Transferrin and iron are subsequently released in endosomes. TfR is typically expressed at low levels on a range of normal cells and is highly expressed on cells with high proliferation rates, including cancer cells (Daniels TR et al., Clin Immunol 2006 121: 144-158; Daniels TR et al., Clin Immunol 2006 121: 159-176, hereby incorporated by reference in its entirety) . Thus, compounds capable of binding to TfR on the surface of TfR-expressing cells and internalising into the cell would be useful for targeted delivery of such compounds. Aptamers capable of binding TfR have been described in WO 2016/061386 and WO2019/033051, each hereby incorporated by reference in its entirety.

Aptamers that bind TfR could find use in providing targeted delivery of therapeutic payloads to cells throughout the body. Further mechanisms for targeted therapeutic delivery are needed.

The present invention has been devised in light of the above considerations.

### Summary of the Invention

The invention relates to ribonucleic acid compounds comprising a targeting moiety, especially a cancer and/or liver targeting moiety, and a CCAAT/enhancer-binding protein β (C/EBPβ) siRNA.

In a first aspect, the invention relates to a ribonucleic acid compound, comprising: (i) an aptamer capable of binding to transferrin receptor (TfR) , and (ii) a CCAAT/enhancer-binding protein β (C/EBPβ) siRNA.

In some embodiments of the first aspect, the aptamer comprises or consists of an RNA sequence having at least 90% sequence identity to SEQ ID NO:1 or SEQ ID NO:40, and said RNA sequence has a length of 29 nucleotides or fewer. In some embodiments, the RNA sequence has a length of 22 nucleotides of fewer. In some embodiments the RNA sequence is 22 nucleotides in length. In some embodiments, the RNA sequence has 100% sequence identity to SEQ ID NO:1 or SEQ ID NO:40. In some embodiments, the ribonucleic acid further comprises (iii) a second aptamer capable of binding to transferrin receptor 2 (TfR2) comprising or consisting of SEQ ID NO:3 or 43.

In some embodiments of the first aspect, the C/EBPβ siRNA comprises or consists of a sequence selected from SEQ ID NOs: 7, 9, 11 and 13. In other embodiments, the C/EBPβ siRNA comprises or consists of a sequence selected from SEQ ID NOs: 15 and 17. In other embodiments, the C/EBPβ siRNA comprises or consists of a sequence selected from SEQ ID NOs: 26 and 27.

In some embodiments of the first aspect, the siRNA comprises one or more nucleotides with a substituted 2' ribose. In some embodiments, the siRNA comprises one or more nucleotides with a substituted 2'F ribose. In some embodiments, the siRNA comprises one or more nucleotides with a substituted 2'-O-Me ribose.

In some embodiments of the first aspect, the aptamer is capable of binding to TfR on a cell surface. In some embodiments, the ribonucleic acid is capable of being internalised into a cell.

In some embodiments of the first aspect, the aptamer is conjugated to the siRNA via a linker. The linker may be a cleavable linker, for example a disulphide bridge (SS).

In a second aspect, the invention relates to a pharmaceutical composition comprising a ribonucleic acid compound according to the first aspect. The pharmaceutical composition may optionally comprise a pharmaceutically acceptable excipient and/or a therapeutic agent. The therapeutic agent may be an anticancer agent, for example gemcitabine or a PD-1 axis inhibitor (e.g. a PD-1 inhibitor, preferably pembrolizumab).

In a third aspect, the invention provides a ribonucleic acid compound according to the first aspect, or a pharmaceutical composition the second aspect, for use in a method of treating or preventing a disease or disorder.

In a fourth aspect, the invention provides for the use of a ribonucleic acid compound according to the first aspect, or a pharmaceutical composition according to the second aspect, in the manufacture of a medicament for use in a method of treating or preventing a disease or disorder.

In a fifth aspect, the invention provides a method of treating or preventing a disease or disorder, the method comprising administering to a subject in need thereof an effective amount of a ribonucleic acid compound according to the first aspect, or of a pharmaceutical composition the second aspect.

In some embodiments of the third to fifth aspects, the disease or disorder is cancer. The cancer may be selected from pancreatic cancer and liver cancer. More specifically, the cancer may be pancreatic ductal adenocarcinoma (PDAC) with liver metastasis.

In some embodiments of the third to fifth aspects, the method further comprises administering an additional therapeutic agent, such as an anticancer agent. The anticancer agent may be gemcitabine and/or or a PD-1 axis inhibitor (e.g. a PD-1 inhibitor, preferably pembrolizumab).

In a sixth aspect, the invention provides a method of delivering a CCAAT/enhancer-binding protein β (C/EBPβ) siRNA to a cell, the method comprising:
i. contacting a cell with a ribonucleic acid compound according to the first aspect, or a pharmaceutical composition the second aspect; and
ii. allowing said ribonucleic acid compound to bind to a transferrin receptor on said cell and pass into said cell thereby delivering said C/EBPβ siRNA into said cell.

Also provided is a C/EBPβ siRNA, which is capable of inhibiting C/EBPβ in a cell. In some embodiments, the C/EBPβ siRNA, when processed by endoribonuclease Dicer, is capable of binding to an mRNA sequence encoding C/EBPβ. In preferred embodiments, the C/EBPβ siRNA comprises or consists of a sequence selected from SEQ ID NOs: 7, 9, 11 and 13, more preferably selected from SEQ ID NOs: 7, and 9.

Also provided is a ribonucleic acid compound comprising: (i) one or more N-Acetylgalactosamine (GaINAc) moieties, and (ii) a CCAAT/enhancer-binding protein β (C/EBPβ) siRNA. These ribonucleic acid compounds find use in any and/or all of the second to fifth aspects listed above in place of the ribonucleic acid compounds of the first aspect. In preferred embodiments, the C/EBPβ siRNA comprises or consists of a sequence selected from SEQ ID NOs: 7, 9, 11 and 13, more preferably selected from SEQ ID NOs: 7, and 9. In some emboduments, the GalNAc moiety is connected to the siRNA by a linker, preferably a aminohexyl (NHC6) linker. In some embodiments, the ribonucleic acid compound comprises or consists of SEQ ID NO:44 or 46.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:

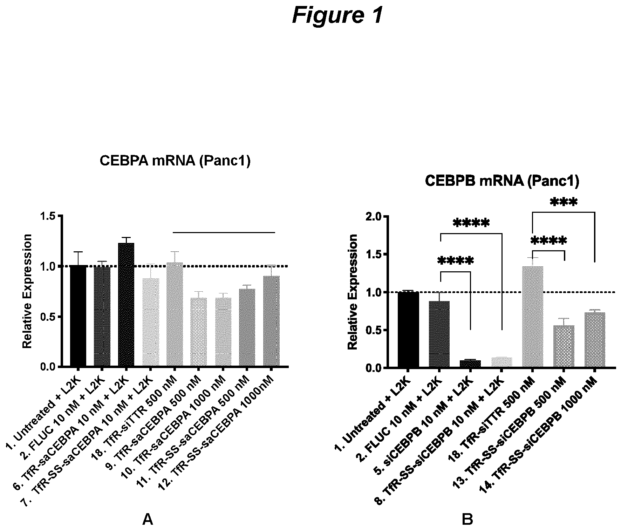

**Figure 1****.** Effects of TfR aptamer-small RNAs on relative mRNA expression levels in Panc1 cells. **(A)** Effects on CEBPA mRNA. From right to left: Untreated + Lipofectamine^{™} 2000 (L2K) ; FLUC 10 nM + L2K; TfR-saCEBPA 10 nM + L2K; TfR-SS-saCEBPA 10 nM + L2K; TfR-siTTR 500 nM (passive delivery) ; TfR-saCEBPA 500 nM (passive delivery) ; TfR-saCEBPA 1000 nM (passive delivery) ; TfR-SS-saCEBPA 500 nM (passive delivery) ; TfR-SS-saCEBPA 1000 nM (passive delivery) . **(B)** Effects on CEBPB mRNA. From right to left: Untreated + L2K; FLUC 10 nM + L2K; TfR-saCEBPB 10 nM + L2K; TfR-SS-saCEBPB 10 nM + L2K; TfR-siTTR 500 nM (passive delivery) ; TfR-SS-saCEBPB 500 nM (passive delivery) ; TfR-SS-saCEBPB 1000 nM (passive delivery) .

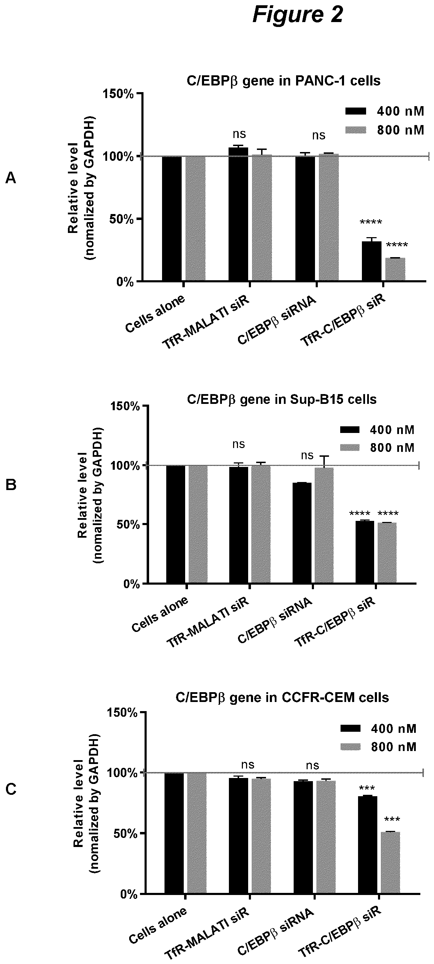

**Figure 2****.** Effects of small RNAs and TfR aptamer-small RNAs on relative CEBPB mRNA expression in multiple human cell lines. **(A)** Effects in PANC-1 cells. From left to right: cells alone (empty vehicle) ; TfR-MALATI siRNA; siCEBPB; TfR-siCEBPB.. **(B)** Effects in Sup-B15 cells. From left to right: cells alone (empty vehicle) ; TfR-MALATI siRNA; siCEBPB; TfR-siCEBPB. **(C)** Effects in CCFR-CEM cells. From left to right: cells alone (empty vehicle) ; TfR-MALATI siRNA; siCEBPB; TfR-siCEBPB. In all three cell lines, each treatment is administered as 400nM and 800 nM concentrations.

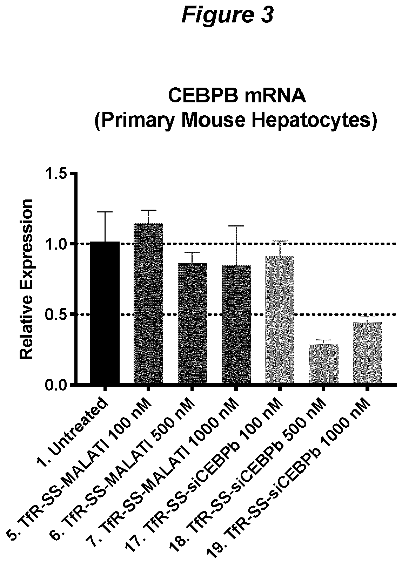

**Figure 3****.** Effects of small RNAs and TfR aptamer-small RNAs on relative CEBPB mRNA expression in primary mouse hepatocytes. From left to right: untreated cells; TfR-SS-MALATI siRNA (100 nM) ; TfR-SS-MALATI siRNA (500 nM) ; TfR-SS-siCEBPb 100 nM; TfR-SS-siCEBPb 500 nM; TfR-SS-siCEBPb 1000 nM

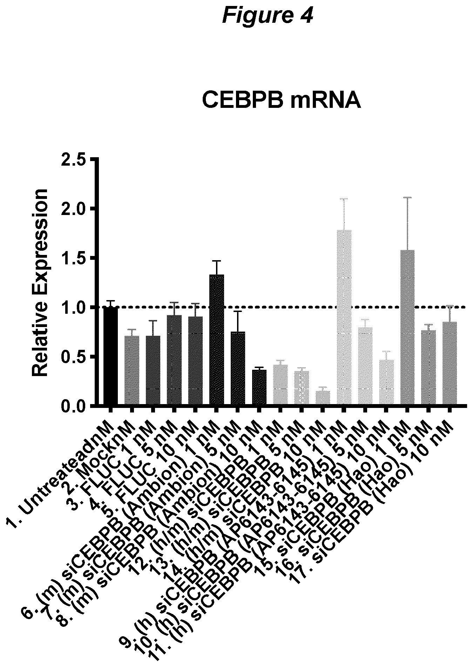

**Figure 4****.** Comparison of CEBPB mRNA suppressive effects of small RNAs designed against different CEBPB sequences in BNL-ME mouse cells. From left to right: untreated; mock (empty vehicle) treated; siFLUC at 1 nM, 5nM and 10 nM; murine (m) siCEBPB (Ambion) at 1 nM, 5nm and 10nM; human-mouse cross reactive (h/m) siCEBPB at 1nM, 5nM and 10 nM; human (h) siCEBPB (AP6143-6145) at 1nM, 5nM and 10 nM, rat siCEBPB (Hao) at 1 nM, 5nM and 10 nM.

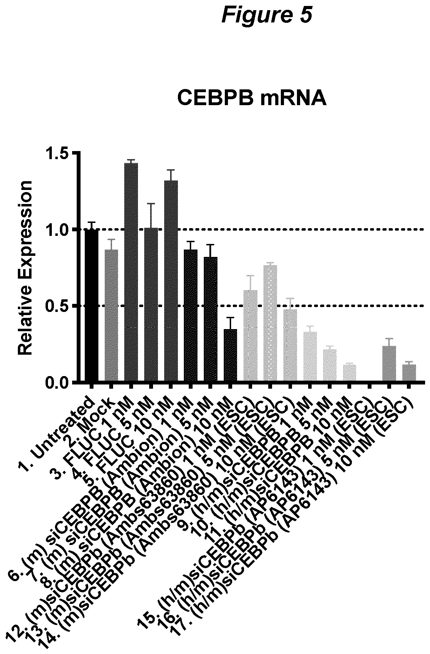

**Figure 5****.** Comparison of CEBPB mRNA suppressive effects of small RNAs with and without ESC 2'-modifications in BNL-ME mouse cells. From left to right: untreated; mock (empty vehicle) treated; siFLUC at 1nM, 5nM and 10 nM; murine-derived naked siRNA ((m) siCEBPB (Ambion) ) at 1nM, 5nm and 10nM; murine-derived ESC modified siRNA ((m) siCEBPB (Ambs63860) ) at 1nM, 5nm and 10nM; human-mouse cross reactive naked siRNA ((h/m) siCEBPB) at 1nM, 5nM and 10 nM; human-mouse cross reactive ESC modified siRNA ((h/m) siCEBPB) at 1nM, 5nM and 10 nM.

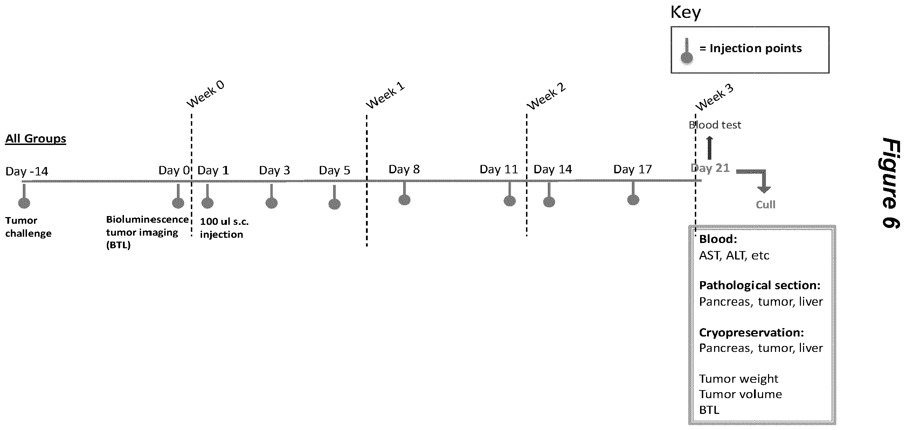

**Figure 6****.** Investigative schema of treatment with TfR-siCEBPb in a mouse model of pancreatic cancer with liver metastasis.

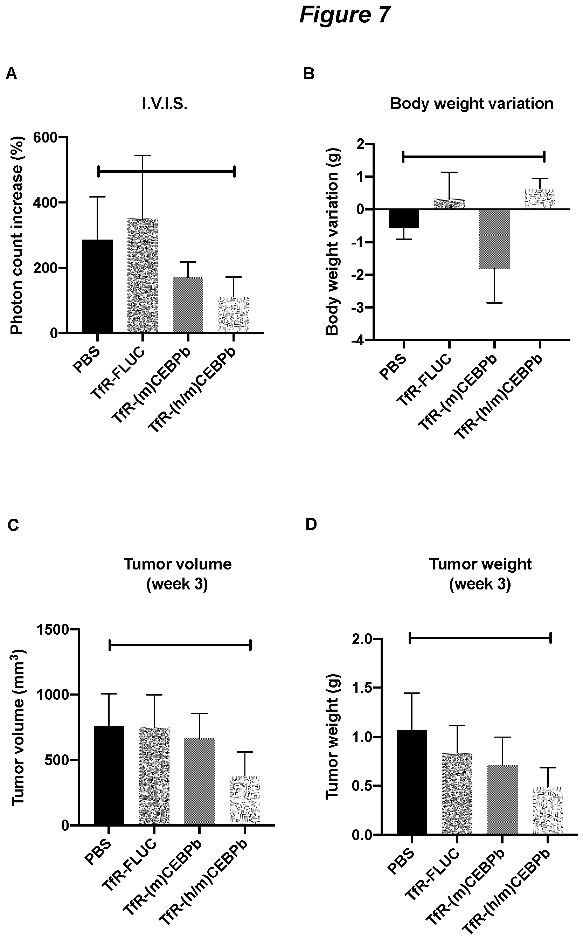

**Figure 7****.** Results of treatment with ribonucleic acids on mouse model of pancreatic cancer with liver metastasis. (A) Change in mean photon count from bioluminescence tumour imaging (BTL), performed using an *in vitro* imaging system (IVIS), at week 3 relative to week 0. Treatment groups (left to right): PBS control, TfR-siFLUC, TfR-SS- siCEBPb ESC seq 1 (SEQ ID NO: 41+50), TfR-SS-(h/m) siCEBPb ESC seq 2 (SEQ ID NO: 34+47), TfR--SS-(h/m) siCEBPb-Tag. (B) Change in mean body weight at week 3 relative to week 0. Treatment groups (left to right): PBS control, TfR-siFLUC, TfR-SS- siCEBPb ESC seq 1 (SEQ ID NO: 41+50), TfR-SS-(h/m) siCEBPb ESC seq 2 (SEQ ID NO: 34+47), TfR--SS-(h/m) siCEBPb-Tag. (C) Mean tumor volume at week 3 (mm³). Treatment groups (left to right): PBS control, TfR-siFLUC, TfR-SS- siCEBPb ESC seq 1 (SEQ ID NO: 41+50), TfR-SS-(h/m) siCEBPb ESC seq 2 (SEQ ID NO: 34+47), TfR--SS-(h/m) siCEBPb-Tag. (D) Mean tumor weights at week 3 (g). Treatment groups (left to right): PBS control, TfR-siFLUC, TfR-SS- siCEBPb ESC seq 1 (SEQ ID NO: 41+50), TfR-SS-(h/m) siCEBPb ESC seq 2 (SEQ ID NO: 34+47), TfR--SS-(h/m) siCEBPb-Tag.

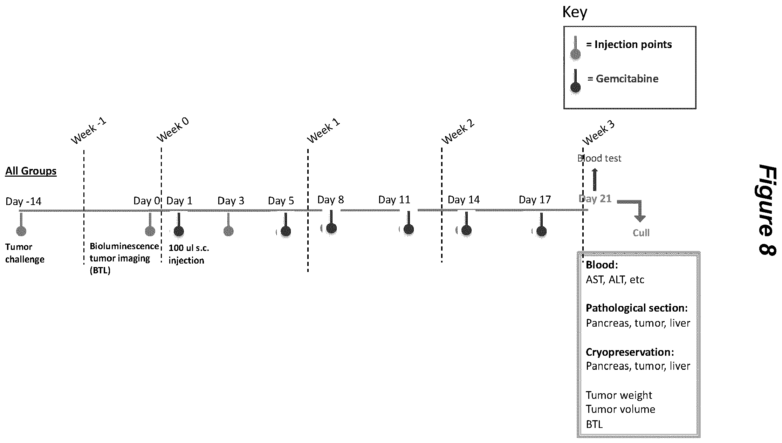

**Figure 8****.** Investigative schema of treatment with TfR-siCEBPb and gemcitabine in a mouse model of pancreatic cancer with liver metastasis.

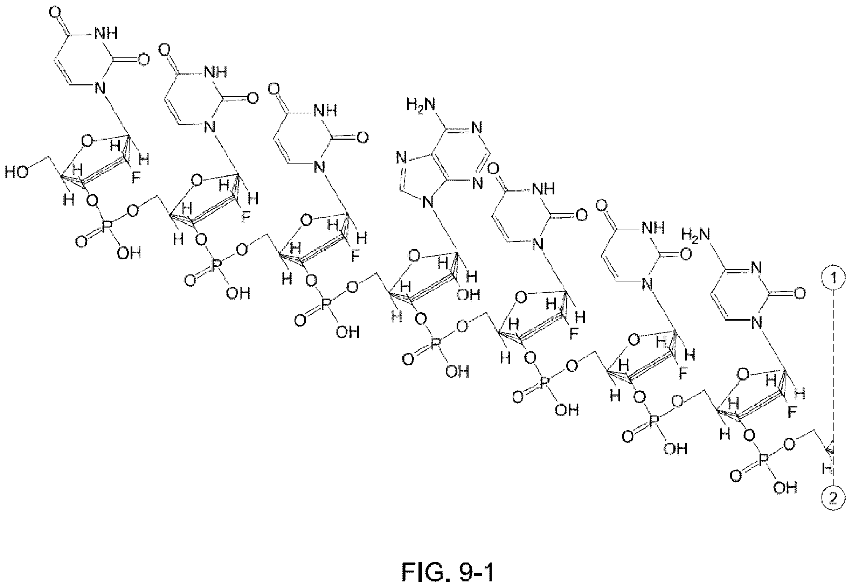

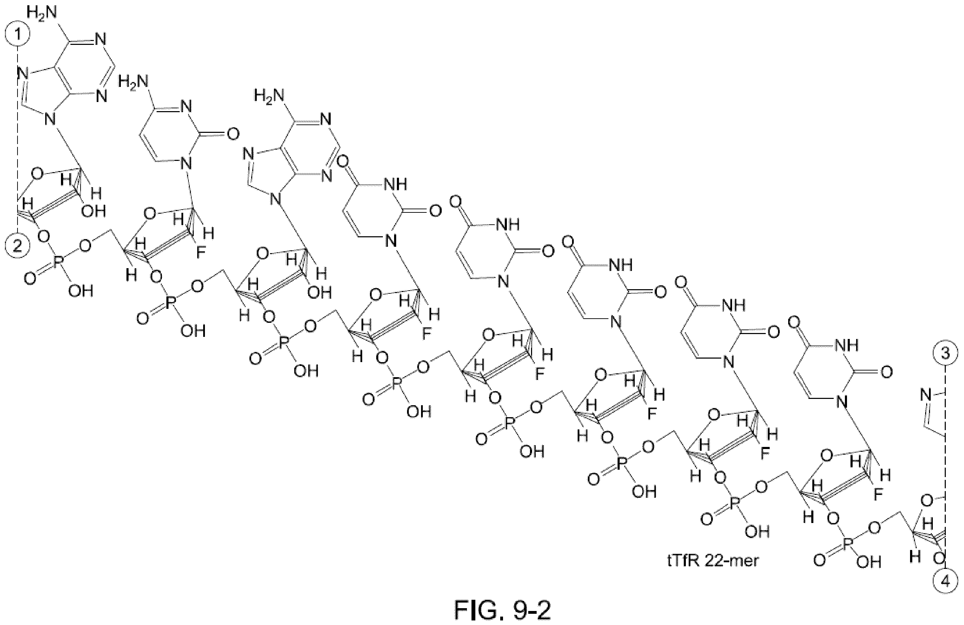

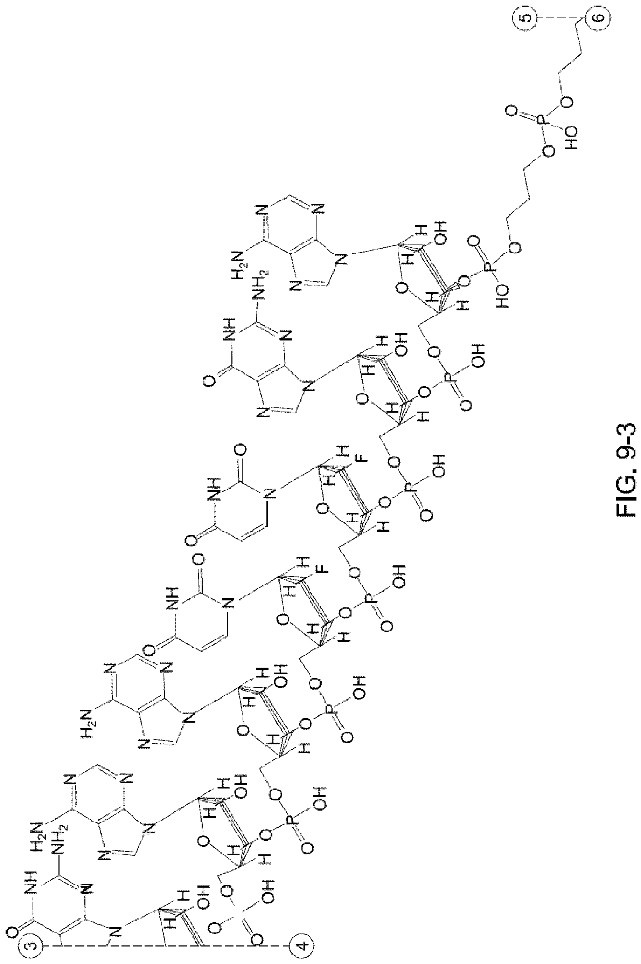

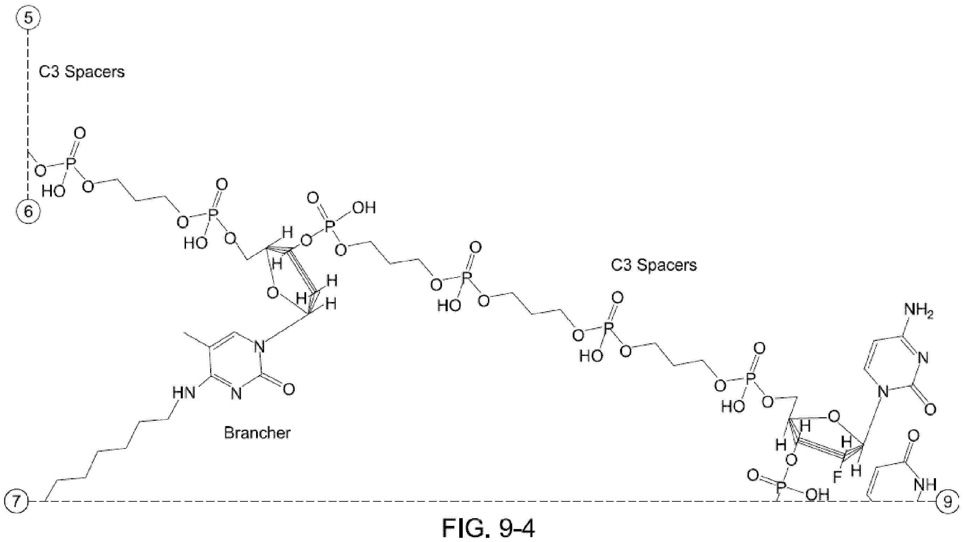

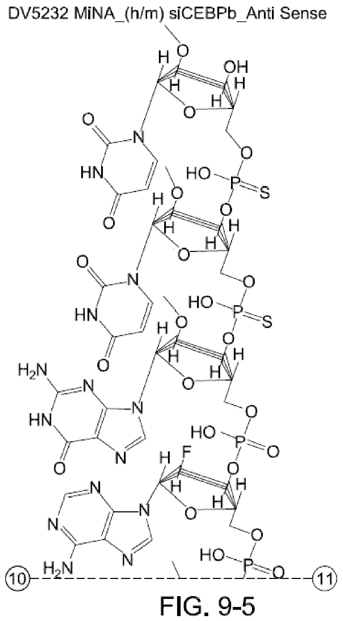

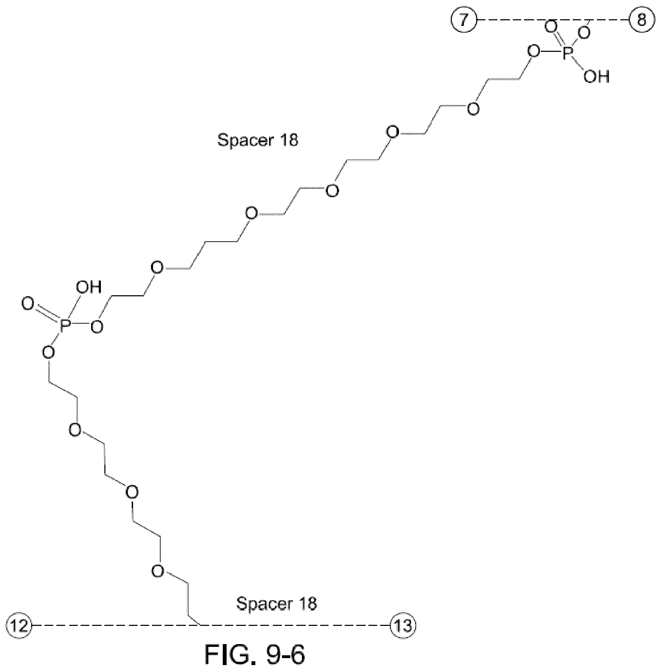

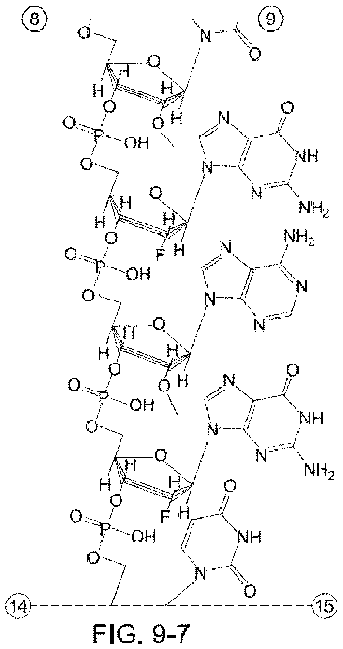

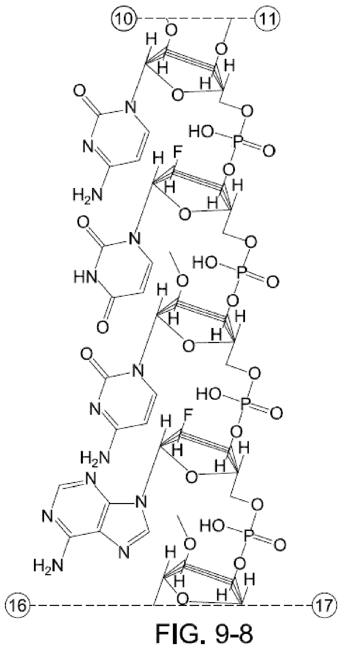

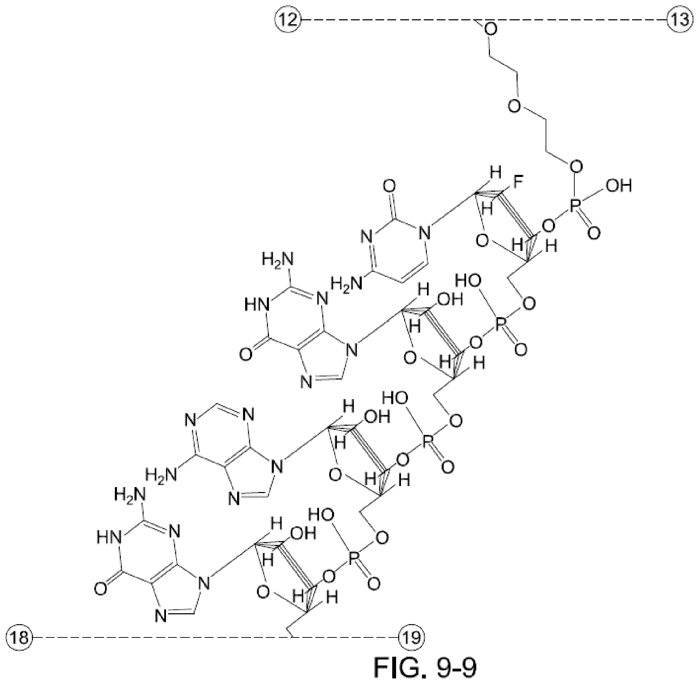

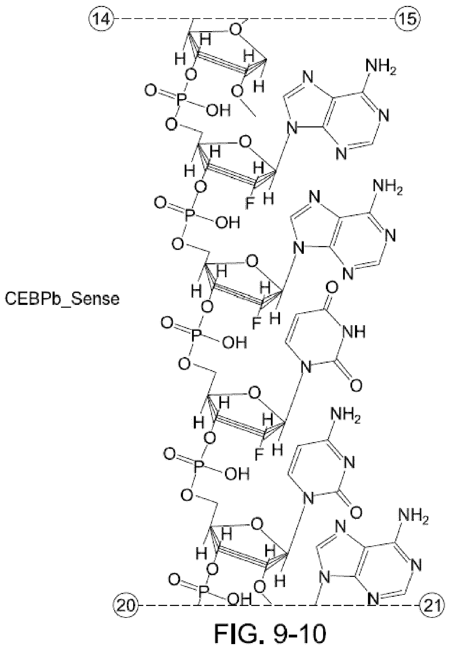

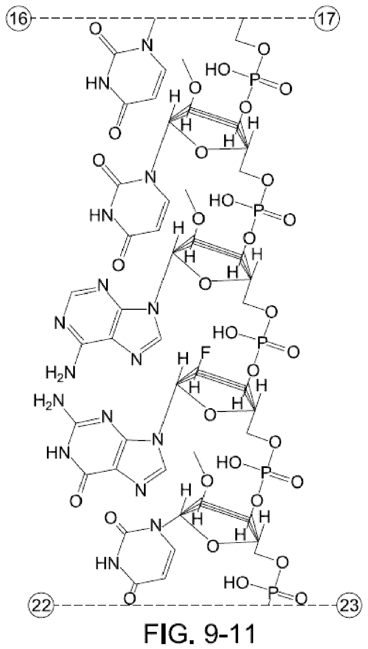

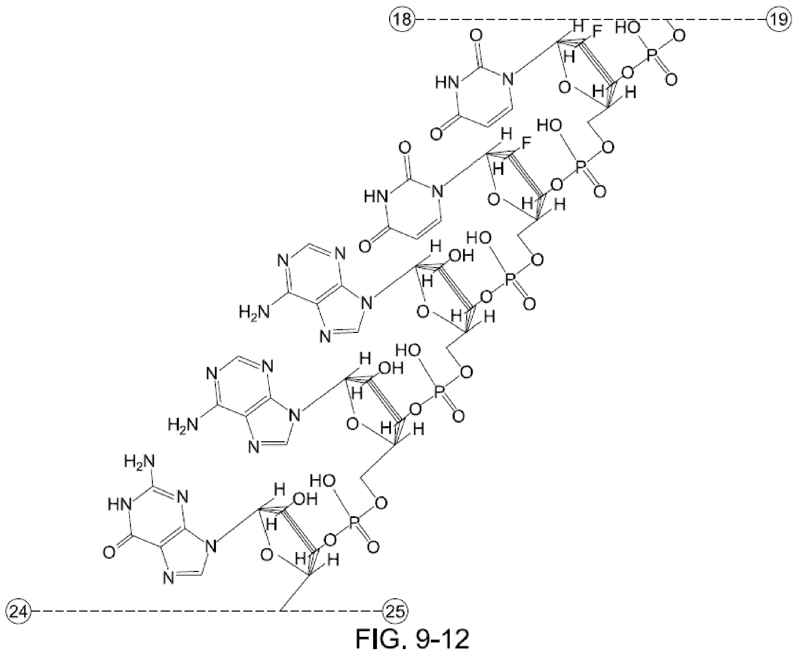

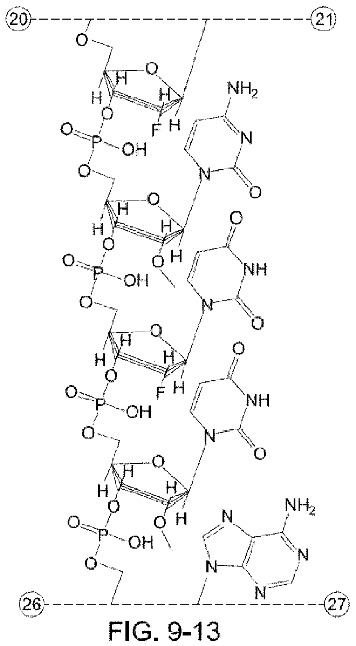

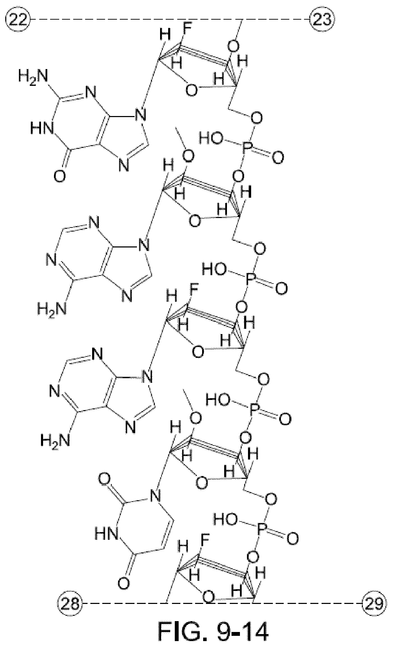

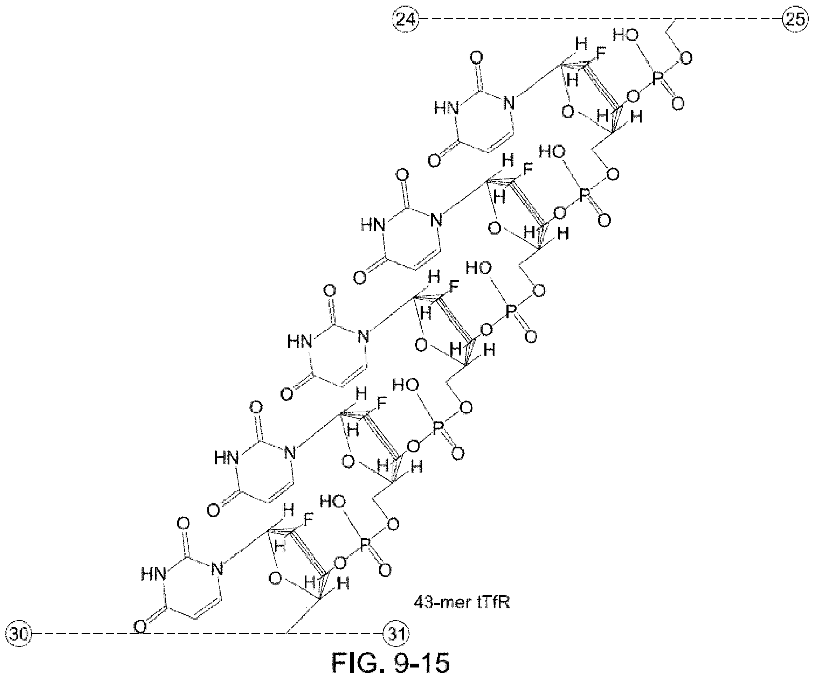

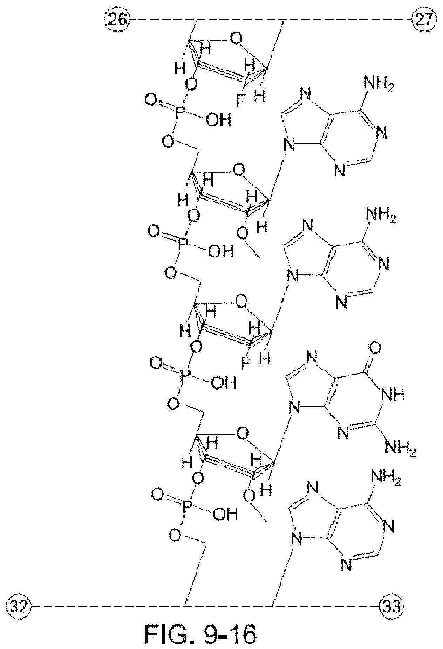

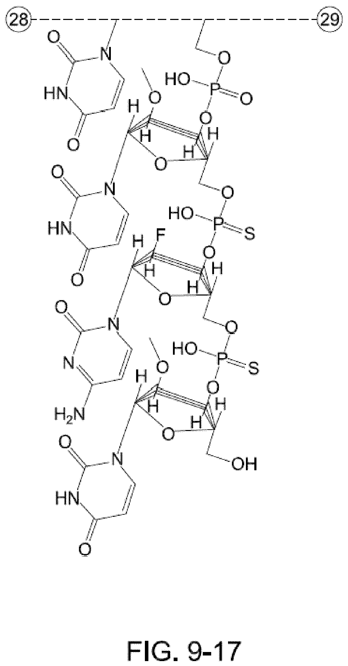

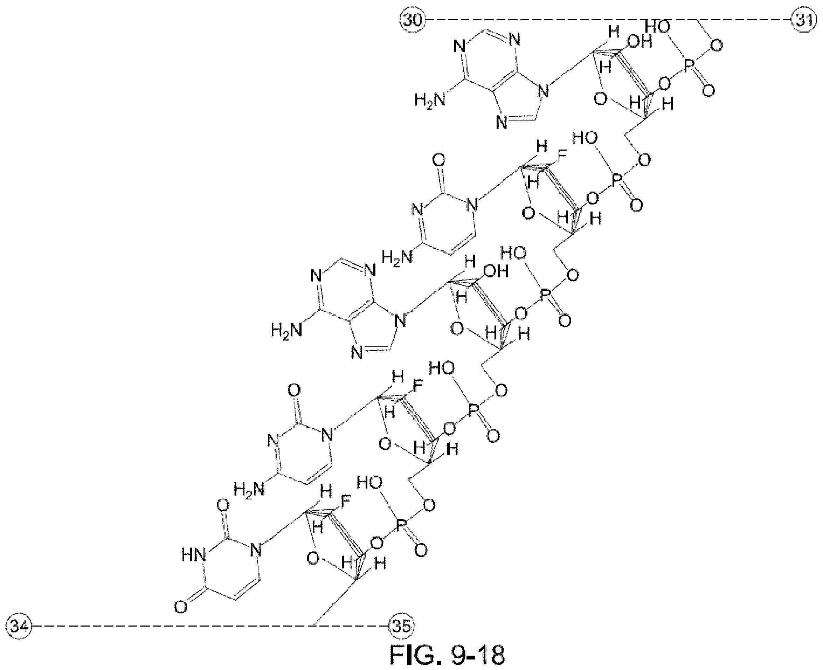

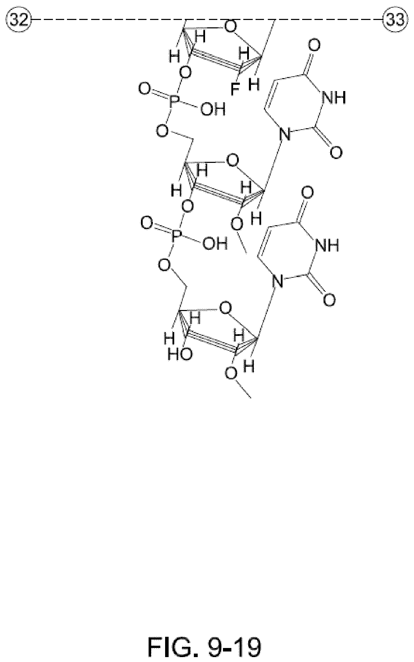

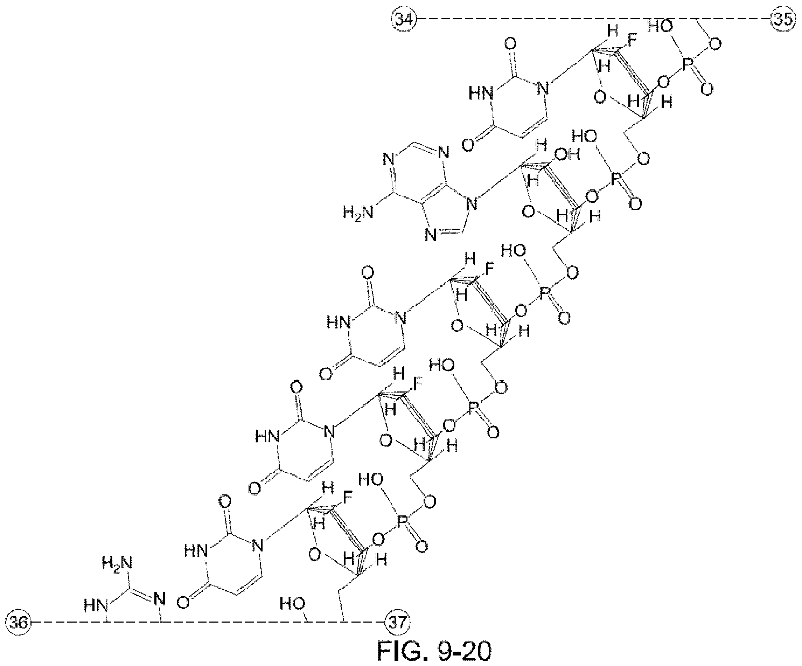

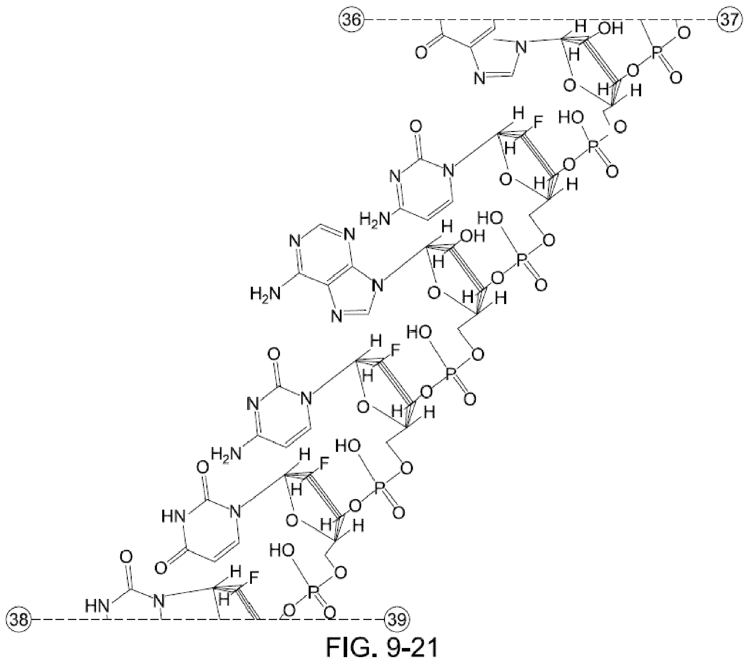

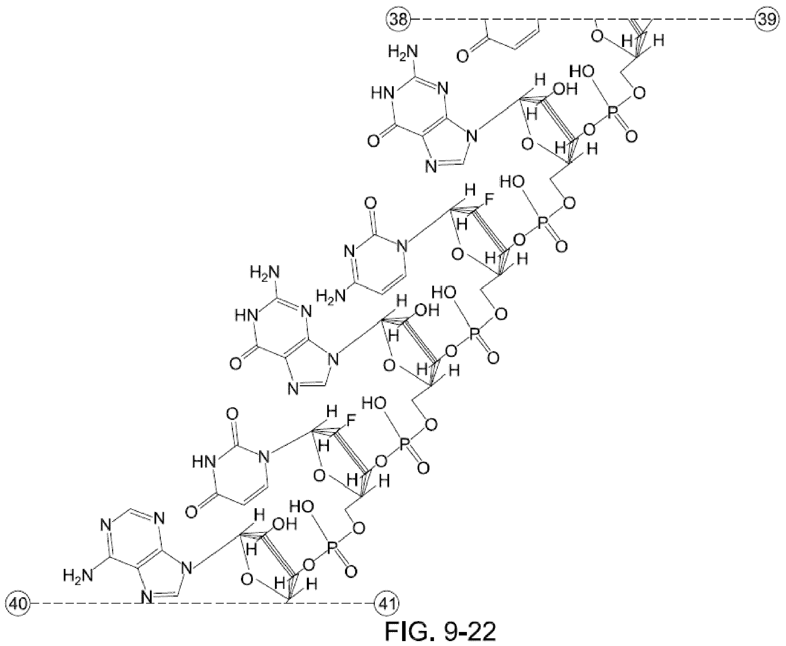

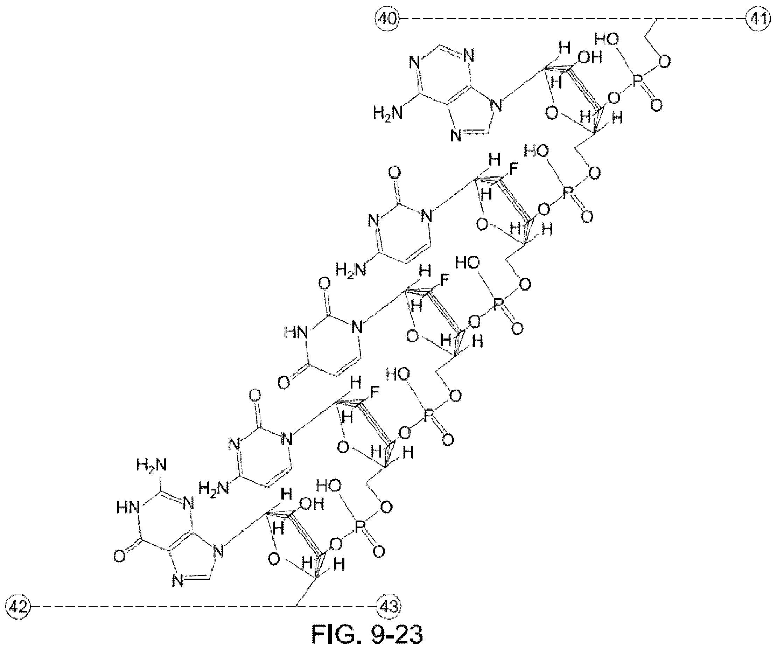

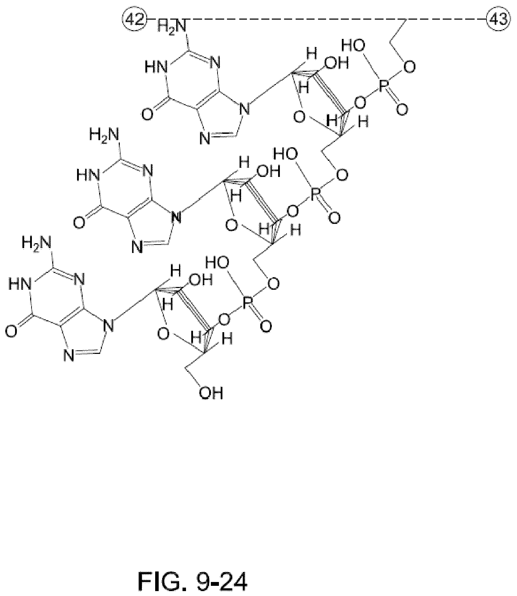

**Figure 9****.** Schematic of "triangular" TfR-siCEBPb ribonucleic acid molecule comprising a TfR1-specific aptamer according to SEQ ID NO:40 (top left), a TfR2-specific aptamer according to SEQ ID NO:43 (bottom left), and a C/EBPβ siRNA (right hand side, SEQ ID NO:15, shown paired with antisense strand SEQ ID NO:16). The TfR1-specific aptamer is linked to a spacer comprising three phosphoramite C3 spacers joined in series. The TfR2-specific aptamer is linked to a spacer two phosphoramite 18 spacers joined in series. The C/EBPβ siRNA is linked to a spacer comprising three phosphoramite C3 spacers joined in series. The spacers are linked through a levulinyl 5-Me-dC brancher, with the C/EBPβ siRNA linker attached 3' to the phosphate group at dC carbon-3, the TfR1-specific aptamer linker attached 5' to the phosphate group at dC carbon-5, and the TfR2-specific aptamer linker attached 3' to the pyrimidine-levulinyl phosphate group

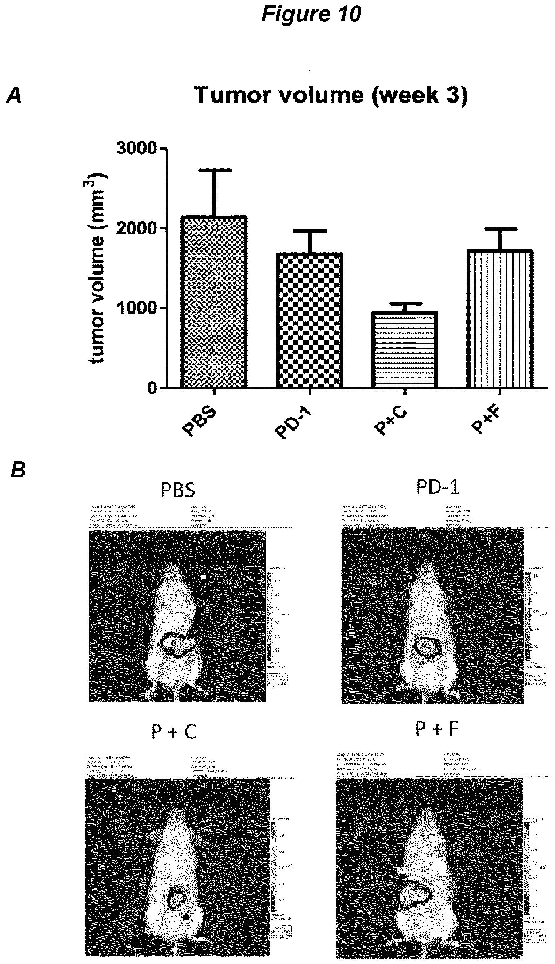

Figure 10. Effect of TfR-siCEBPb in combination with PD-1 inhibitor pembrolizumab in a mouse model of PDAC. **A.** Measure of tumor volume (mm³) at week 3 after treatment in PBS (leftmost), pembrolizumab alone (second from left), pembrolizumab in combination with TfR-siCEBPb (second from right), and pembrolizumab in combination with TfR-siFLUC (rightmost). **B.** Bioluminescence tumour imaging (BTL) of representative mice at week 3 after treatment in PBS (top left), pembrolizumab alone (top right), pembrolizumab in combination with TfR-siCEBPb (bottom left), and pembrolizumab in combination with TfR-siFLUC (bottom right).

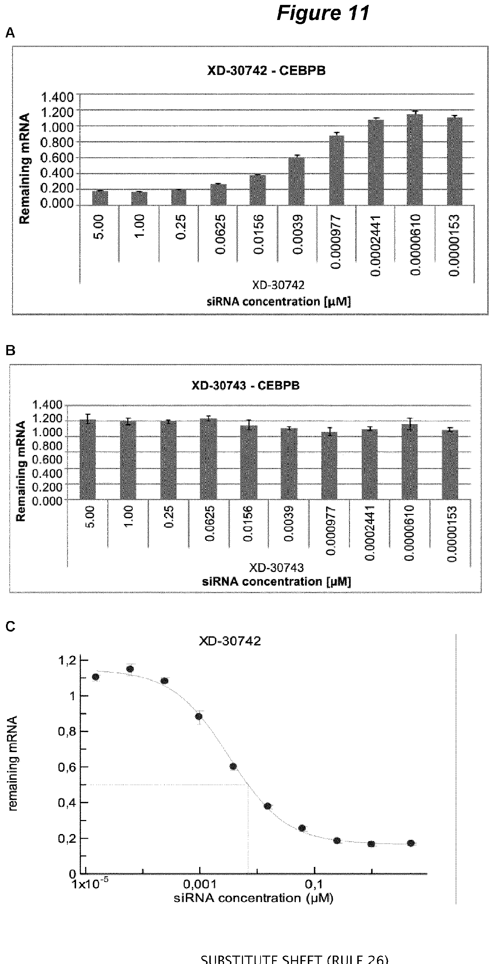

**Figure 11****.** Dose response for GAINAc-(h/m)siCEBPb in mouse primary hepatocytes following 48h incubation as reported by bDNA quantification. **A.** Dose response series for XD-30742 (GAINAc-(h/m)siCEBPb), columns represent a 5X series dilution from 5 uM to 15.3 pM. **B.** Dose response series for XD-30743 (GAINAc-siFLUC) control, columns represent a 5X series dilution from 5 uM to 15.3 pM. **C.** Response curve for XD-30742 (GAINAc-(h/m)CEBPb) across the dilution series. Curve fitting was performed by Xlfit.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

### Definitions

While various embodiments and aspects of the present invention are shown and described herein, it will be obvious to those skilled in the art that such embodiments and aspects are provided by way of example only. Numerous variations, changes, and substitutions will now occur to those skilled in the art without departing from the invention. It should be understood that various alternatives to the embodiments of the invention described herein may be employed in practicing the invention.

The section headings used herein are for organizational purposes only and are not to be construed as limiting the subject matter described. All documents, or portions of documents, cited in the application including, without limitation, patents, patent applications, articles, books, manuals, and treatises are hereby expressly incorporated by reference in their entirety for any purpose.

The abbreviations used herein have their conventional meaning within the chemical and biological arts. The chemical structures and formulae set forth herein are constructed according to the standard rules of chemical valency known in the chemical arts.

Unless defined otherwise, technical and scientific terms used herein have the same meaning as commonly understood by a person of ordinary skill in the art. See, e.g., Singleton et al., DICTIONARY OF MICROBIOLOGY AND MOLECULAR BIOLOGY 2nd ed., J. Wiley & 20 Sons (New York, NY 1994) ; Sambrook et al., MOLECULAR CLONING, A LABORATORY MANUAL, Cold Springs Harbor Press (Cold Springs Harbor, NY 1989) . Any methods, devices and materials similar or equivalent to those described herein can be used in the practice of this invention. The following definitions are provided to facilitate understanding of certain terms used frequently herein and are not meant to limit the scope of the present disclosure.

"Nucleic acid" refers to deoxyribonucleotides or ribonucleotides and polymers thereof in either single-, double- or multiple-stranded form, or complements thereof. The term "polynucleotide" refers to a linear sequence of nucleotides. The term "nucleotide" typically refers to a single unit of a polynucleotide, i.e., a monomer. Nucleotides can be ribonucleotides, deoxyribonucleotides, or modified versions thereof. Examples of polynucleotides contemplated herein include single and double stranded DNA, single and double stranded RNA (including siRNA) , and hybrid molecules having mixtures of single and double stranded DNA and RNA. Nucleic acids can be linear or branched. For example, nucleic acids can be a linear chain of nucleotides or the nucleic acids can be branched, e.g., such that the nucleic acids comprise one or more arms or branches of nucleotides. Optionally, the branched nucleic acids are repetitively branched to form higher ordered structures such as dendrimers and the like.

Nucleic acids, including nucleic acids with a phosphothioate backbone can include one or more reactive moieties. As used herein, the term reactive moiety includes any group capable of reacting with another molecule, e.g., a nucleic acid or polypeptide through covalent, noncovalent or other interactions. By way of example, the nucleic acid can include an amino acid reactive moiety that reacts with an amino acid on a protein or polypeptide through a covalent, non-covalent or other interaction.

The terms also encompass nucleic acids containing known nucleotide analogs or modified backbone residues or linkages, which are synthetic, naturally occurring, and non-naturally occurring, which have similar binding properties as the reference nucleic acid, and which are metabolized in a manner similar to the reference nucleotides. Examples of such analogs include, without limitation, phosphodiester derivatives including, e.g., phosphoramidate, phosphorodiamidate, phosphorothioate (also known as phosphothioate) , phosphorodithioate, phosphonocarboxylic acids, phosphonocarboxylates, phosphonoacetic acid, phosphonoformic acid, methyl phosphonate, boron phosphonate, or O-methylphosphoroamidite linkages (see Eckstein, Oligonucleotides and Analogues: A Practical Approach, Oxford University Press) ; and peptide nucleic acid backbones and linkages. Other analog nucleic acids include those with positive backbones; non-ionic backbones, modified sugars, and non-ribose backbones (e.g. phosphorodiamidate morpholino oligos or locked nucleic acids (LNA) ) , including those described in U.S. Patent Nos. 5,235,033 and 5,034,506, and Chapters 6 and 7, ASC Symposium Series 580, Carbohydrate Modifications in Antisense Research, Sanghui & Cook, eds. Nucleic acids containing one or more carbocyclic sugars are also included within one definition of nucleic acids. Modifications of the ribose-phosphate backbone may be done for a variety of reasons, e.g., to increase the stability and half-life of such molecules in physiological environments or as probes on a biochip. Mixtures of naturally occurring nucleic acids and analogs can be made; alternatively, mixtures of different nucleic acid analogs, and mixtures of naturally occurring nucleic acids and analogs may be made. In embodiments, the internucleotide linkages in DNA are phosphodiester, phosphodiester derivatives, or a combination of both.

The words "complementary" or "complementarity" refer to the ability of a nucleic acid in a polynucleotide to form a base pair with another nucleic acid in a second polynucleotide. For example, the sequence A-G-T is complementary to the sequence T-C-A. Complementarity may be partial, in which only some of the nucleic acids match according to base pairing, or complete, where all the nucleic acids match according to base pairing.

The term "probe" or "primer", as used herein, is defined to be one or more nucleic acid fragments whose specific hybridization to a sample can be detected. A probe or primer can be of any length depending on the particular technique it will be used for. For example, PCR primers are generally between 10 and 40 nucleotides in length, while nucleic acid probes for, e.g., a Southern blot, can be more than a hundred nucleotides in length. The probe may be unlabeled or labeled as described below so that its binding to the target or sample can be detected. The probe can be produced from a source of nucleic acids from one or more particular (preselected) portions of a chromosome, e.g., one or more clones, an isolated whole chromosome or chromosome fragment, or a collection of polymerase chain reaction (PCR) amplification products. The length and complexity of the nucleic acid fixed onto the target element is not critical to the invention. One of skill can adjust these factors to provide optimum hybridization and signal production for a given hybridization procedure, and to provide the required resolution among different genes or genomic locations.

The probe may also be isolated nucleic acids immobilized on a solid surface (e.g., nitrocellulose, glass, quartz, fused silica slides) , as in an array. In some embodiments, the probe may be a member of an array of nucleic acids as described, for instance, in WO 96/17958. Techniques capable of producing high density arrays can also be used for this purpose (see, e.g., Fodor (1991) Science 767-773; Johnston (1998) Curr. Biol. 8: R171-R174; Schummer (1997) Biotechniques 23:1087-1092; Kern (1997) Biotechniques 23:120-124; U.S. Patent No. 5,143,854) .

The term "gene" means the segment of DNA involved in producing a protein; it includes regions preceding and following the coding region (leader and trailer) as well as intervening sequences (introns) between individual coding segments (exons) . The leader, the trailer as well as the introns include regulatory elements that are necessary during the transcription and the translation of a gene. Further, a "protein gene product" is a protein expressed from a particular gene.

The word "expression" or "expressed" as used herein in reference to a gene means the transcriptional and/or translational product of that gene. The level of expression of a DNA molecule in a cell may be determined on the basis of either the amount of corresponding mRNA that is present within the cell or the amount of protein encoded by that DNA produced by the cell. The level of expression of non-coding nucleic acid molecules (e.g., siRNA) may be detected by standard PCR or Northern blot methods well known in the art. See, Sambrook et al., 1989 Molecular Cloning: A Laboratory Manual, 18.1-18.88.

An "antisense nucleic acid" as referred to herein is a nucleic acid (e.g. DNA or RNA molecule) that is complementary to at least a portion of a specific target nucleic acid (e.g. an mRNA translatable into a protein) and is capable of reducing transcription of the target nucleic acid (e.g. mRNA from DNA) or reducing the translation of the target nucleic acid (e.g. mRNA) or altering transcript splicing (e.g. single stranded morpholino oligo) . See, e.g., Weintraub, Scientific American, 262:40 (1990) . Typically, synthetic antisense nucleic acids (e.g. oligonucleotides) are generally between 15 and 25 bases in length. Thus, antisense nucleic acids are capable of hybridizing to (e.g. selectively hybridizing to) a target nucleic acid (e.g. target mRNA) . In embodiments, the antisense nucleic acid hybridizes to the target nucleic acid sequence (e.g. mRNA) under stringent hybridization conditions. In embodiments, the antisense nucleic acid hybridizes to the target nucleic acid (e.g. mRNA) under moderately stringent hybridization conditions. Antisense nucleic acids may comprise naturally occurring nucleotides or modified nucleotides such as, e.g., phosphorothioate, methylphosphonate, and -anomeric sugar-phosphate, backbone modified nucleotides.

In the cell, the antisense nucleic acids hybridize to the corresponding mRNA, forming a double-stranded molecule. The antisense nucleic acids interfere with the translation of the mRNA, since the cell will not translate an mRNA that is double-stranded. The use of antisense methods to inhibit the *in vitro* translation of genes is well known in the art (Marcus-Sakura, Anal. Biochem., 172:289 (1988) ) . Further, antisense molecules which bind directly to the DNA may be used. Antisense nucleic acids may be single or double stranded nucleic acids. Non-limiting examples of antisense nucleic acids include siRNAs (including their derivatives or pre-cursors, such as nucleotide analogs) , short hairpin RNAs (shRNA) , micro RNAs (miRNA) , saRNAs (small activating RNAs) and small nucleolar RNAs (snoRNA) or certain of their derivatives or pre-cursors.

The term "isolated", when applied to a nucleic acid or protein, denotes that the nucleic acid or protein is essentially free of other cellular components with which it is associated in the natural state. It can be, for example, in a homogeneous state and may be in either a dry or aqueous solution. Purity and homogeneity are typically determined using analytical chemistry techniques such as polyacrylamide gel electrophoresis or high performance liquid chromatography. A protein that is the predominant species present in a preparation is substantially purified.

The term "purified" denotes that a nucleic acid or protein gives rise to essentially one band in an electrophoretic gel. In some embodiments, the nucleic acid or protein is at least 50% pure, optionally at least 65% pure, optionally at least 75% pure, optionally at least 85% pure, optionally at least 95% pure, and optionally at least 99% pure.

The term "isolated" may also refer to a cell or sample cells. An isolated cell or sample cells are a single cell type that is substantially free of many of the components which normally accompany the cells when they are in their native state or when they are initially removed from their native state. In certain embodiments, an isolated cell sample retains those components from its natural state that are required to maintain the cell in a desired state. In some embodiments, an isolated (e.g. purified, separated) cell or isolated cells, are cells that are substantially the only cell type in a sample. A purified cell sample may contain at least 60%, 70%, 75%, 80%, 85%, 90%, 95%, 96%, 97%, 98%, 99%, or 100% of one type of cell. An isolated cell sample may be obtained through the use of a cell marker or a combination of cell markers, either of which is unique to one cell type in an unpurified cell sample. In some embodiments, the cells are isolated through the use of a cell sorter. In some embodiments, antibodies against cell proteins are used to isolate cells.

As used herein, the term "conjugate" refers to the association between atoms or molecules. The association can be direct or indirect. For example, a conjugate between a nucleic acid (e.g., ribonucleic acid) and a compound moiety as provided herein can be direct, e.g., by covalent bond, or indirect, e.g., by non-covalent bond. Optionally, conjugates are formed using conjugate chemistry including, but are not limited to nucleophilic substitutions (e.g., reactions of amines and alcohols with acyl halides, active esters) , electrophilic substitutions (e.g., enamine reactions) and additions to carbon-carbon and carbon-heteroatom multiple bonds (e.g., Michael reaction, Diels-Alder addition) . These and other useful reactions are discussed in, for example, March, ADVANCED ORGANIC CHEMISTRY, 3rd Ed., John Wiley & Sons, New York, 1985; Hermanson, BIOCONJUGATE TECHNIQUES, Academic Press, San Diego, 1996; and Feeney et al., MODIFICATION OF PROTEINS; Advances in Chemistry Series, Vol. 198, American Chemical Society, Washington, D.C., 1982. Thus, the nucleic acid acids can be attached to a compound moiety through its backbone. Optionally, the ribonucleic acid includes one or more reactive moieties, e.g., an amino acid reactive moiety, that facilitates the interaction of the ribonucleic acid with the compound moiety.

Useful reactive moieties or functional groups used for conjugate chemistries herein include, for example:
(a) carboxyl groups and various derivatives thereof including, but not limited to, N-hydroxysuccinimide esters, N-hydroxybenztriazole esters, acid halides, acyl imidazoles, thioesters, p-nitrophenyl esters, alkyl, alkenyl, alkynyl and aromatic esters;
(b) hydroxyl groups which can be converted to esters, ethers, aldehydes, etc;
(c) haloalkyl groups wherein the halide can be later displaced with a nucleophilic group such as, for example, an amine, a carboxylate anion, thiol anion, carbanion, or an alkoxide ion, thereby resulting in the covalent attachment of a new group at the site of the halogen atom;
(d) dienophile groups which are capable of participating in Diels-Alder reactions such as, for example, maleimido groups;
(e) aldehyde or ketone groups such that subsequent derivatization is possible via formation of carbonyl derivatives such as, for example, imines, hydrazones, semicarbazones or oximes, or via such mechanisms as Grignard addition or alkyllithium addition;
(f) sulfonyl halide groups for subsequent reaction with amines, for example, to form sulfonamides;
(g) thiol groups, which can be converted to disulfides, reacted with acyl halides, or bonded to metals such as gold;
(h) amine or sulfhydryl groups, which can be, for example, acylated, alkylated or oxidized;
(i) alkenes, which can undergo, for example, cycloadditions, acylation, Michael addition, etc;
(j) epoxides, which can react with, for example, amines and hydroxyl compounds;
(k) phosphoramidites and other standard functional groups useful in nucleic acid synthesis;
(l) metal silicon oxide bonding;
(m) metal bonding to reactive phosphorus groups (e.g. phosphines) to form, for example, phosphate diester bonds; and
(n) sulfones, for example, vinyl sulfone.

The reactive functional groups can be chosen such that they do not participate in, or interfere with, the chemical stability of the proteins described herein. By way of example, the nucleic acids can include a vinyl sulfone or other reactive moiety. Optionally, the nucleic acids can include a reactive moiety having the formula S-S-R. R can be, for example, a protecting group. Optionally, R is hexanol. As used herein, the term hexanol includes compounds with the formula C₆H₁₃OH and includes, 1-hexanol, 2-hexanol, 3-hexanol, 2-methyl-1-pentanol, 3-methyl-1-pentanol, 4-methyl-1-pentanol, 2-methyl-2-pentanol, 3-methyl-2-pentanol, 4-methyl-2-pentanol, 2-methyl-3-pentanol, 3-methyl-3-pentanol, 2,2-dimethyl-1-butanol, 2,3-dimethyl-1-butanol, 3,3-dimethyl-1-butanol, 2,3-dimethyl-2-butanol, 3,3-dimethyl-2-butanol, and 2-ethyl-1-butanol. Optionally, R is 1-hexanol.

As used herein, the term "about" means a range of values including the specified value, which a person of ordinary skill in the art would consider reasonably similar to the specified value. In embodiments, the term "about" means within a standard deviation using measurements generally acceptable in the art. In embodiments, about means a range extending to +/- 10% of the specified value. In some embodiments, about means the specified value.

The terms "protein", "peptide", and "polypeptide" are used interchangeably to denote an amino acid polymer or a set of two or more interacting or bound amino acid polymers. The terms apply to amino acid polymers in which one or more than one amino acid residue is an artificial chemical mimetic of a corresponding naturally occurring amino acid, as well as to naturally occurring amino acid polymers and non-naturally occurring amino acid polymers.

The term "amino acid" refers to naturally occurring and synthetic amino acids, as well as amino acid analogs and amino acid mimetics that function in a manner similar to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, i.e., an a carbon that is bound to a hydrogen, a carboxyl group, an amino group, and an R group, e.g., homoserine, norleucine, methionine sulfoxide, methionine methyl sulfonium. Such analogs have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions in a manner similar to a naturally occurring amino acid. The terms "non-naturally occurring amino acid" and "unnatural amino acid" refer to amino acid analogs, synthetic amino acids, and amino acid mimetics which are not found in nature.

Amino acids may be referred to herein by either their commonly known three letter symbols or by the one-letter symbols recommended by the IUPAC-IUB Biochemical
Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of skill will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence with respect to the expression product, but not with respect to actual probe sequences.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.

The following eight groups each contain amino acids that are conservative substitutions for one another: 1) Alanine (A) , Glycine (G) ; 2) Aspartic acid (D) , Glutamic acid (E) ; 3) Asparagine (N) , Glutamine (Q) ; 4) Arginine (R) , Lysine (K) ; 5) Isoleucine (I) , Leucine (L) , Methionine (M) , Valine (V) ; 6) Phenylalanine (F) , Tyrosine (Y) , Tryptophan (W) ; 7) Serine (S) , Threonine (T) ; and 8) Cysteine (C) , Methionine (M) (see, e.g., Creighton, Proteins (1984)) .

For specific proteins described herein (e.g., TfR) , the named protein includes any of the protein's naturally occurring forms, variants or homologs that maintain the protein transcription factor activity (e.g., within at least 50%, 80%, 90%, 95%, 96%, 97%, 98%, 99% or 100% activity compared to the native protein) . In some embodiments, variants or homologs have at least 90%, 95%, 96%, 97%, 98%, 99% or 100% amino acid sequence identity across the whole sequence or a portion of the sequence (e.g. a 50, 100, 150 or 200 continuous amino acid portion) compared to a naturally occurring form. In other embodiments, the protein is the protein as identified by its NCBI sequence reference. In other embodiments, the protein is the protein as identified by its NCBI sequence reference, homolog or functional fragment thereof.

A "cell" as used herein, refers to a cell carrying out metabolic or other function sufficient to preserve or replicate its genomic DNA. A cell can be identified by well-known methods in the art including, for example, presence of an intact membrane, staining by a particular dye, ability to produce progeny or, in the case of a gamete, ability to combine with a second gamete to produce a viable offspring. Cells may include prokaryotic and eukaryotic cells. Prokaryotic cells include but are not limited to bacteria. Eukaryotic cells include but are not limited to yeast cells and cells derived from plants and animals, for example mammalian, insect (e.g., spodoptera) and human cells.

"Anti-cancer agent" is used in accordance with its plain ordinary meaning and refers to a composition (e.g. compound, drug, antagonist, inhibitor, modulator) having antineoplastic properties or the ability to inhibit the growth or proliferation of cells. In embodiments, an anticancer agent is a chemotherapeutic. In embodiments, an anti-cancer agent is an agent identified herein having utility in methods of treating cancer. In embodiments, an anti-cancer agent is an agent approved by the FDA or similar regulatory agency of a country other than the USA, for treating cancer. Examples of anti-cancer agents include, but are not limited to, MEK (e.g. MEK1, MEK2, or MEK1 and MEK2) inhibitors (e.g. XL518, CI-1040, PD035901, selumetinib/AZD6244, GSK1120212/trametinib, GDC-0973, ARRY-162, ARRY-300,

AZD8330, PD0325901, U0126, PD98059, TAK-733, PD318088, AS703026, BAY 869766) , alkylating agents (e.g., cyclophosphamide, ifosfamide, chlorambucil, busulfan, melphalan, mechlorethamine, uramustine, thiotepa, nitrosoureas, nitrogen mustards (e.g., mechloroethamine, cyclophosphamide, chlorambucil, meiphalan) , ethylenimine and methylmelamines (e.g., hexamethlymelamine, thiotepa) , alkyl sulfonates (e.g., busulfan) , nitrosoureas (e.g., carmustine, lomustine, semustine, streptozocin) , triazenes (decarbazine) ) , anti-metabolites (e.g., 5-azathioprine, leucovorin, capecitabine, fludarabine, gemcitabine, pemetrexed, raltitrexed, folic acid analog (e.g., methotrexate) , or pyrimidine analogs (e.g., fluorouracil, floxouridine, Cytarabine ) , purine analogs (e.g., mercaptopurine, thioguanine, pentostatin) , etc.) , plant alkaloids (e.g., vincristine, vinblastine, vinorelbine, vindesine, podophyllotoxin, paclitaxel, docetaxel, etc.) , topoisomerase inhibitors (e.g., irinotecan, topotecan, amsacrine, etoposide (VP 16) , etoposide phosphate, teniposide, etc.) , anti tumor antibiotics (e.g., doxorubicin, adriamycin, daunorubicin, epirubicin, actinomycin, bleomycin, mitomycin, mitoxantrone, plicamycin, etc.) , platinum-based compounds (e.g. cisplatin, oxaloplatin, carboplatin) , anthracenedione (e.g., mitoxantrone) , substituted urea (e.g., hydroxyurea) , methyl hydrazine derivative (e.g., procarbazine ) , or adrenocortical suppressant (e.g., mitotane, aminoglutethimide) , epipodophyllotoxins (e.g., etoposide ), PD-1 axis inhibitors (e.g. pembrolizumab).

Further examples of anti-cancer agents include, but are not limited to, antibiotics (e.g., daunorubicin, doxorubicin, bleomycin) , enzymes (e.g., L-asparaginase) , inhibitors of mitogen-activated protein kinase signaling (e.g. U0126, PD98059, PD184352, PD0325901, ARRY-142886, SB239063, SP600125, BAY 43-9006, wortmannin, or LY294002) , mTOR inhibitors, antibodies (e.g., rituxan) , 5-aza-2'-deoxycytidine, doxorubicin, vincristine, etoposide, gemcitabine, imatinib (Gleevec.RTM.) , geldanamycin, 17-N-Allylamino-17-Demethoxygeldanamycin (17-AAG) , bortezomib, trastuzumab, anastrozole; angiogenesis inhibitors; antiandrogen, antiestrogen; antisense oligonucleotides; apoptosis gene modulators; apoptosis regulators; arginine deaminase; BCR/ ABL antagonists; beta lactam derivatives; bFGF inhibitor; bicalutamide; camptothecin derivatives; casein kinase inhibitors (ICOS) ; clomifene analogues; cytarabine dacliximab; dexamethasone; estrogen agonists; estrogen antagonists; etanidazole; etoposide phosphate; exemestane; fadrozole; finasteride; fludarabine; fluorodaunorunicin hydrochloride; gadolinium texaphyrin; gallium nitrate; gelatinase inhibitors; gemcitabine; glutathione inhibitors; hepsulfam; immunostimulant peptides; insulin-like growth factor-I receptor inhibitor; interferon agonists; interferons; interleukins; letrozole; leukemia inhibiting factor; leukocyte alpha interferon; leuprolide+estrogen+progesterone; leuprorelin; matrilysin inhibitors; matrix metalloproteinase inhibitors; MIF inhibitor; mifepristone; mismatched double stranded RNA; monoclonal antibody; mycobacterial cell wall extract; nitric oxide modulators; oxaliplatin; panomifene; pentrozole; phosphatase inhibitors; plasminogen activator inhibitor; platinum complex; platinum compounds; prednisone; proteasome inhibitors; protein A-based immune modulator; protein kinase C inhibitor; protein kinase C inhibitors, protein tyrosine phosphatase inhibitors; purine nucleoside phosphorylase inhibitors; ras farnesyl protein transferase inhibitors; ras inhibitors; ras-GAP inhibitor; ribozymes; signal transduction inhibitors; signal transduction modulators; single chain antigen-binding protein; stem cell inhibitor; stem-cell division inhibitors; stromelysin inhibitors; synthetic glycosaminoglycans; tamoxifen methiodide; telomerase inhibitors; thyroid stimulating hormone; translation inhibitors; tyrosine kinase inhibitors; urokinase receptor antagonists; steroids (e.g., dexamethasone) , finasteride, aromatase inhibitors, gonadotropin-releasing hormone agonists (GnRH) such as goserelin or leuprolide, adrenocorticosteroids (e.g., prednisone ), progestins (e.g., hydroxyprogesterone caproate, megestrol acetate, medroxyprogesterone acetate) , estrogens (e.g., diethlystilbestrol, ethinyl estradiol) , antiestrogen (e.g., tamoxifen) , androgens (e.g., testosterone propionate, fluoxymesterone) , antiandrogen (e.g., flutamide) , immunostimulants (e.g., Bacillus Calmette-Guerin (BCG) , levamisole, interleukin-2, alpha-interferon, etc.) , monoclonal antibodies (e.g., anti-CD20, anti-HER2, anti-CD52, anti-HLA-DR, and anti-VEGF monoclonal antibodies) , immunotoxins (e.g., anti-CD33 monoclonal antibody-calicheamicin conjugate, anti-CD22 monoclonal antibody-pseudomonas exotoxin conjugate, etc.) , radioimmunotherapy (e.g., anti-CD20 monoclonal antibody conjugated to ¹¹¹In, ⁹⁰Y, or ¹³¹I, etc.) , triptolide, homoharringtonine, dactinomycin, doxorubicin, epirubicin, topotecan, itraconazole, vindesine, cerivastatin, vincristine, deoxyadenosine, sertraline, pitavastatin, irinotecan, clofazimine, 5-nonyloxytryptamine, vemurafenib, dabrafenib, erlotinib, gefitinib, EGFR inhibitors, epidermal growth factor receptor (EGFR) -targeted therapy or therapeutic (e.g. gefitinib (Iressa^{™}) , erlotinib (Tarceva^{™}) , cetuximab (Erbitux^{™}) , lapatinib (Tykerb^{™}) , panitumumab (Vectibix^{™}) , vandetanib (Caprelsa^{™}) , afatinib/BIBW2992, CI-1033/canertinib, neratinib/HKI-272, CP-724714, TAK-285, AST-1306, ARRY334543, ARRY-380, AG-1478, dacomitinib/PF299804, OSI-420/desmethyl erlotinib, AZD8931, AEE788, pelitinib/EKB-569, CUDC-101, WZ8040, WZ4002, WZ3146, AG-490, XL647, PD153035, BMS-599626) , sorafenib, imatinib, sunitinib, dasatinib, or the like.

"PD-1 axis inhibitor" as used herein refers to any agent capable of inhibiting or disrupting signalling in the PD-1 axis (i.e. PD-1, PD-L1, PD-L2, CD80, B7.1). In particular embodiments, a PD-1 axis inhibitor is an inhibitor of PD-1, PD-L1, and/or PD-L2 activity or function. In some embodiments, an inhibitor of PD-1 activity or function is selected from pembrolizumab, nivolumab, cemiplimab, dostarlimab, JTX-4014, spartalizumab (PDR001), camrelizumab, sintilimab, tislelizumab, toripalimab, INCMGA00012 (MGA012), AMP-224 and AMP-514 (MEDI0680). In some embodiments, an inhibitor of PD-L1 activity or function is selected from atezolizumab, avelumab, envafolimab, durvalumab, BMS-936559 (MDX-1105), CK-301, SHR-1316 (HTI-1088), CS-1001, CBT-502 (TQB-2450) and BGB-A333.

"Chemotherapeutic" or "chemotherapeutic agent" is used in accordance with its plain ordinary meaning and refers to a chemical composition or compound having antineoplastic properties or the ability to inhibit the growth or proliferation of cells.

Additionally, the ribonucleic acid compound described herein can be co-administered with or covalently attached to conventional immunotherapeutic agents including, but not limited to, immunostimulants (e.g., Bacillus Calmette-Guerin (BCG) , levamisole, interleukin-2, alphainterferon, etc.) , monoclonal antibodies (e.g., anti-CD20, anti-HER2, anti-CD52, anti-HLA-DR, anti-PD-1 and anti-VEGF monoclonal antibodies) , immunotoxins (e.g., anti-CD33 monoclonal antibody-calicheamicin conjugate, anti-CD22 monoclonal antibody-pseudomonas exotoxin conjugate, etc.) , and radioimmunotherapy (e.g., anti-CD20 monoclonal antibody conjugated to ¹¹¹In, ⁹⁰Y, or ¹³¹I, etc.) .

In a further embodiment, the ribonucleic acid compounds described herein can be co-administered with conventional radiotherapeutic agents including, but not limited to, radionuclides such as ⁴⁷Sc, ⁶⁴Cu, ⁶⁷Cu, ⁸⁹Sr, ⁸⁶Y, ⁸⁷Y, ⁹⁰Y, ¹⁰⁵Rh, ¹¹¹Ag, ¹¹¹In, ¹¹⁷Sn, ¹⁴⁹Pm, ¹⁵³Sm, ¹⁶⁶Ho, ¹⁷⁷Lu, ¹⁸⁶Re, ¹⁸⁸Re, ²¹¹At, and ²¹²Bi, optionally conjugated to antibodies directed against tumor antigens.

The term "sample" includes sections of tissues such as biopsy and autopsy samples, and frozen sections taken for histological purposes. Such samples include blood and blood fractions or products (e.g., bone marrow, serum, plasma, platelets, red blood cells, and the like) , sputum, tissue, cultured cells (e.g., primary cultures, explants, and transformed cells) , stool, urine, other biological fluids (e.g., prostatic fluid, gastric fluid, intestinal fluid, renal fluid, lung fluid, cerebrospinal fluid, and the like) , etc. A sample is typically obtained from a "subject" such as a eukaryotic organism, most preferably a mammal such as a primate, e.g., chimpanzee or human; cow; dog; cat; a rodent, e.g., guinea pig, rat, mouse; rabbit; or a bird; reptile; or fish. In some embodiments, the sample is obtained from a human.

A "control" sample or value refers to a sample that serves as a reference, usually a known reference, for comparison to a test sample. For example, a test sample can be taken from a test condition, e.g., in the presence of a test compound, and compared to samples from known conditions, e.g., in the absence of the test compound (negative control) , or in the presence of a known compound (positive control) . A control can also represent an average value gathered from a number of tests or results. One of skill in the art will recognize that controls can be designed for assessment of any number of parameters. For example, a control can be devised to compare therapeutic benefit based on pharmacological data (e.g., half-life) or therapeutic measures (e.g., comparison of side effects) . One of skill in the art will understand which controls are valuable in a given situation and be able to analyze data based on comparisons to control values. Controls are also valuable for determining the significance of data. For example, if values for a given parameter are widely variant in controls, variation in test samples will not be considered as significant.

"Disease" or "condition" refer to a state of being or health status of a patient or subject capable of being treated with a compound, pharmaceutical composition, or method provided herein. In embodiments, the disease is cancer (e.g. liver cancer, pancreatic cancer, pancreatic liver metastases, brain cancer, prostate cancer, renal cancer, metastatic cancer, melanoma, castration-resistant prostate cancer, breast cancer, triple negative breast cancer, glioblastoma, ovarian cancer, lung cancer, squamous cell carcinoma (e.g., head, neck, or oesophagus) , colorectal cancer, leukemia, acute myeloid leukemia, lymphoma, B cell lymphoma, or multiple myeloma) , an infectious disease (e.g., HN infection) , an inflammatory disease (e.g., rheumatoid arthritis) or a metabolic disease (e.g., diabetes) . In embodiments, the disease is a disease related to (e.g. caused by) an aberrant activity of TfR, TfR phosphorylation, or TfR pathway activity, or pathway activated by TfR. In some embodiments, the disease is cancer (e.g. prostate cancer, renal cancer, metastatic cancer, melanoma, castration-resistant prostate cancer, breast cancer, triple negative breast cancer, glioblastoma, ovarian cancer, lung cancer, squamous cell carcinoma (e.g., head, neck, or oesophagus) , colorectal cancer, leukemia, acute myeloid leukemia, lymphoma, B cell lymphoma, or multiple myeloma) .

As used herein, the term "cancer" refers to all types of cancer, neoplasm or malignant tumors found in mammals, including leukemia, lymphoma, carcinomas and sarcomas. Exemplary cancers that may be treated with a compound, pharmaceutical composition, or method provided herein include pancreatic cancer, liver cancer (e.g. hepatocellular carcinoma) , pancreatic liver metastases, lymphoma, sarcoma, bladder cancer, bone cancer, brain cancer (e.g. brain tumor, medulloblastoma, glioblastoma, glioblastoma multiforme) , cervical cancer, colon cancer, oesophageal cancer, gastric cancer, head and neck cancer, kidney cancer, myeloma, thyroid cancer, leukemia, prostate cancer, breast cancer (e.g. triple negative, ER positive, ER negative, chemotherapy resistant, herceptin resistant, HER2 positive, doxorubicin resistant, tamoxifen resistant, ductal carcinoma, lobular carcinoma, primary, metastatic) , ovarian cancer, lung cancer (e.g. non-small cell lung carcinoma, squamous cell lung carcinoma, adenocarcinoma, large cell lung carcinoma, small cell lung carcinoma, carcinoid, sarcoma) , glioma, neuroblastoma, melanoma, castration-resistant prostate cancer, squamous cell carcinoma (e.g., head, neck, or esophagus) , colorectal cancer, acute myeloid leukemia, B cell lymphoma, multiple myeloma, Hodgkin's Disease, Non-Hodgkin's Lymphoma, rhabdomyosarcoma, mesothelioma, endometrial cancer, thrombocytosis, Waldenstrom macroglobulinemia (WM) , insulanoma, malignant carcinoid, premalignant skin lesions, testicular cancer, malignant hypercalcemia, adrenal cortical cancer, neoplasms of the endocrine or exocrine pancreas, medullary thyroid cancer, medullary thyroid carcinoma, papillary thyroid cancer, Paget's Disease of the Nipple, Phyllodes Tumors, Lobular Carcinoma, Ductal Carcinoma, cancer of the pancreatic stellate cells, or cancer of the hepatic stellate cells. Additional examples include cancer of the endocrine system, brain, breast, bone, cervix, colon, head & neck, oesophagus, liver, kidney, lung, non-small cell lung, ovary, stomach, mouth, skin, uterus, endometrium, pancreas, thyroid, bladder, prostate, testicle or genitourinary tract.

The term "leukemia" refers broadly to progressive, malignant diseases of the blood-forming organs and is generally characterized by a distorted proliferation and development of leukocytes and their precursors in the blood and bone marrow. Leukemia is generally clinically classified on the basis of (1) the duration and character of the disease-acute or chronic; (2) the type of cell involved; myeloid (myelogenous) , lymphoid (lymphogenous) , or monocytic; and (3) the increase or non-increase in the number abnormal cells in the blood-leukemic or aleukemic (subleukemic) . Exemplary leukemias that may be treated with a compound, pharmaceutical composition, or method provided herein include, for example, acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairycell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micro myeloblastic leukemia, monocytic leukemia, myeloblastic leukemia, myelocytic leukemia, myeloid granulocytic leukemia, myelomonocytic leukemia, Naegeli leukemia, plasma cell leukemia, multiple myeloma, plasmacytic leukemia, promyelocytic leukemia, Rieder cell leukemia, Schilling's leukemia, stem cell leukemia, subleukemic leukemia, or undifferentiated cell leukemia.

The term "sarcoma" generally refers to a tumor which is made up of a substance like the embryonic connective tissue and is generally composed of closely packed cells embedded in a fibrillar or homogeneous substance. Sarcomas that may be treated with a compound, pharmaceutical composition, or method provided herein include a chondrosarcoma, fibrosarcoma, lymphosarcoma, melanosarcoma, myxosarcoma, osteosarcoma, Abemethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, or telangiectaltic sarcoma.

The term "melanoma" is taken to mean a tumor arising from the melanocytic system of the skin and other organs. Melanomas that may be treated with a compound, pharmaceutical composition, or method provided herein include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, or superficial spreading melanoma.

The term "carcinoma" refers to a malignant new growth made up of epithelial cells tending to infiltrate the surrounding tissues and give rise to metastases. Exemplary carcinomas that may be treated with a compound, pharmaceutical composition, or method provided herein include, for example, medullary thyroid carcinoma, familial medullary thyroid carcinoma, acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, ductal carcinoma, carcinoma durum, embryonal carcinoma, encephaloid carcinoma, epiermoid carcinoma, carcinoma epitheliale adenoides, exophytic carcinoma, carcinoma ex ulcere, carcinoma fibrosum, gelatiniforni carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypernephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lobular carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticum, carcinoma telangiectodes, transitional cell carcinoma, carcinoma tuberosum, tubular carcinoma, tuberous carcinoma, verrucous carcinoma, or carcinoma villosum.

As used herein, the terms "metastasis," "metastatic," and "metastatic cancer" can be used interchangeably and refer to the spread of a proliferative disease or disorder, e.g., cancer, from one organ or another non-adjacent organ or body part. Cancer occurs at an originating site, e.g., breast, which site is referred to as a primary tumor, e.g., primary breast cancer. Some cancer cells in the primary tumor or originating site acquire the ability to penetrate and infiltrate surrounding normal tissue in the local area and/or the ability to penetrate the walls of the lymphatic system or vascular system circulating through the system to other sites and tissues in the body. A second clinically detectable tumor formed from cancer cells of a primary tumor is referred to as a metastatic or secondary tumor. When cancer cells metastasize, the metastatic tumor and its cells are presumed to be similar to those of the original tumor. Thus, if lung cancer metastasizes to the breast, the secondary tumor at the site of the breast consists of abnormal lung cells and not abnormal breast cells. The secondary tumor in the breast is referred to a metastatic lung cancer. Thus, the phrase metastatic cancer refers to a disease in which a subject has or had a primary tumor and has one or more secondary tumors. The phrases non-metastatic cancer or subjects with cancer that is not metastatic refers to diseases in which subjects have a primary tumor but not one or more secondary tumors. For example, metastatic lung cancer refers to a disease in a subject with or with a history of a primary lung tumor and with one or more secondary tumors at a second location or multiple locations, e.g., in the breast.

The term "associated" or "associated with" in the context of a substance or substance activity or function associated with a disease (e.g., diabetes, cancer (e.g. prostate cancer, renal cancer, metastatic cancer, melanoma, castration-resistant prostate cancer, breast cancer, triple negative breast cancer, glioblastoma, ovarian cancer, lung cancer, squamous cell carcinoma (e.g., head, neck, or esophagus) , colorectal cancer, leukemia, acute myeloid leukemia, lymphoma, B cell lymphoma, or multiple myeloma)) means that the disease (e.g., diabetes, cancer (e.g. prostate cancer, renal cancer, metastatic cancer, melanoma, castration-resistant prostate cancer, breast cancer, triple negative breast cancer, glioblastoma, ovarian cancer, lung cancer, squamous cell carcinoma (e.g., head, neck, or esophagus) , colorectal cancer, leukemia, acute myeloid leukemia, lymphoma, B cell lymphoma, or multiple myeloma) or viral disease (e.g., HN infection associated disease) ) is caused by (in whole or in part) , or a symptom of the disease is caused by (in whole or in part) the substance or substance activity or function.

The term "aberrant" as used herein refers to different from normal. When used to describe enzymatic activity, aberrant refers to activity that is greater or less than a normal control or the average of normal non-diseased control samples. Aberrant activity may refer to an amount of activity that results in a disease, wherein returning the aberrant activity to a normal or non-disease-associated amount (e.g. by using a method as described herein) , results in reduction of the disease or one or more disease symptoms.

"Contacting" is used in accordance with its plain ordinary meaning and refers to the process of allowing at least two distinct species (e.g. chemical compounds including biomolecules, or cells) to become sufficiently proximal to react, interact or physically touch. It should be appreciated, however, that the resulting reaction product can be produced directly from a reaction between the added reagents or from an intermediate from one or more of the added reagents which can be produced in the reaction mixture. Contacting may include allowing two species to react, interact, or physically touch, wherein the two species may be a nucleic acid compound as described herein and a cell (e.g., cancer cell) .

### Ribonucleic acid compounds

The present invention relates to ribonucleic acid aptamer-siRNA fusion compounds.

The term "aptamer" as provided herein refers to oligonucleotides (e.g. short oligonucleotides or deoxyribonucleotides) , that bind (e.g. with high affinity and specificity) to proteins, peptides, and small molecules. Aptamers typically have defined secondary or tertiary structure owing to their propensity to form complementary base pairs and, thus, are often able to fold into diverse and intricate molecular structures. The three-dimensional structures are essential for aptamer binding affinity and specificity, and specific three-dimensional interactions drives the formation of aptamer-target complexes. Aptamers can be selected *in vitro* from very large libraries of randomized sequences by the process of systemic evolution of ligands by exponential enrichment (SELEX as described in Ellington AD, Szostak JW (1990) In vitro selection of RNA molecules that bind specific ligands. Nature 346:818-822; Tuerk C, Gold L (1990) Systematic evolution of ligands by exponential enrichment: RNA ligands to bacteriophage T4 DNA polymerase. Science 249:505-510) or by developing SOMAmers (slow off-rate modified aptamers) (Gold L et al. (2010) Aptamer-based multiplexed proteomic technology for biomarker discovery. PLoS ONE 5(12) :e15004) . Applying the SELEX and the SOMAmer technology includes for instance adding functional groups that mimic amino acid side chains to expand the aptamer's chemical diversity. As a result high affinity aptamers for almost any protein target are enriched and identified. Aptamers exhibit many desirable properties for targeted drug delivery, such as ease of selection and synthesis, high binding affinity and specificity, flexible structure, low immunogenicity, and versatile synthetic accessibility.

The present invention provides ribonucleic acid compounds comprising: (i) an aptamer capable of binding to transferrin receptor (TfR) , and (ii) a CCAAT/enhancer-binding protein β (C/EBPβ) siRNA. The ribonucleic acid compounds are *inter alia* capable of binding a transferrin receptor (TfR) . In preferred embodiments, the TfR is on a cell and, in some cases, the ribonucleic acid compounds are internalised into the cell.

The term "TfR" as provided herein includes any of the transferrin receptor (TfR) protein naturally occurring forms, homologs or variants that maintain the activity of TfR (e.g., within at least 50%, 80%, 90%, 95%, 96%, 97%, 98%, 99% or 100% activity compared to the native protein) . In some embodiments, variants or homologs have at least 90%, 95%, 96%, 97%, 98%, 99% or 100% amino acid sequence identity across the whole sequence or a portion of the sequence (e.g. a 50, 100, 150 or 200 continuous amino acid portion) compared to a naturally occurring form. In embodiments, the TfR protein is the protein as identified by the NCBI sequence reference GI:189458817 (NCBI Reference Sequence: NP_003225.2; SEQ ID NO:6) . In embodiments, the TfR protein is the protein as encoded by the nucleotide sequence identified by the NCBI sequence reference GI:189458816 (NCBI Reference Sequence: NM_003234.3) . In embodiments, the TfR protein is the protein as encoded by the nucleotide sequence identified by the NCBI sequence reference GI:189458818 (NCBI Reference Sequence: NM_001128148.2) . In embodiments, the TfR protein is the protein as identified by the NCBI sequence reference GI:189458817 (NCBI Reference Sequence: NP_003225.2; SEQ ID NO:6) , homolog or functional fragment thereof. In embodiments, the TfR protein is the protein as encoded by the nucleotide sequence identified by the NCBI sequence reference GI:189458816 (NCBI Reference Sequence: NM_003234.3) , homolog or functional fragment thereof. In embodiments, the TfR protein is the protein as encoded by the nucleotide sequence identified by the NCBI sequence reference GI:189458818 (NCBI Reference Sequence: NM_001128148.2) , homolog or functional fragment thereof. In embodiments, the TfR protein is encoded by a nucleic acid sequence corresponding to NCBI Gene ID: 7037.

TfR is expressed at low levels on normal cells. Cells with high-proliferation rates, such as activated immune cells and cancers, present upregulated expression of TfR. The ribonucleic acid compounds of the present invention thus provide a mechanism to deliver CCAAT/enhancer-binding protein β (C/EBPβ) siRNA to a broad variety of cells via TfR binding through the aptamer portion. The ribonucleic acid compounds may be internalised into TfR-expressing cells, thus providing an efficient mechanism for targeted intracellular delivery.

WO 2016/061386 describes ribonucleic acid compounds that are capable of binding TfR. The ribonucleic acid compounds in WO 2016/061386 comprise RNA sequences having at least 30 nucleotides and are exemplified by compounds comprising RNA sequences that are 87 or 43 nucleotides in length. The three-dimensional structure of a ribonucleic acid compound, e.g. an aptamer, is essential for determining binding affinity and specificity.

Thus, the ribonucleic acid compounds of the present invention provide highly specific and efficient means for targeted delivery of CCAAT/enhancer-binding protein β (C/EBPβ) siRNA to TfR-expressing cancers, as shown herein.

In some aspects, aptamer capable of binding TfR comprises, or consists of, an RNA sequence having at least 80% sequence identity to SEQ ID NO:1 or 40, and wherein the RNA sequence has a length of 29 nucleotides or fewer.

In some embodiments the aptamer RNA sequence has at least 85%, at least 87%, at least 90%, at least 91%, at least 92%, at least 93%, at least 94%, at least 95%, at least 96%, at least 97%, at least 98% or at least 99% sequence identity to SEQ ID NO:1 or 40. In some embodiments, the aptamer RNA sequence has at least 90% sequence identity to SEQ ID NO:1 or 40. In some embodiments, the aptamer RNA sequence has 100% sequence identity to SEQ ID NO:1 or 40. In some embodiments, the aptamer RNA sequence consists of SEQ ID NO:1 or 40. In some embodiments, the aptamer RNA sequence binds to a transferrin receptor (TfR) . In some embodiments, the TfR is on a cell surface. In some embodiments, the nucleic acid compound is capable of being internalised into a cell. In some cases, the cell is a TfR-expressing cell.

In some embodiments the aptamer RNA sequence has at least 80% sequence identity to a nucleic acid that hybridises to SEQ ID NO:1 or 40. In some cases the aptamer RNA sequence has at least 85%, at least 87%, at least 90%, at least 91%, at least 92%, at least 93%, at least 94%, at least 95%, at least 96%, at least 97%, at least 98%, at least 99%, or at least 100% sequence identity to a nucleic acid that hybridises to SEQ ID NO:1 or 40.

SEQ ID NO:40 corresponds to a 2' ESC substituted variant of SEQ ID NO:1. Where "SEQ ID NO:1" is referred to throughout this description, unless otherwise stated it may be substituted for "SEQ ID NO:40". In some aspects, the aptamer capable of binding TfR comprises, or consists of, an aptamer RNA sequence having at least 80% sequence identity to SEQ ID NO:5, and wherein the aptamer RNA sequence has a length of 29 nucleotides or fewer.

In some embodiments the aptamer RNA sequence has at least 85%, at least 87%, at least 90%, at least 91%, at least 92%, at least 93%, at least 94%, at least 95%, at least 96%, at least 97%, at least 98% or at least 99% sequence identity to SEQ ID NO:5. In some embodiments, the aptamer RNA sequence has at least 90% sequence identity to SEQ ID NO:5. In some embodiments, the aptamer RNA sequence has 100% sequence identity to SEQ ID NO:5. In some embodiments, the aptamer RNA sequence consists of SEQ ID NO:5. In some embodiments, the aptamer RNA sequence binds to a transferrin receptor (TfR) . In some embodiments, the TfR is on a cell surface. In some embodiments, the nucleic acid compound is capable of being internalised into a cell. In some cases, the cell is a TfR-expressing cell.

In some cases the aptamer RNA sequence has at least 80% sequence identity to a nucleic acid that hybridises to SEQ ID NO:5. In some cases the aptamer RNA sequence has at least 85%, at least 87%, at least 90%, at least 91%, at least 92%, at least 93%, at least 94%, at least 95%, at least 96%, at least 97%, at least 98%, at least 99%, or at least 100% sequence identity to a nucleic acid that hybridises to SEQ ID NO:5.

In some embodiments an aptamer RNA sequence provided herein has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer. In some cases the aptamer RNA sequence has a length of 22 nucleotides or fewer. In some embodiments the aptamer RNA sequence is between 16 and 29 nucleotides in length. In some embodiments the aptamer RNA sequence is between 16 and 22 nucleotides in length.

In some embodiments the aptamer RNA sequence is 16 nucleotides in length. In some embodiments the aptamer RNA sequence is 17 nucleotides in length. In some embodiments the aptamer RNA sequence is 18 nucleotides in length. In some embodiments the aptamer RNA sequence is 19 nucleotides in length. In some embodiments the aptamer RNA sequence is 20 nucleotides in length. In some embodiments the aptamer RNA sequence is 21 nucleotides in length. In some embodiments the aptamer RNA sequence is 22 nucleotides in length. In some embodiments the aptamer RNA sequence is 23 nucleotides in length. In some embodiments the aptamer RNA sequence is 24 nucleotides in length. In some embodiments the aptamer RNA sequence is 25 nucleotides in length. In some embodiments the aptamer RNA sequence is 26 nucleotides in length. In some embodiments the aptamer RNA sequence is 27 nucleotides in length. In some embodiments the aptamer RNA sequence is 28 nucleotides in length. In some embodiments the aptamer RNA sequence is 29 nucleotides in length.

In some cases, the aptamer RNA sequence has at least 80% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some cases, the aptamer RNA sequence has at least 85% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some cases, the aptamer RNA sequence has at least 87% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some cases, the aptamer RNA sequence has at least 90% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some cases, the aptamer RNA sequence has at least 91% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some cases, the aptamer RNA sequence has at least 92% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some cases, the aptamer RNA sequence has at least 93% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some cases, the aptamer RNA sequence has at least 94% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some cases, the aptamer RNA sequence has at least 95% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some cases, the aptamer RNA sequence has at least 96% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some cases, the aptamer RNA sequence has at least 97% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some cases, the aptamer RNA sequence has at least 98% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some cases, the aptamer RNA sequence has at least 99% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some cases, the aptamer RNA sequence has at least 100% sequence identity to SEQ ID NO:1 or SEQ ID NO:5 and has a length of 29 nucleotides or fewer, 28 nucleotides or fewer, 27 nucleotides or fewer, 26 nucleotides or fewer, 25 nucleotides or fewer, 24 nucleotides or fewer, 23 nucleotides or fewer, 22 nucleotides or fewer, 21 nucleotides or fewer, 20 nucleotides or fewer, 19 nucleotides or fewer, 18 nucleotides or fewer, 17 nucleotides or fewer, or 16 nucleotides or fewer.

In some embodiments, an aptamer RNA sequence provided herein comprises or consists of SEQ ID NO:1 and further comprises 1, 2, 3, 4 or more nucleotide modifications to SEQ ID NO:1. Such modifications may be nucleotide additions, substitutions, and/or deletions.

In some cases, an aptamer RNA sequence provided herein differs by 1, 2, 3, or 4 nucleotides compared to SEQ ID NO:1.

In some embodiments, an aptamer RNA sequence comprises SEQ ID NO:1 wherein one or more of positions 1, 2, 3, 20, 21 and/or 22 are substituted for alternative nucleic acid residues, for example AMP, GMP, UMP, CMP, dAMP, dGMP, dTMP, and/or dCMP. That is, in some embodiments, the aptamer RNA sequence comprises SEQ ID NO:5 and 3 additional nucleotides at each of the 5' end and 3' end.

In some embodiments, the aptamer RNA sequence comprises or consists of SEQ ID NO:5 and further comprises 1, 2, 3, 4 or more nucleotide modifications to SEQ ID NO:5. Such modifications may be nucleotide additions, substitutions, and/or deletions.

In some cases, an aptamer RNA sequence provided herein differs by 1, 2, 3, or 4 nucleotides compared to SEQ ID NO:5.

Nucleotide positions 4 to 8 of SEQ ID NO:1 are predicted to form base pairing with nucleotide positions 15 to 19. Nucleotide positions 1 to 5 of SEQ ID NO:5 are predicted to form base pairing with nucleotide positions 12 to 16. In some embodiments, an aptamer RNA sequence comprises SEQ ID NO:1, wherein nucleotides at any i.e. one or more of positions 4 to 8 and/or 15 to 19 are substituted with nucleotide residues that result in non-canonical base pairing. In some embodiments, an aptamer RNA sequence comprises SEQ ID NO:5, wherein nucleotides at any i.e. one or more of positions 1 to 5 and/or 12 to 16 are substituted with nucleotide residues that result in non-canonical base pairing. For example, A or U/T may be replaced with C or G and vice versa. In some cases, an U-A pairing may be replaced with U-G.

In some embodiments, the aptamer RNA sequence has at least 85% sequence identity to any of SEQ ID NOs 1, 2, 3, 4 or 5.

In some aspects, aptamer capable of binding TfR comprises, or consists of, an aptamer RNA sequence having at least 85% sequence identity to SEQ ID NO:20, wherein said nucleic acid sequence is at least 30 nucleotides in length and at most 50 nucleotides in length. In some embodiments, the nucleic acid sequence is SEQ ID NO: 19 or 20.

In some embodiments, the aptamer RNA sequence has at least 85% sequence identity to SEQ ID NO:20, wherein said aptamer RNA sequence is at least 30 nucleotides in length and at most 42 nucleotides in length.

In some embodiments, the aptamer RNA sequence is 32 nucleotides in length and preferably has at least 85% sequence identity to SEQ ID NO:20. In some embodiments, the aptamer RNA sequence has 100% sequence identity to SEQ ID NO:20.

In some embodiments, the nucleic acid sequence has at least 85%, at least 86%, at least 87%, at least 88%, at least 89%, at least 90%, at least 91%, at least 92%, at least 93%, at least 94%, at least 95%, at least 96%, at least 97%, at least 98% or at least 99% sequence identity to SEQ ID NO:20. In some embodiments, the nucleic acid sequence has 85%, 86%, 87%, 88%, 89%, 90%, 91%, 92%, 93%, 94%, 95%, 96%, 97%, 98%, 99% or 100% sequence identity to SEQ ID NO:5. In some embodiments, the nucleic acid sequence consists of SEQ ID NO:20.

In some embodiments, the nucleic acid sequence hybridises with a SEQ ID NO:20. In some embodiments, the nucleic acid sequence hybridises with a sequence which has at least 85%, at least 86%, at least 87%, at least 88%, at least 89%, at least 90%, at least 91%, at least 92%, at least 93%, at least 94%, at least 95%, at least 96%, at least 97%, at least 98% or at least 99% sequence identity to SEQ ID NO:20. In some embodiments, the nucleic acid sequence hybridises with a sequence which has 85%, 86%, 87%, 88%, 89%, 90%, 91%, 92%, 93%, 94%, 95%, 96%, 97%, 98%, 99% or 100% sequence identity to SEQ ID NO:20.

In some embodiments, the nucleic acid sequence is 30, 31, 32, 33, 34, 35, 36, 37, 38, 39, 40, 41, or 42 nucleotides in length. In some embodiments, the nucleic acid sequence is 44, 45, 46, 47, 48, 49 or 50 nucleotides in length. In some embodiments, the nucleic acid sequence is less than 43 nucleotides in length. In some embodiments, the nucleic acid sequence is more than 43 nucleotides in length.

In some embodiments, the ribonucleic acid compound comprises, or consists of a nucleic acid sequence that is at least 30 and at most 46 nucleotides in length. In some embodiments, the ribonucleic acid compound comprises, or consists of a nucleic acid sequence that is at least 44 and at most 50 nucleotides in length. In some embodiments, the ribonucleic acid compound comprises, or consists of a nucleic acid sequence that is at least 44 and at most 46 nucleotides in length. In some embodiments, the ribonucleic acid compound comprises, or consists of a nucleic acid sequence having a length of 46 nucleotides. In some embodiments, the ribonucleic acid compound comprises, or consists of a nucleic acid sequence that is at least 30 and at most 40 nucleotides in length. In some embodiments, the ribonucleic acid compound comprises, or consists of a nucleic acid sequence that is at least 32 and at most 40 nucleotides in length. In some embodiments, the ribonucleic acid compound comprises, or consists of a nucleic acid sequence that is at least 35 and at most 40 nucleotides in length. In some embodiments, the ribonucleic acid compound comprises, or consists of a nucleic acid sequence that is at least 30 and at most 35 nucleotides in length. In some embodiments, the ribonucleic acid compound comprises, or consists of a nucleic acid sequence that is at least 30 and at most 32 nucleotides in length. In some embodiments, the ribonucleic acid compound comprises, or consists of a nucleic acid sequence having a length of 40 nucleotides. In some embodiments, the ribonucleic acid compound comprises, or consists of a nucleic acid sequence having a length of 32 nucleotides.

In some embodiments, the nucleic acid sequence is from 23 to 50, from 23 to 46, from 23 to 42, from 23 to 32, from 30 to 50, from 30 to 46, from 30 to 42, from 30 to 35, from 32 to 42, from 32 to 46, from 32 to 50, from 40 to 42, from 44 to 46, from 44 to 50, or from 46 to 50 nucleotides in length.

In some embodiments, an aptamer RNA sequence provided herein comprises or consists of SEQ ID NO: 19 or 20, and further comprises 1, 2, 3, 4 or more nucleotide modifications to SEQ ID NO: 19 or 20. Such modifications may be nucleotide additions, substitutions, and/or deletions.

In some cases, an aptamer RNA sequence provided herein differs by 1, 2, 3, or 4 nucleotides compared to SEQ ID NO: 19 or 20.

In some embodiments, an aptamer RNA sequence comprises SEQ ID NO: 19 or 20 wherein one or more of positions 1, 2, 3, 20, 21 and/or 22 are substituted for alternative nucleic acid residues, for example AMP, GMP, UMP, CMP, dAMP, dGMP, dTMP, and/or dCMP. That is, in some embodiments, the aptamer RNA sequence comprises SEQ ID NO:5 and 3 additional nucleotides at each of the 5' end and 3' end.

In some aspects, the aptamer capable of binding TfR comprises, or consists of, an aptamer RNA sequence having at least 80% sequence identity to SEQ ID NO:2, 3, 21 or 22, and wherein the aptamer RNA sequence is at least 40 nucleotides in length. In embodiments, the aptamer capable of binding TfR comprises, or consists of, an aptamer RNA sequence having at least 80% sequence identity to SEQ ID NO:2, or 3, , and wherein the aptamer RNA sequence is at least 40 nucleotides in length. In embodiments, the aptamer capable of binding TfR comprises, or consists of, an aptamer RNA sequence having at least 80% sequence identity to SEQ ID NO:2, 3 or 22, and wherein the aptamer RNA sequence is at least 40 nucleotides in length. In embodiments, the aptamer capable of binding TfR comprises, or consists of, an aptamer RNA sequence having at least 80% sequence identity to SEQ ID NO:2 or 3. and wherein the aptamer RNA sequence is at least 40 nucleotides in length.

Where the aptamer RNA sequence has at least 80% (80% or more) sequence identity to SEQ ID NO:2, 3, 21 or 22, the aptamer RNA sequence may have 80%, 85%, 90%, 91%, 92%, 93%, 94%, 95%, 96%, 97%, 98%, 99% or 100% sequence identity to SEQ ID NO:2, 3, 21 or 22.

In some embodiments, the aptamer RNA sequence has at least 80% (80% or more) sequence identity to a nucleic acid that hybridizes to a sequence selected from SEQ ID NO:2, 3, 21 or 22, and is at least 40 nucleotides in length. In some embodiments, the aptamer RNA sequence has 80%, 85%, 90%, 91%, 92%, 93%, 94%, 95%, 96%, 97%, 98%, 99% or 100% sequence identity to a nucleic acid that hybridises to a sequence selected from SEQ ID NO:2, 3, 21 or 22. In some embodiments, the aptamer RNA sequence hybridizes to a sequence that has 80%, 85%, 90%, 91%, 92%, 93%, 94%, 95%, 96%, 97%, 98%, 99% or 100% sequence identity to SEQ ID NO:2, 3, 21 or 22 and which is at least 40 nucleotides in length.

Where the aptamer RNA sequence is at least 50 (50 nucleotides or more) nucleotides in length, the aptamer RNA sequence may be at least 50, 55, 60, 65, 70, 75, 80, 85, 90, 95, 100, 110, 120, 130, 140, 150, 160, 170, 180, 190 or 200 nucleotides in length. In some embodiments, the aptamer RNA sequence is at least 50 nucleotides in length. Where the aptamer RNA sequence is at least 40 (40 nucleotides or more) nucleotides in length, the aptamer RNA sequence is at least 40, 41, 42, 43, 44, 45, 45, 47, 48, 49, 50, 55, 60, 65, 70, 75, 80, 85, 90, 95, 100, 110, 120, 130, 140, 150, 160, 170, 180, 190 or 200 nucleotides in length. Thus, in some embodiments, the aptamer RNA sequence is about 43 nucleotides in length.

In some embodiments, an aptamer RNA sequence provided herein comprises or consists of SEQ ID NO: 2, 3 or 21, and further comprises 1, 2, 3, 4 or more nucleotide modifications to SEQ ID NO: 2, 3 or 21. Such modifications may be nucleotide additions, substitutions, and/or deletions.

In some cases, an aptamer RNA sequence provided herein differs by 1, 2, 3, or 4 nucleotides compared to SEQ ID NO: 2, 3 or 21.

In some embodiments, an aptamer RNA sequence comprises SEQ ID NO: 2, 3 or 21 wherein one or more of positions 1, 2, 3, 20, 21 and/or 22 are substituted for alternative nucleic acid residues, for example AMP, GMP, UMP, CMP, dAMP, dGMP, dTMP, and/or dCMP. That is, in some embodiments, the aptamer RNA sequence comprises SEQ ID NO:5 and 3 additional nucleotides at each of the 5' end and 3' end.

Any RNA sequence as described herein may further comprise additional nucleotides. Additional nucleotides may be added onto the 5' end, the 3' end, or both the 5' and 3' ends of the RNA sequence. In some embodiments, an aptamer RNA sequence comprising SEQ ID NO:1, 2, 3, 5, 20 or 21 as described herein comprises a total of 1, 2, 3, 4, 5, 6, 7, 8, 9, 10, 11, 12, 13 or more additional nucleotides. Where an aptamer RNA sequence comprises SEQ ID NO:1, the aptamer RNA sequence may comprise a total of 7 or fewer additional nucleotides. Where an aptamer RNA sequence comprises SEQ ID NO:5, the aptamer RNA sequence may comprise a total of 13 or fewer additional nucleotides.

In any embodiments described herein, the aptamer RNA sequence may be an aptamer. In any embodiment described herein, the nucleic acid is capable of binding to a transferrin receptor (TfR) . In any embodiments described herein, the aptamer RNA sequence is capable of binding to a transferrin receptor (TfR) . Thus, an aptamer RNA sequence provided herein may have at least 80% (at least 85% etc, as described hereinabove) sequence identity to SEQ ID NO:1 or SEQ ID NO:5 and a length of 16 to 29 nucleotides (as described hereinabove) , and wherein the aptamer RNA sequence is capable of binding to a transferrin receptor (TfR) . In any embodiment, the nucleic acid compound binds to TfR. In any embodiment, the aptamer RNA sequence binds to TfR. In any embodiment, the TfR is on a cell surface. In any embodiment, the nucleic acid compound is capable of being internalised into a cell. In any embodiment, the cell is a TfR-expressing cell.

In some embodiments, the ribonucleic acid compound has a 5'-GGG motif at the 5' end of the aptamer RNA sequence. Without wishing to be bound by theory, a 5'-GGG motif at the 5' end of the aptamer RNA sequence may increase the extent to which the ribonucleic acid compound is internalised into a cell. A 5'-GGG motif may be sufficient, if not necessary, for internalisation into a cell. In some embodiments, the ribonucleic acid compound does not have a 5'-GGG motif at the 5' end of the RNA sequence.

In all embodiments of any of the aspects of the invention, the ribonucleic acid compound sequence is capable of binding to a transferrin receptor (TfR) via the aptamer RNA sequence. In some embodiments, the aptamer RNA sequence binds to a transferrin receptor (TfR) . In some embodiments, the TfR is on a cell surface. In some embodiments, the ribonucleic acid compound is capable of being internalised into a cell. In some cases, the cell is a TfR-expressing cell.

The term "on a cell surface" as used herein refers to the location of a molecule, e.g. transferrin receptor protein, on the surface of a cell. The molecule may be associated in some way with the cell membrane. For example, the molecule may be an integral or transmembrane protein which spans the cell membrane and comprises a cytosolic domain and an extracellular domain, the molecule may be lipid anchored i.e. covalently bound to single or multiple lipid molecules in the cell membrane, or the molecule may be attached to an integral membrane protein.

The term "capable of being internalised into a cell" as used herein refers to the ability of a nucleic molecule of the present invention to be transported from the outside of a cell into a cell. This may be performed by cellular mechanisms such as endocytosis or phagocytosis. In some cases, the nucleic acids are internalised after they bind to TfR, for example by clathrin-mediated endocytosis of a TfR-nucleic acid complex (see e.g. Qian ZM et al., Pharmacol Rev. 2002, 54(4) :561-587) .

A "TfR-expressing cell" is a cell which produces TfR and displays TfR on the cell surface, e.g. as a membrane protein.

In some embodiments, the aptamer RNA sequence has an equilibrium dissociation constant (K^{D}) for TfR of less than about 1×10⁻⁸, 1×10⁻⁹, 1×10⁻¹⁰, 1×10⁻¹¹, or less than 1×10⁻¹² M. In some embodiments, the aptamer RNA sequence has K^{D} for TfR of between 1×10⁻⁸ and 1×10⁻¹³ M, between 1×10⁻⁹ and 1×10⁻¹³ M, between 1×10⁻¹⁰ and 1×10⁻¹³ M, between 1×10⁻¹¹ and 1×10⁻¹³ M, or between 1×10⁻¹² and 1×10⁻¹³ M. In some embodiments, the aptamer RNA sequence has a K^{D} for TfR of between 1 and 5 ×10⁻¹⁰ M, of between 1 and 5 ×10⁻¹¹ M, or of between 5 ×10⁻¹³ and 1 ×10⁻¹² M. In some embodiments, the aptamer RNA sequence has a K^{D} for TfR of between 1 ×10⁻⁹ and 1 ×10⁻¹⁰ M, 1 ×10⁻¹⁰ and 1 ×10⁻¹¹ M, or of 1 ×10⁻¹² and 1 ×10⁻¹³ M. In some embodiments, the aptamer RNA sequence has a higher affinity for TfR than TR14.

In some embodiments, the aptamer RNA sequence is capable of binding to both TfR1 and TfR2.

The ribonucleic acid molecules of the invention comprise a CCAAT/enhancer-binding protein β (C/EBPβ) siRNA.

As used herein, a "siRNA," "small interfering RNA," "small RNA," or "RNAi", refers to a nucleic acid that forms a double stranded RNA, which double stranded RNA has the ability to reduce or inhibit expression of a gene or target gene when expressed in the same cell as the gene or target gene. The complementary portions of the nucleic acid that hybridize to form the double stranded molecule typically have substantial or complete identity. Typically, the siRNA technique uses a synthetic double-stranded nucleic acid duplex, in which each strand is from 19-23 nucleotides in length, with 2 nucleotides in each strand forming a 3' overhang. These 3' overhangs may be RNA or DNA whereas the double-stranded region is entirely RNA. Typically, the 3' overhangs are symmetrical and have the sequence 5'-UU-3' or 5'-UG-3' (5'-TT-3' or 5'-TG-3' if the 3' overhang is DNA) . In one embodiment, a siRNA or RNAi is a nucleic acid that has substantial or complete identity to a target gene and forms a double stranded siRNA. In embodiments, the siRNA inhibits gene expression by interacting with a complementary cellular mRNA thereby interfering with the expression of the complementary mRNA. Typically, the nucleic acid is at least about 15-50 nucleotides in length (e.g., each complementary sequence of the double stranded siRNA is 15-50 nucleotides in length, and the double stranded siRNA is about 15-50 base pairs in length) . In other embodiments, the length is 20-30 base nucleotides, preferably about 20-25 or about 24-30 nucleotides in length, e.g., 20, 21, 22, 23, 24, 25, 26, 27, 28, 29, or 30 nucleotides in length. The processes for the generation of siRNAs is well-known to the skilled person.

The term "C/EBPb", "C/EBPβ" or "C/EBPbeta" as provided herein includes any of the CCAAT (cytosine-cytosine-adenosine-adensoine-thymidine) /enhancer-binding protein beta (C/EBPb) naturally occurring forms, homologs or variants that maintain the transcription factor activity of C/EBPbeta (e.g., within at least 50%, 80%, 90%, 95%, 96%, 97%, 98%, 99% or 100% activity compared to the native protein) . In some embodiments, variants or homologs have at least 90%, 95%, 96%, 97%, 98%, 99% or 100% amino acid sequence identity across the whole sequence or a portion of the sequence (e.g. a 50, 100, 150 or 200 continuous amino acid portion) compared to a naturally occurring form. In embodiments, the C/EBPbeta protein is the protein as identified by the NCBI sequence reference NP_001272807.1, NP_001272807.1, Gl: 6753404 (NP_034013.1) , Gl: 567757572 (NP_001274668.1) , Gl: 567757569 (NP_001274667.1) , or homolog or functional fragment thereof. In embodiments, the C/EBPbeta protein is encoded by a nucleic acid sequence corresponding to Gene ID:1051.

A C/EBPβ siRNA is able to bind and suppress the expression and/or expression of a C/EBPβ mRNA. The C/EBPβ mRNA may comprise or consist of a sequence having 70%, 80%, 90%, 95% or 100% sequence identity to SEQ ID NO:23.

The C/EBPβ siRNA may comprise multiple nucleic acid sequences as described herein. In some embodiments, the C/EBPβ siRNA comprises two or more nucleic acid sequences separated by a linker, preferably a cleavable linker.

The C/EBPβ siRNA is capable of reducing or inhibiting expression of C/EBPβ mRNA within a cell. The siRNA is capable, when suitably introduced into or expressed within a mammalian cell that otherwise expresses C/EBPβ, of suppressing C/EBPβ expression by RNAi. In some embodiments, the cell is a TfR expressing cell, preferably a cell which expresses TfR on its cell surface. In some embodiments, the C/EBPβ siRNA is capable, when suitably introduced into or expressed within a mammalian cell that otherwise expresses C/EBPβ, of reducing or inhibiting expression of C/EBPβ mRNA to a level 50% or less, 45% or less, 40% or less, 35% or less, 30% or less, 25% or less, 20% or less, 15% or less, or 10% or less of that observed in a cell which differs solely in that the siRNA is not present.

The C/EBPβ siRNA may be covalently attached to the aptamer. In some embodiments, the C/EBPβ siRNA is attached to the aptamer by a linker. As used herein, a "linker" is any structure which connects two moieties in a ribonucleic acid molecule. A linker may comprise one or more nucleotides, in which case it may be referred to as a "nucleotide spacer". However, the skilled person will appreciate that a variety of linkers are available in the art.

In some embodiments, the linker comprises or consists of a cleavable linker. In some embodiments, the cleavable linker is cleaved within or by a component located within a cell. Advantageously, this allows the release of the C/EBPβ siRNA from the aptamer once the ribonucleic acid construct of the invention has undergone uptake into a cell. An exemplary cleavable linker is a disulphide bridge (SS linker), for example the disulphide bridge introduced through use of a 5'-Thiol C-6 Disulfide Modifier CED phosphoramidite (iSpC3iSpC3C6-SS-C6iSpC3iSpC3).

In some embodiments, the C/EBPβ siRNA comprises a nucleic acid sequence with at least 80%, at least 85%, at least 90%, or at least 95% sequence identity to a sequence selected from SEQ ID NOs 7, 9, 11 and 13, and which is capable of reducing or inhibiting expression of C/EBPβ mRNA within a cell. In some embodiments, the C/EBPβ siRNA comprises a nucleic acid sequence comprising no more than 3, 2 or 1 substitutions relative to SEQ ID NOs: 7, 9, 11 and 13. In some embodiments, the C/EBPβ siRNA comprises a nucleic acid sequence having a continuous 18, 19 or 20 amino acids of any one of SEQ ID NOs: 7, 9, 11 and 13. In some embodiments, the C/EBPβ siRNA comprises a nucleic acid sequence having amino acids 1-18 of any one of SEQ ID NOs: 7, 9, 11 and 13. In some embodiments, the C/EBPβ siRNA comprises a nucleic acid sequence having amino acids 1-19 of any one of SEQ ID NOs: 7, 9, 11 and 13. In some embodiments, the C/EBPβ siRNA comprises a nucleic acid sequence selected from SEQ ID NOs 7, 9, 11 and 13.

In some embodiments, the C/EBPβ siRNA comprises a nucleic acid sequence with at least 80%, at least 85%, at least 90%, or at least 95% sequence identity to a sequence selected from SEQ ID NOs 7, and 9, and which is capable of reducing or inhibiting expression of C/EBPβ mRNA within a cell. In some embodiments, the C/EBPβ siRNA comprises a nucleic acid sequence comprising no more than 3, 2 or 1 substitutions relative to SEQ ID NOs: 7, and 9. In some embodiments, the C/EBPβ siRNA comprises a nucleic acid sequence having a continuous 18, 19 or 20 amino acids of any one of SEQ ID NOs: 7, and 9. In some embodiments, the C/EBPβ siRNA comprises a nucleic acid sequence having amino acids 1-18 of any one of SEQ ID NOs: 7, and 9. In some embodiments, the C/EBPβ siRNA comprises a nucleic acid sequence having amino acids 1-19 of any one of SEQ ID NOs: 7, and 9. In some embodiments, the C/EBPβ siRNA comprises a nucleic acid sequence having amino acids 1-20 of any one of SEQ ID NOs: 7, and 9. In some embodiments, the C/EBPβ siRNA comprises a nucleic acid sequence selected from SEQ ID NOs 7, and 9.

In some embodiments, the C/EBPβ siRNA comprises a nucleic acid sequence which hybridises to one of SEQ ID NOs 8, 10, 12 and 14. In some embodiments, the C/EBPβ siRNA comprises a nucleic acid sequence which hybridises to one of SEQ ID NOs 8 or 10.

In some embodiments, the C/EBPβ siRNA comprises a nucleic acid sequence which binds a region of C/EBPβ mRNA which at least partially overlaps with the binding site of one of SEQ ID NOs 8, 10, 12 and 14. A partially overlapping binding site may comprise 1, 2, 3, 4, 5, 6, 7, 8, 9, 10, 11, 12, 13, 14, 15, 16, 17, 18, 19, 20 or more overlapping residues. The overlap may be total i.e. the binding site of the siRNA may comprise or consist of that of SEQ ID NOs: 8, 10, 12 or 14 in its entirety. In some embodiments, the C/EBPβ siRNA competes with one of SEQ ID NOs 8, 10, 12 or 10 for binding to C/EBPβ mRNA.

In some embodiments, the C/EBPβ siRNA nucleic acid sequence comprises one or more ribo/deoxyribo nucleobases modified with a fluoro (F) , amino (NH₂) or O-methyl (OCH₃) group. In some cases, the nucleobases are modified at the 2' position. Heavy modification of siRNA, even full-length embellishment, does not affect siRNA activity, and these judicious modification strategies can significantly enhance siRNA stability or biocompatibility.

In some cases, the nucleobases are modified at the 2' position, and the modifications are selected from a fluoro (F) or O-methyl (OCH₃) group. 2' F and OCH₃ modifications may be according to the principles of Enhance Stabilisation Chemistry (ESC) strategy, as outlined in Hu et al, Signal Transduction and Targeted Therapy (2020) 5:101, which is herein incorporated by reference. These changes markedly enhanced siRNA potency and duration. Reading the sense strand 5' to 3', an siRNA modified according to ESC comprises the following 2' nucleobase modifications: (F) , (OCH₃) , (F) , (OCH₃) , (F) , (OCH₃) , (F) , (OCH₃) , (F) , (F) , (F) , (OCH₃) , (F) , (OCH₃) , (F) , (OCH₃) , (F) , (OCH₃) , (F) , (OCH₃) , (F) . Reading the antisense strand 5' to 3', an siRNA modified according to ESC comprises the following 2' nucleobase modifications: (OCH₃) , (F) , (OCH₃) , (F) , (OCH₃) , (F) , (OCH₃) , (F) , (OCH₃) , (F) , (OCH₃) , (OCH₃) , (OCH₃) , (F) , (OCH₃) , (F) , (OCH₃) , (F) , (OCH₃) , (F) , (OCH₃) . Any 3' overhanging residues, as discussed above whether on the sense or antisense strand, are modified (OCH₃) . In some embodiments, the C/EBPβ siRNA comprises an ESC modified sequence selected from SEQ ID NOs: 15 and 17.

Any of the ribonucleic acid compounds may additionally comprise one or more N-Acetylgalactosamine (GaINAc) moieties. In some embodiments, the moiety comprises 1, 2, 3, 4, 5, 6, 7, 8, 9, 10 or more GalNAc residues.

### Alternative targeting moieties

Disclosed herein are ribonucleic acid compounds which differ from those as outlined above by replacing the aptamer portion with an alternative targeting moiety. As used herein, an "alternative targeting moiety" is any moiety capable of selectively binding to one or more surface proteins on a cell. In some embodiments, a targeting moiety is internalised into the cell after binding the surface protein. In some embodiments, the surface protein is differentially expressed (i.e. is present or disproportionately expressed, preferably exclusively present) on one or more cell types. In some embodiments, the surface protein is differentially expressed on cancerous cells compared to healthy cells. In some embodiments, the surface protein is differentially expressed on hepatocytes.

An exemplary alternative targeting moiety comprises one or more N-Acetylgalactosamine (GaINAc) residue. GalNAc is an amino sugar derivative of galactose, capable of binding to asialoglycoprotein receptors on hepatocytes (Nair et al). As such, GalNAc finds use as a targeting ligand for siRNA in the context of treating cancers of the liver. In some embodiments, the moiety comprises 1, 2, 3, 4, 5, 6, 7, 8, 9, 10 or more GalNAc residues.

### Sequence identity

The terms "identical" or percent "identity," in the context of two or more nucleic acid or polypeptide sequences, refer to two or more sequences or subsequences that are the same or have a specified percentage of amino acid residues or nucleotides that are the same (i.e., about 60% identity, preferably 65%, 70%, 75%, 80%, 85%, 87%, 90%, 91%, 92%, 93%, 94%, 95%, 96%, 97%, 98%, 99%, or higher identity over a specified region, when compared and aligned for maximum correspondence over a comparison window or designated region) as measured using e.g. a BLAST or BLAST 2.0 sequence comparison algorithms with default parameters described below, or by manual alignment and visual inspection (see, e.g., NCBI web site http://www.ncbi.nlm.nih.gov/BLAST/ or the like) . Such sequences are then said to be "substantially identical." This definition also refers to, or may be applied to, the compliment of a test sequence. The definition also includes sequences that have deletions and/or additions, as well as those that have substitutions. As described below, the preferred algorithms can account for gaps and the like.

For sequence comparisons, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Preferably, default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from about 10 to 600, usually about 50 to about 200, more usually about 100 to about 150 in which a sequence may be compared to a reference sequence of the same number of contiguous positions after the two sequences are optimally aligned. Methods of alignment of sequences for comparison are well known in the art. Optimal alignment of sequences for comparison can be conducted in various ways known to a person of skill in the art, e.g., by the local homology algorithm of Smith & Waterman, Adv. Appl. Math. 2:482 (1981) , by the homology alignment algorithm of Needleman & Wunsch, J. Mol. Biol. 48:443 (1970) , by the search for similarity method of Pearson & Lipman, Proc. Nat'1. Acad. Sci. USA 85:2444 (1988) , by computerized implementations of these algorithms (GAP, BESTFIT, PASTA, and FASTA in the Wisconsin Genetics Software Package, Genetics Computer Group, 575 Science Dr., Madison, WI) , or by manual alignment and visual inspection (see, e.g., Current Protocols in Molecular Biology (Ausubel et al., eds. 1995 supplement) ) . Publicly available computer software may be used such as ClustalOmega (Söding, J. 2005, Bioinformatics 21, 951-960) , T-coffee (Notredame et al. 2000, J. Mol. Biol. (2000) 302, 205-217) , Kalign (Lassmann and Sonnhammer 2005, BMC Bioinformatics, 6(298) ) and MAFFT (Katoh and Standley 2013, Molecular Biology and Evolution, 30(4) 772-780 software. When using such software, the default parameters, e.g. for gap penalty and extension penalty, are preferably used.

A preferred example of algorithm that is suitable for determining percent sequence identity and sequence similarity are the BLAST and BLAST 2.0 algorithms, which are described in Altschul et al., Nuc. Acids Res. 25:3389-3402 (1977) and Altschul et al., J. Mol. Biol. 215:403-410 (1990) , respectively.

### Sequences

The aptamers TR14 and TR14 S2 are disclosed in WO2016/061386, whilst TR14 ST1-3 is disclosed in WO2019/033051.

| **SEQ ID NO:** | | **DESCRIPTI ON** | **SEQUENCE (5' → 3')** |
|---|---|---|---|
| | 1. | TR14 S1-3 (22mer) | UUUAUUCACAUUUUUGAAUUGA |
| | 2. | TR14 (87mer) | GGGAGACAAGAAUAAACGCUCAAUGCGUUCACGUUUAUUCACAUUUUU GAAUUGAGCAUGAGCUUCGACAGGAGGCUCACAACAGGC |
| | 3. | TR14 S2 (43mer) | GGGGCUCAAUGCGUUCACGUUUAUUCACAUUUUUGAAUUGAGC |
| | 4. | 8mer loop region | ACAUUUUU |
| | 5. | TR14 16mer truncation | AUUCACAUUUUUGAAU |
| | 6. | Human transferrin receptor protein 1 isoform 1 (GI:1894588 17; NCBI Reference Sequence: NP_003225. 2) (GI:1894588 16; NCBI Reference Sequence: NM_003234. 3 (transcript variant 1) ) (GI:1894588 18; NCBI Reference Sequence: NM_0011281 48.2 (transcript variant 2) | MMDQARSAFSNLFGGEPLSYTRFSLARQVDGDNSHVEMKLAVDEEENA DNNTKANVTKPKRCSGSICYGTIAVIVFFLIGFMIGYLGYCKGVEPKT ECERLAGTESPVREEPGEDFPAARRLYWDDLKRKLSEKLDSTDFTGTI KLLNENSYVPREAGSQKDENLALYVENQFREFKLSKVWRDQHFVKIQV KDSAQNSVIIVDKNGRLVYLVENPGGYVAYSKAATVTGKLVHANFGTK KDFEDLYTPVNGSIVIVRAGKITFAEKVANAESLNAIGVLIYMDQTKF PIVNAELSFFGHAHLGTGDPYTPGFPSFNHTQFPPSRSSGLPNIPVQT ISRAAAEKLFGNMEGDCPSDWKTDSTCRMVTSESKNVKLTVSNVLKEI KILNIFGVIKGFVEPDHYVVVGAQRDAWGPGAAKSGVGTALLLKLAQM FSDMVLKDGFQPSRSIIFASWSAGDFGSVGATEWLEGYLSSLHLKAFT YINLDKAVLGTSNFKVSASPLLYTLIEKTMQNVKHPVTGQFLYQDSNW ASKVEKLTLDNAAFPFLAYSGIPAVSFCFCEDTDYPYLGTTMDTYKEL IERIPELNKVARAAAEVAGQFVIKLTHDVELNLDYERYNSQLLSFVRD LNQYRADIKEMGLSLQWLYSARGDFFRATSRLTTDFGNAEKTDRFVMK KLNDRVMRVEYHFLSPYVSPKESPFRHVFWGSGSHTLPALLENLKLRK QNNGAFNETLFRNQLALATWTIQGAANALSGDVWDIDNEF |
| | 7. | *Human*/*mous e CEBPB* siRNA (AP6143) sense | CUG AGU AAU CAC UUA AAG AUU |
| | 8. | *Human*/*mous e CEBPB* siRNA (AP6143) antisense | UCU UUA AGU GAU UAC UCA GUU |
| | 9. | *Human CEBPB* siRNA AP6143-6145 sense | CUG AGU AAU CGC UUA AAG AUU |
| | 10. | *Human CEBPB* siRNA AP6143-6145 antisense | UCU UUA AGC GAU UAC UCA GUU |
| | 11. | *Mouse CEBPB* siRNA sense (Ambion/Am bs63860) | GCA CCC UGC GGA ACU UGU Utt |
| | | | Wherein "t" is a DNA residue, which may be substituted for an RNA "U" residue |
| | 12. | *Mouse CEBPB* siRNA antisense (Ambion/Am bs63860) | AAC AAG UUC CGC AGG GUG Ctg |
| | | | Wherein "t" and "g" are DNA residues, which may be substituted for corresponding RNA residues |
| | 13. | *Rat CEBPB* siRNA ("Hao") sense, as reported in Yi et al, 2017. | AAC UGC UGG CCU CGG CGG GUC |
| | 14. | *Rat CEBPB* siRNA ("Hao") antisense | GAC CCG CCG AGG CCA GCA GUU |
| | 15. | *Human*/*mous e CEBPB* siRNA (AP6143) sense, ESC modified | C(F) U(OMe) G(F) A(OMe) G(F) U(OMe) A(F) A(F) U(F) C(OMe) A(F) C(OMe) U(F) U(OMe) A(F) A(OMe) A(F) G(OMe) A(F) *U(OMe) *U(OMe) |
| | 16. | *Human*/*mous e CEBPB* siRNA (AP6143) antisense, ESC modified | U(OMe)* C(F) U(OMe)* U(F) U(OMe) A(F) A(OMe) G(F) U(OMe) G(F) A(OMe) U(OMe) U(OMe) A(F) C(OMe) U(F) C(OMe) A(F) G(OMe) *U(OMe) *U(OMe) |
| | 17. | *Mouse CEBPB* siRNA (Ambs63860) sense, ESC modified | G(F) C(OMe) A(F) C(OMe) C(F) C(OMe) U(F) G(F) C(F) G(OMe) G(F) A(OMe) A(F) C(OMe) U(F) U(OMe) G(F) U(OMe) U(F) *U(OMe) *U(OMe) |
| | 18. | *Mouse CEBPB* siRNA (Ambs63860) antisense, ESC modified | A(OMe) *A(F) *C(OMe) A(F) A(OMe) G(F) U(OMe) U(F) C(OMe) C(F) G(OMe) C(OMe) A(OMe) G(F) G(OMe) G(F) U(OMe) G(F) C(OMe) *U(OMe) *G(OMe) |
| | 19. | TR14 ST1-1 (40-nt) | GCUCAAUGCGUUCACGUUUAUUCACAUUUUUGAAUUGAGC |
| | 20. | TR14 ST1-2 (32-nt) | AAUGCGUUCACGUUUAUUCACAUUUUUGAAUU |
| | 21. | TR18 (87nt) | GGGAGACAAGAAUAAACGCUCAAUGCGUUUACGUUUAUUCA CAUUUUUGAAUUGAGCAUGAGCUUCGACAGGAGGCUCACAACAGGC |
| | 22. | TR14 S1 (46-nt) | GGGGCUCAAUGCGUUCACGUUUAUUCACAUUUUUGAAU UGAGCAUG |
| | 23. | Human CCAAT/enha ncer-binding protein beta mRNA (NM_001285 878.1 Homo sapiens CCAAT enhancer binding protein beta | TCCCAATCCCGGGGCGGCCGGGCGGGGGTGGGCAGGGGGCGTGAGGCC GCCCCTGCGTCCCGGGGGCCCCCCGAAAACGCGCTCCGGGTGCCCGGT CCCTCCGCTGCGCCCTGCCGCCGTCCTCCCGGGGGTCTCGGGCGGCCG CGGCCGTGTCCTTCGCGTCCCGGCGGCGCGGCGGGAGGGGCCGGCGTG ACGCAGCGGTTGCTACGGGCCGCCCTTATAAATAACCGGGCTCAGGAG AAACTTTAGCGAGTCAGAGCCGCGCACGGGACTGGGAAGGGGACCCAC CCGAGGGTCCAGCCACCAGCCCCCTCACTAATAGCGGCCACCCCGGCA GCGGCGGCAGCAGCAGCAGCGACGCAGCGGCGACAGCTCAGAGCAGGG AGGCCGCGCCACCTGCGGGCCGGCCGGAGCGGGCAGCCCCAGGCCCCC TCCCCGGGCACCCGCGTTCATGCAACGCCTGGTGGCCTGGGACCCAGC ATGTCTCCCCCCTGCCGCCGCCGCCGCCTGCCTTTAAATCCATGGAAGT GGCCAACTTCTACTACGAGGCGGACTGCTTGGCTGCTGCGTACGGCGG CAAGGCGGCCCCCGCGGCGCCCCCCGCGGCCAGACCCGGGCCGCGCCC |
| | | (CEBPB) , transcript variant 1, mRNA) | CCCCGCCGGCGAGCTGGCAGCATCGGCGACCACGAGCGCGCCATCGA CTTCAGCCCGTACCTGGAGCCGCTGGGCGCGCCGCAGGCCCCGGCGCC CGCCACGGCCACGGACACCTTCGAGGCGGCTCCGCCCGCGCCCGCCCC CGCGCCCGCCTCCTCCGGGCAGCACCACGACTTCCTCTCCGACCTCTT CTCCGACGACTACGGGGGCAAGAACTGCAAGAAGCCGGCCGAGTACGG CTACGTGAGCCTGGGGCGCCTGGGGGCCGCCAAGGGCGCGCTGCACCC CGGCTGCTTCGCGCCCCTGCACCCACCGCCCCCGCCGCCGCCGCCGCC CGCCGAGCTCAAGGCGGAGCCGGGCTTCGAGCCCGCGGACTGCAAGCG GAAGGAGGAGGCCGGGGCGCCGGGCGGCGGCGCAGGCATGGCGGCGGG CTTCCCGTACGCGCTGCGCGCTTACCTCGGCTACCAGGCGGTGCCGAG CGGCAGCAGCGGGAGCCTCTCCACGTCCTCCTCGTCCAGCCCGCCCGG CACGCCGAGCCCCGCTGACGCCAAGGCGCCCCCGACCGCCTGCTACGC GGGGGCCGCGCCGGCGCCCTCGCAGGTCAAGAGCAAGGCCAAGAAGAC CGTGGACAAGCACAGCGACGAGTACAAGATCCGGCGCGAGCGCAACAA CATCGCCGTGCGCAAGAGCCGCGACAAGGCCAAGATGCGCAACCTGGA GACGCAGCACAAGGTCCTGGAGCTCACGGCCGAGAACGAGCGGCTGCA GAAGAAGGTGGAGCAGCTGTCGCGCGAGCTCAGCACCCTGCGGAACTT GTTCAAGCAGCTGCCCGAGCCCCTGCTCGCCTCCTCCGGCCACTGCTA GCGCGGCCCCCGCGCGTCCCCCTGCCGGCCGGGGCTGAGACTCCGG GGAGCGCCCGCGCCCGCGCCCTCGCCCCCGCCCCCGGCGGCGCCGGCA AAACTTTGGCACTGGGGCACTTGGCAGCGCGGGGAGCCCGTCGGTAAT TTTAATATTTTATTATATATATATATCTATATTTTTGTCCAAACCAAC CGCACATGCAGATGGGGCTCCCGCCCGTGGTGTTATTTAAAGAAGAAA CGTCTATGTGTACAGATGAATGATAAACTCTCTGCTTCTCCCTCTGCC CCTCTCCAGGCGCCGGCGGGCGGGCCGGTTTCGAAGTTGATGCAATCG GTTTAAACATGGCTGAACGCGTGTGTACACGGGACTGACGCAACCCAC GTGTAACTGTCAGCCGGGCCCTGAGTAATCGCTTAAAGATGTTCCTAC GGGCTTGTTGCTGTTGATGTTTTGTTTTGTTTTGTTTTTTGGTCTTTT TTTGTATTATAAAAAATAATCTATTTCTATGAGAAAAGAGGCGTCTGT ATATTTTGGGAATCTTTTCCGTTTCAAGCATTAAGAACACTTTTAATA AACTTTTTTTTGAGAATGGTTACAAAGCCTTTTGGGGGCAGTAAAAAA A |
| | 24. | saCEBPa sense | GACCAGUGACAAUGACCGCUU |
| | 25. | saCEBPa antisese | AAGCGGUCAUUGUCACUGGUC |
| | 26. | *Human CEBPB* siRNA sense, ESC modified | C(F) U(OMe) G(F) A(OMe) G(F) U(OMe) A(F) A(F) U(F) C(OMe) G(F) C(OMe) U(F) U(OMe) A(F) A(OMe) A(F) G(OMe) A(F) U(OMe) U(OMe) |
| | 27. | *Human CEBPB* siRNA antisense, ESC modified | U(OMe) C(F) U(OMe) U(F) U(OMe) A(F) A(OMe) G(F) C(OMe) G(F) A(OMe) U(OMe) U(OMe) A(F) C(OMe) U(F) C(OMe) A(F) G(OMe) U(OMe) U(OMe) |
| | 28. | siTTR sense | AACAGUGUUCUUGCUCUAUAA |
| | 29. | siTTR antisense | UUAUAGAGCAAGAACACUGUU |
| | 30. | MALATI siRNA sense | GGGCUUCUCUUAACAUUUAUU |
| | 31. | MALATI siRNA antisense | UAAAUGUUAAGAGAAGCCCUU |
| | 32. | siFLUC sense | CUU ACG CUG AGU ACU UCG AUU |
| | 33. | siFLUC antisense | UCG AAG UAC UCA GCG UAA GUU |
| | 34. and 47. respectively (joined by linker) | TfR-SS-siCEBPB (AP6143) seq 2 - sense | U(F) U(F) U(F) rA U(F) U(F) C(F) A C(F) A U(F) U(F) U(F) U(F) U(F) G A A U(F) U(F) G A - iSpC3iSpC3C6-SS-C6iSpC3iSpC3 - C(OMe) U(OMe) G(OMe) A(OMe) G(OMe) U(OMe) A(OMe) A(OMe) U C G C U U A A(OMe) A(OMe) G(OMe) A(OMe) *U(OMe) *U(OMe) |
| | 35. | TfR-SS-siCEBPB (AP6143) antisense | U *C *U  U U A  A G C  G A U  U A C  U C A  G* U(OMe)  *U(OMe) |
| | 36. and 48 respectively (joined by linker) | TfR-SS-siCEBPB (AP6145) sense | U(F) U(F) U(F)  A U(F) U(F) C(F)  AC(F)  A U(F) U(F) U(F) U(F) U(F)  G  A A U(F) U(F)  G A - iSpC3iSpC3C6-SS-C6iSpC3iSpC3 -C(OMe) U(OMe) G(OMe) A(OMe) G(OMe) U(OMe) A(OMe) A(OMe)  U C G  C U U  A A(OMe) A(OMe) G(OMe) A(OMe) U(OMe) U(OMe) |
| | 37. | TfR-SS-siCEBPB (AP6145) antisense | U(OMe) C(F) U(OMe) U(F) U(OMe) A(F) A(OMe) G(F) C(OMe) G(F) A(OMe) U(OMe) U(OMe) A(F) C(OMe) U(F) C(OMe) A(F) G(OMe) *U(OMe) *U(OMe) |
| | 38. and 49 respectively (joined by linker) | TfR-SS-CEBPA (AP6511) sense | UUUAUUCACAUUUUUGAAUUGA - SS - GACCAGUGACAAUGACCGCUU |
| | 39. | TfR-SS-CEBPA (AP6511) antisense | GCGGUCAUUGUCACUGGUCUU |
| | 40. | TR14 S1-3 (22mer), ESC modified | U(F) U(F) U(F) A U(F) U(F) C(F) A C(F) A U(F) U(F) U(F) U(F) U(F) G A A U(F) U(F) G A |
| | 41. and 50, respectively (joined by linker) | (m)siCEBPb (Ambs63860) - Seq 1-sense | U(F) U(F) U(F) A U(F) U(F) C(F) A C(F) A U(F) U(F) U(F) U(F) U(F) G A A U(F) U(F) G A - iSpC3iSpC3C6-SS-C6iSpC3iSpC3 - G(F) C(OMe) A(F) C(OMe) C(F) C(OMe) U(F) G(F) C(F) G(OMe) G(F) A(OMe) A(F) C(OMe) U(F) U(OMe) G(F) U(OMe) U(F) U(OMe) U(OMe) |
| | 42. | (m)siCEBPb (Ambs63860) - Seq 1-antisense | A* A(F)* C(OMe) A(F) A(OMe) G(F) U(OMe) U(F) C(OMe) C(F) G(OMe) C(OMe) A(OMe) G(F) G(OMe) G(F) U(OMe) G(F) C(OMe) U(OMe) G(OMe) |
| | 43. | TR14 S2 (42mer), ESC modified | G G G G C(F) U(F) C(F) A A U(F) G C(F) G U(F) U(F) C(F) A C(F) G U(F) U(F) U(F) A U(F) U(F) C(F) A C(F) A U(F) U(F) U(F) U(F) U(F) G A A U(F) U(F) G A G C(F) |
| | 44. | GalNAc-(h/m)siCEBP b (XD-30742) sense | (GalNAc3)(NHC6) *C(F) U(OMe) G(F) A(OMe) G(F) U(OMe) A(F) A(F) U(F) C(OMe) A(F) C(OMe) U(F) U(OMe) A(F) A(OMe) A(F) G(OMe) A(F) *U(OMe) *U(OMe) |
| | 45. | GalNAc-siFLUC XD-30743 sense | (GalNAc3)(NHC6) *C(F) U(OMe) U(F) A(OMe) C(F) G(OMe) C(F) U(F) G(F) A(OMe) G(F) U(OMe) A(F) C(OMe) U(F) U(OMe) C(F) G(OMe) A(F) *U(OMe) *U(OMe) |
| | 46. | GalNAc-(h)siCEBPb sense | (GalNAc3)(NHC6) C(F) U(OMe) G(F) A(OMe) G(F) U(OMe) A(F) A(F) U(F) C(OMe) G(F) C(OMe) U(F) U(OMe) A(F) A(OMe) A(F) G(OMe) A(F) U(OMe) U(OMe) |

### Modifications

The ribonucleic acid compounds described herein may contain chemical modifications, e.g. as defined herein, to enhance their functional characteristics, such as nuclease resistance or binding affinity. The modifications may be present in a ribonucleic acid compound, a RNA sequence and/or in a nucleotide-based compound moiety or compound, e.g. a saRNA, siRNA, miRNA, mRNA.

In some cases, modifications may be made to the base, sugar ring, or phosphate group of one or more nucleotides.

In some cases, the ribonucleic acid compounds described herein comprise one or more modified nucleobases. In some cases, the nucleobases are modified at the 2' position, the 3' position, the 5' position or the 6' position. For example, the ribonucleic acid compounds may comprise one or more ribo/deoxyribo nucleobases modified at the 2' position with a fluoro (F) , amino (NH₂) or O-methyl (OMe) group. In some cases, the ribonucleic acid compounds may comprise one or more 2'-aminopyrimidines, 2'-fluoropyrimidines, 2'-O-methyl nucleotides and/or 'locked' nucleotides (LNA) (see e.g. Lin, Y et al., Nucleic Acids Res. 1994 22, 5229-5234 (1994) ; Ruckman, J. et al.. J. Biol. Chem. 1998 273, 20556-20567; Burmeister, PE et al., Chem. Biol. 2005 12, 25-33; Kuwahara, M. & Obika, S. Artif. DNA PNA XNA 2013 4, 39-48; Veedu, R. N. & Wengel, J. Mol. Biosyst. 2009 5,787-792) . In some cases, the ribonucleic acid compounds comprise one or more L-form nucleic acids (see e.g. Maasch, C et al., Nucleic Acids Symp. Ser. (Oxf.) 2008 52, 61-62) . Other suitable nucleic acid modifications will be apparent to those skilled in the art (see, e.g. Ni S et al., Int. J. Mol. Sci 2017 18, 1683, hereby incorporated by reference in its entirety). A modified version of SEQ ID NO:1 is SEQ ID NO:40. An unmodified amino acid may be denoted by a preceding "r", for example "C(F)G(OMe)rA" indicates a 2'fluorpyrimidine modified C residue, a 2'-O-methyl modified G residue, and an unmodified A residue.

In some embodiments, the ribonucleic acid compounds comprise one or more ribo/deoxyribo nucleobases with a phosphorothioate (PS) backbone modification. This may be denoted in a sequence as described herein with an asterisk ("*") between two residues (e.g. AG*CU, indicating a PS modification between G and C). A PS modification may be combined with further modifications, for example a 2' substitution.

In some embodiments, a ribonucleic acid compound as described herein may comprise one or more 2' modification relative to the sequence provided herein, the modification selected from 2'(F) and 2'(OMe). In some embodiments, a ribonucleic acid compound as described herein may comprise at least two, three, four, five, six, seven, eight, nine, ten or more 2' modifications relative to an unmodified sequence provided herein, the modifications independently selected from 2'(F) and 2'(OMe). In some embodiments, a ribonucleic acid compound as described herein may comprise at least one, two, three, four, five, six, seven, eight, nine, ten or more 2'(F) modifications and/or at least one, two, three, four, five, six, seven, eight, nine, ten or more 2'(OMe) modifications relative to an unmodified sequence provided herein. In other embodiments, a ribonucleic acid compound as described herein may comprise at least one, two, three, four, five, six, seven, eight, nine, ten or more fewer 2'(F) modifications and/or at least one, two, three, four, five, six, seven, eight, nine, ten or more fewer 2'(OMe) modifications than a modified sequence provided herein.

In some cases, a sense and/or antisense strand of a nucleotide compound moiety, e.g., mRNA, miRNA, siRNA or saRNA, may comprise a nucleotide overhang. For example, said overhang may be a 2-nucleotide (UU) overhang. Said overhang may be on the 3' end of one or both strands. An overhang may favour Dicer recognition of the nucleotide compound moiety.

In some cases, the ribonucleic acid compounds described herein comprise an inverted thymidine cap on the 3' end, or comprise 3'-biotin. In some cases, the phosphodiester linkage in the ribonucleic acid compounds in replaced with methylphosphonate or phosphorothioate analogue, or triazole linkages (see Ni S et al., *supra*) .

In some cases, the ribonucleic acid compounds described herein comprise one or more copies of the C3 spacer phosphoramite. Spacers may be incorporated internally, e.g. between an RNA sequence and a siRNA, or at the 5' or 3' end of the nucleotide sequence to attach e.g. imaging moieties.

In some cases, the ribonucleic acid compounds described herein comprise modifications to increase half-life and/or resist renal clearance. For example, the compounds may be modified to include cholesterol, dialkyl lipids, proteins, liposomes, organic or inorganic nanomaterials, nanoparticles, inert antibodies or polyethylene glycol (PEG) e.g. 20kDa PEG, 40 kDa PEG. Such modifications may be at the 5'-end of the compounds. In some cases, the modification comprises a molecule with a mass above the cut-off threshold for the renal glomerulus (~30-50 kDa) . In some cases, the nucleic compounds may be formulated with pluronic gel. For examples of suitable modifications and formulations see e.g. Ni et al, *supra,* and Zhou and Rossi, Nat Rev Drug Disc 2017, 16 181-202; both hereby incorporated by reference in their entirety.

The ribonucleic acid compounds described herein may comprise a tag, such as an albumin tag. An albumin tag may be attached to the ribonucleic acid compound at the RNA sequence or at a moiety. A tag, such as an albumin tag, may be attached via a linker sequence, for example a poly-uridine (poly-U) linker. A poly-U linker may be about 20, about 19, about 18, about 17, about 16, about 15, about 14, about 13, about 12, about 11, about 10, about 9, about 8, about 7, about 6 or about 5 residues in length. A poly-U linker may be between 1 and 20, between 5 and 15, or between 8 and 12 residues in length. Other tags may include: poly(His) tag, chitin binding protein (CBP) , maltose binding protein (MBP) , Strep-tag and glutathione-S-transferase (GST) . The compounds may comprise an nucleic acid affinity tag, as described in, for example, Srisawat C and Engelke DR, Methods. 2002 26(2) : 156-161 and Walker et al., Methods Mol Biol. 2008; 488: 23-40, hereby incorporated by reference in their entirety. Other suitable tags will be readily apparent to one skilled in the art.

The ribonucleic acid compounds described herein may comprise spacer or linker sequences between the nucleic acid portion and a compound moiety and/or tag. Suitable spacer or linker sequences will be readily apparent to one skilled in the art.

### Triangular ribonucleic acid compounds

Two transferrin receptors, transferrin receptor 1 (TfR1) and transferrin receptor 2 (TfR2), have been characterized in humans. Both are cell membrane proteins, however their expression differs within the body. TfR1 is expressed throughout the body, but is highly (>100,000X) upregulated in cancer compared to normal cells. TfR1 is also capable of facilitating transport traversing the blood-brain barrier. TfR2 meanwhile is expressed only in hepatocytes. Aptamers which preferentially, or exclusively, bind to different forms of TfR therefore can be used for differential trafficking of cargo to different destinations.

In some embodiments, the ribonucleic acid compound comprises (i) an aptamer capable of binding to transferrin receptor (TfR), and (ii) a CCAAT/enhancer-binding protein β (C/EBPβ) siRNA, wherein the aptamer is capable of binding (e.g. preferentially or exclusively binding) to TfR1. Such a ribonucleic acid compound may be particularly useful for preferentially delivering the siRNA to cancer cells over normal cells, and for traversing the blood-brain barrier. Exemplary aptamers capable of binding to TfR1 include SEQ ID NOs: 1, 2, 5, 22, 40, or an aptamer comprising or consisting of an RNA sequence having at least 90% sequence identity to SEQ ID NO:1 or SEQ ID NO:40, where said RNA sequence has a length of 29 nucleotides or fewer. The ribonucleic acid compound may additionally comprise (iii) one or more GAINAc residue.

In other embodiments, the aptamer is capable of binding (e.g. preferentially or exclusively binding) to TfR2. Such a ribonucleic acid compound may be particularly useful for preferentially delivering the siRNA to liver cells. The 43nt truncation TR14 S2 (SEQ ID NO:3 or 43) preferentially binds TfR2 and may find use in such embodiments.

In some embodiments, the aptamer is capable of binding to TfR1 and TfR2. Such a ribonucleic acid compound may be particularly useful for preferentially delivering the siRNA to cancerous liver cells compared to non-liver and/or non-cancerous cells. It may be especially useful for preferentially delivering siRNA to cancerous liver cells compared to non-cancerous liver cells. Exemplary aptamers capable of binding both TfR1 and TfR2 comprise or consist of the 87nt TR14 (SEQ ID NO:2), the 46nt TR14 S1 (SEQ ID NO:22), or RNA sequences having at least 90% identity and more than 43 nucleotides thereof.

Whilst binding to TfR1 and TfR2 may be advantageous, it is not necessary for a single aptamer to posses both properties. Instead, it may be preferred to combine an aptamer which exclusively or preferentially binds TfR1 and an aptamer which exclusively or preferentially binds TfR2 within a single ribonucleic acid compound of the invention. This may have several advantages, including easier synthesis, shorter total length, and better specificity for either or both TfRs when compared to a bispecific aptamer.

In some embodiments, the ribonucleic acid compound comprises two or more aptamers capable of binding to transferrin receptor, preferably two different transferring receptors. For example, a ribonucleic acid compound may comprise (i) a first aptamer capable of (e.g. preferentially or exclusively) binding to transferrin receptor (TfR), the transferrin receptor being TfR1, (ii) a CCAAT/enhancer-binding protein β (C/EBPβ) siRNA, and (iii) a second aptamer capable of (e.g. preferentially or exclusively) binding to transferrin receptor 2 (TfR2), in any order. Such a ribonucleic acid compound may be termed a "triangular ribonucleic acid compound", as it comprises three elements. Suitable first and second aptamers are provided above. Preferably, the first aptamer may comprise or consist of an RNA sequence having at least 90% sequence identity to SEQ ID NO:1 or SEQ ID NO:40, where said RNA sequence has a length of 29 nucleotides or fewer, and may comprise or consist of SEQ ID NO:1 or SEQ ID NO:40. The second aptamer may comprise or consist of SEQ ID NO: 3 or 43. The ribonucleic acid compound may additionally comprise (iv) one or more GAINAc residue.

In some embodiments of the triangular ribonucleic acid compound, the first aptamer is conjugated to the second aptamer. In other embodiments, the first and second aptamer are each independently conjugated to the C/EBPβ siRNA. In some embodiments, the first aptamer, second aptamer and the C/EBPβ siRNA are linked through an additional moiety, for example a spacer and/or brancher moiety. A spacer moiety is any moiety having a length and which is able to link two nucleic acids, preferably a 5' linking end and a 3' linking end (i.e. a spacer with a phosphodiester bond forming group at one end of a length and a phosphodiester bond receiving group on the other). Suitable spacer moieties include for example phosphoramite spacers e.g phosphoramidite 18 (18-O-Dimethoxytritylhexaethyleneglycol,1-[(2-cyanoethyl)-(N,N-diisopropyl)]-phosphoramidite) or phosphoramite C3 (3-(4,4'-Dimethoxytrityloxy)propyl-1-[(2-cyanoethyl)-(N,N-diisopropyl)]-phosphoramidite). Brancher moieties are any moiety capable of forming bonds to three or more spacers and/or nucleic acids (e.g. which has three or more receiving phosphate groups), and include deoxycytidine derived branchers, for example 5-Me-dC phosphoramidite (5'-Dimethoxytrityl-N4-(O-levulinyl-6-oxyhexyl)-5-Methyl-2'-deoxyCytidine,3'-[(2-cyanoethyl)-(N,N-diisopropyl)]-phosphoramidite) and levulinyl 5-me dC.

In some embodiments, the triangular ribonucleic acid compound comprises a first aptamer comprising or consisting of SEQ ID NO:1 or 40, a second aptamer comprising or consisting of SEQ ID NO:3 or 43, and a C/EBPβ siRNA comprising or consisting of SEQ ID NO:15 or 26. The first aptamer, second aptamer and C/EBPβ siRNA may each be linked to one or more spacers, and the spacers may each be linked to one another by a brancher moiety.

In a particular embodiment, the triangular ribonucleic acid compound comprises:
- A first aptamer comprising or consisting of SEQ ID NO:40,
- a second aptamer comprising or consisting of SEQ ID NO:43,
- a C/EBPβ siRNA comprising or consisting of SEQ ID NO:15 or 26,
wherein:
- the first aptamer is linked to a first spacer comprising a phosphoramite C3, optionally three phosphoramite C3 spacers joined in series
- the second aptamer is linked to a second spacer comprising a phosphoramite 18, optionally two phosphoramite 18 spacers joined in series,
- the C/EBPβ siRNA is linked to a third spacer comprising a phosphoramite C3, optionally three phosphoramite C3 spacers joined in series,
and wherein the first, second and third spacers are linked through a brancher, optionally a levulinyl 5-Me-dC brancher.

An exemplary triangular ribonucleic acid compound according to this embodiment is shown in Figure 9.

### Functional characteristics

The ribonucleic acid compounds described herein may be characterised by reference to certain functional properties.

In some embodiments, any nucleic/ribonucleic/deoxyribonucleic acid compound described herein may possess one or more of the following properties:
Binds to transferrin receptor (TfR) ;
Capable of binding to TfR;
Binds specifically to TfR;
Capable of binding specifically to TfR;
Binds to TfR on the surface of a cell;
Capable of binding to TfR on the surface of a cell;
Capable of being internalised by a cell;
Capable of delivering a payload, e.g. an siRNA, into a cell;
Capable of traversing the blood-brain barrier;
Capable of being transported into the brain;
Capable of delivering a payload, e.g. an siRNA, into the brain.

The binding of a ribonucleic acid compound to a transferrin receptor can be determined by, e.g., surface plasmon resonance technology, as illustrated herein and described in Drescher et al., Methods Mol Biol. 2009;493: 323-343.

The term "internalised," "internalising," or "internalisation" as provided herein refers to a composition (e.g., a compound, a ribonucleic acid compound, a therapeutic agent, an imaging agent) being drawn into the cytoplasm of a cell (e.g. after being engulfed by a cell membrane) .

### Pharmaceutical formulations

The present invention provides pharmaceutical compositions comprising the ribonucleic acid compounds described herein, optionally comprising a pharmaceutically acceptable excipient.

The ribonucleic acid compounds described herein may be formulated as pharmaceutical compositions or medicaments for clinical use and may comprise a pharmaceutically acceptable carrier, diluent, excipient or adjuvant. The composition may be formulated for topical, parenteral, systemic, intracavitary, intravenous, intra-arterial, intramuscular, intrathecal, intraocular, intraconjunctival, intratumoral, subcutaneous, intradermal, intrathecal, oral or transdermal routes of administration which may include injection or infusion. Suitable formulations may comprise the antigen-binding molecule in a sterile or isotonic medium. Medicaments and pharmaceutical compositions may be formulated in fluid, including gel, form. Fluid formulations may be formulated for administration by injection or infusion (e.g. via catheter) to a selected region of the human or animal body.

In some cases, the ribonucleic acid compound according to the present invention are formulated for injection or infusion, e.g. into a blood vessel or tumour.

Pharmaceutical compositions of the ribonucleic acid compounds provided herein may include compositions having a therapeutic moiety contained in a therapeutically or prophylactically effective amount, i.e., in an amount effective to achieve its intended purpose. The pharmaceutical compositions of the ribonucleic acid compounds provided herein may include compositions having imaging moieties contained in an effective amount, i.e., in an amount effective to achieve its intended purpose. The actual amount effective for a particular application will depend, *inter alia,* on the condition being treated, tested, detected, or diagnosed. When administered in methods to treat a disease, such compositions will contain an amount of active ingredient effective to achieve the desired result, e.g., modulating the activity of a target molecule, and/or reducing, eliminating, or slowing the progression of disease symptoms. Determination of a therapeutically or prophylactically effective amount of a therapeutic moiety provided herein is well within the capabilities of those skilled in the art, especially in light of the detailed disclosure herein. When administered in methods to diagnose or detect a disease, such compositions will contain an amount of an imaging moiety described herein effective to achieve the desired result, e.g., detecting the absence or presence of a target molecule, cell, or tumour in a subject. Determination of a detectable amount of an imaging moiety provided herein is well within the capabilities of those skilled in the art, especially in light of the detailed disclosure herein.

The dosage and frequency (single or multiple doses) administered to a mammal can vary depending upon a variety of factors, for example, whether the mammal suffers from another disease; the route of administration; size, age, sex, health, body weight, body mass index, and diet of the recipient; nature and extent of symptoms of the disease being treated, kind of concurrent treatment, complications from the disease being treated or other health-related problems. Other therapeutic regimens or agents can be used in conjunction with the methods and compositions described herein including embodiments thereof. Adjustment and manipulation of established dosages (e.g., frequency and duration) are well within the ability of those skilled in the art.

For any composition (e.g., the ribonucleic acid compounds provided, as well as combinations of an anticancer agent and the ribonucleic acid compound provided) described herein, the therapeutically effective amount can be initially determined from cell culture assays. Target concentrations will be those concentrations of active compound(s) that are capable of achieving the methods described herein, as measured using the methods described herein or known in the art. As is well known in the art, effective amounts for use in humans can also be determined from animal models. For example, a dose for humans can be formulated to achieve a concentration that has been found to be effective in animals. The dosage in humans can be adjusted by monitoring effectiveness and adjusting the dosage upwards or downwards, as described above. Adjusting the dose to achieve maximal efficacy in humans based on the methods described above and other methods is well within the capabilities of the ordinarily skilled artisan.

In one aspect, provided herein is a pharmaceutical composition including a ribonucleic acid compound as described herein, including embodiments thereof, and a pharmaceutically acceptable excipient.

In some aspects, the pharmaceutical composition includes a ribonucleic acid compound as provided herein, including embodiments thereof, and a therapeutic agent. In some embodiments, the ribonucleic acid compound comprises a compound moiety. In some embodiments, the ribonucleic acid compound and the therapeutic agent are not covalently attached. A therapeutic agent as provided herein refers to a composition (e.g. compound, drug, antagonist, inhibitor, modulator) having a therapeutic effect. In some embodiments, the therapeutic agent is an anticancer agent. In some embodiments, the pharmaceutical composition includes a pharmaceutically acceptable excipient.

In some aspects, there is provided a pharmaceutical composition comprising a ribonucleic acid compound as provided herein, including embodiments thereof, and a compound as described herein. That is, the composition comprises the ribonucleic acid compound and a compound, e.g. a therapeutic or diagnostic molecule, which does not form part of the ribonucleic acid compound itself. In some cases, the ribonucleic acid compound comprises a compound moiety. In some cases, the pharmaceutical composition additionally comprises a therapeutic agent.

Pharmaceutical compositions may be prepared using a pharmaceutically acceptable "carrier" composed of materials that are considered safe and effective. "Pharmaceutically acceptable" refers to molecular entities and compositions that are "generally regarded as safe", e.g., that are physiologically tolerable and do not typically produce an allergic or similar untoward reaction, such as gastric upset and the like, when administered to a human. In some embodiments, this term refers to molecular entities and compositions approved by a regulatory agency of the US federal or a state government, as the GRAS list under section 204(s) and 409 of the Federal Food, Drug and Cosmetic Act, that is subject to premarket review and approval by the FDA or similar lists, the U.S. Pharmacopeia or another generally recognised pharmacopeia for use in animals, and more particularly in humans.

The term "carrier" refers to diluents, binders, lubricants and disintegrants. Those with skill in the art are familiar with such pharmaceutical carriers and methods of compounding pharmaceutical compositions using such carriers.

The pharmaceutical compositions provided herein may include one or more excipients, e.g., solvents, solubility enhancers, suspending agents, buffering agents, isotonicity agents, antioxidants or antimicrobial preservatives. When used, the excipients of the compositions will not adversely affect the stability, bioavailability, safety, and/or efficacy of the active ingredients, i.e. a ribonucleic acid compound used in the composition. Thus, the skilled person will appreciate that compositions are provided wherein there is no incompatibility between any of the components of the dosage form. Excipients may be selected from the group consisting of buffering agents, solubilizing agents, tonicity agents, chelating agents, antioxidants, antimicrobial agents, and preservatives.

Non-limiting examples of pharmaceutically acceptable excipients include water, NaCl, normal saline solutions, lactated Ringer's, normal sucrose, normal glucose, binders, fillers, disintegrants, lubricants, coatings, sweeteners, flavours, salt solutions (such as Ringer's solution) , alcohols, oils, gelatins, carbohydrates such as lactose, amylose or starch, fatty acid esters, hydroxymethycellulose, polyvinyl pyrrolidine, and colours, and the like. Such preparations can be sterilized and, if desired, mixed with auxiliary agents such as lubricants, preservatives, stabilizers, wetting agents, emulsifiers, salts for influencing osmotic pressure, buffers, colouring, and/or aromatic substances and the like that do not deleteriously react with the compounds of the invention. One of skill in the art will recognize that other pharmaceutical excipients are useful in the present invention.

The term "pharmaceutically acceptable salt" refers to salts derived from a variety of organic and inorganic counter ions well known in the art and include, by way of example only, sodium, potassium, calcium, magnesium, ammonium, tetraalkylammonium, and the like; and when the molecule contains a basic functionality, salts of organic or inorganic acids, such as hydrochloride, hydrobromide, tartrate, mesylate, acetate, maleate, oxalate and the like.

The term "composition" is intended to include the formulation of the active compound with encapsulating material as a carrier providing a capsule in which the active component with or without other carriers, is surrounded by a carrier, which is thus in association with it. Similarly, cachets and lozenges are included. Tablets, powders, capsules, pills, cachets, and lozenges can be used as solid dosage forms suitable for oral administration.

The pharmaceutical composition is optionally in unit dosage form. In such form the preparation is subdivided into unit doses containing appropriate quantities of the active component. The unit dosage form can be a packaged composition, the package containing discrete quantities of composition, such as packeted tablets, capsules, and powders in vials or ampoules. Also, the unit dosage form can be a capsule, tablet, cachet, or lozenge itself, or it can be the appropriate number of any of these in packaged form. The unit dosage form can be of a frozen dispersion.

Medicaments and pharmaceutical compositions according to aspects of the present invention may be formulated for administration by a number of routes, including but not limited to, parenteral, intravenous, intra-arterial, intramuscular, intratumoural, oral and nasal. The medicaments and compositions may be formulated in fluid or solid form. Fluid formulations may be formulated for administration by injection to a selected region of the human or animal body.

Administration is preferably in a "therapeutically effective amount", this being sufficient to show benefit to the individual. The actual amount administered, and rate and time-course of administration, will depend on the nature and severity of the disease being treated. Prescription of treatment, e.g. decisions on dosage etc, is within the responsibility of general practitioners and other medical doctors, and typically takes account of the disorder to be treated, the condition of the individual patient, the site of delivery, the method of administration and other factors known to practitioners. Examples of the techniques and protocols mentioned above can be found in Remington's Pharmaceutical Sciences, 20th Edition, 2000, pub. Lippincott, Williams & Wilkins.

### Methods of delivery

As described above the ribonucleic acid compounds, e.g. ribo/deoxyribonucleic acid compounds provided herein, including embodiments thereof, may be used to deliver a siRNA C/EBPβ moiety into a cell. Upon binding of the ribonucleic acid compound to TfR on a cell, the siRNA C/EBPβ moiety may be internalized by the cell while being covalently attached to the ribonucleic acid compound. Thus, in one aspect, a method of delivering a siRNA C/EBPβ moiety into a cell is provided. The method includes, (i) contacting a cell with the ribonucleic acid compound, or composition, as provided herein including embodiments thereof and (ii) allowing the ribonucleic acid compound to bind to a TfR on the cell and pass into the cell thereby delivering the siRNA C/EBPβ moiety into the cell.

The methods may be performed *in vitro, ex vivo,* or *in vivo.*

### Therapeutic and prophylactic applications

The ribonucleic acid compounds, e.g. ribo/deoxyribonucleic acid compounds, and compositions provided herein find use in therapeutic and prophylactic methods.

The present invention provides ribonucleic acid compounds and compositions described herein for use in a method of medical treatment or prophylaxis. The invention also provides the use of ribonucleic acid compounds and compositions described herein in the manufacture of medicaments for treating or preventing a disease or disorder. The invention described herein also provides methods of treating or preventing a disease or disorder, comprising administering to a subject in need thereof a therapeutically or prophylactically effective amount of a ribonucleic acid compound or composition described herein.

As used herein, "treatment" or "treating," or "palliating" or "ameliorating" are used interchangeably herein. These terms refer to an approach for obtaining beneficial or desired results including but not limited to therapeutic benefit and/or a prophylactic benefit. By therapeutic benefit is meant eradication or amelioration of the underlying disorder being treated. Also, a therapeutic benefit is achieved with the eradication or amelioration of one or more of the physiological symptoms associated with the underlying disorder such that an improvement is observed in the patient, notwithstanding that the patient may still be afflicted with the underlying disorder. For prophylactic benefit, the compositions may be administered to a patient at risk of developing a particular disease, or to a patient reporting one or more of the physiological symptoms of a disease, even though a diagnosis of this disease may not have been made. Treatment includes preventing the disease, that is, causing the clinical symptoms of the disease not to develop by administration of a protective composition prior to the induction of the disease; suppressing the disease, that is, causing the clinical symptoms of the disease not to develop by administration of a protective composition after the inductive event but prior to the clinical appearance or reappearance of the disease; inhibiting the disease, that is, arresting the development of clinical symptoms by administration of a protective composition after their initial appearance; preventing re-occurring of the disease and/or relieving the disease, that is, causing the regression of clinical symptoms by administration of a protective composition after their initial appearance.

The ribonucleic acid compounds described herein find use in the treatment or prevention of any disease/disorder which would benefit from the delivery of said compounds, and/or associated therapeutic or imaging moieties, to cells expressing TfR.

It will be appreciated that the therapeutic and prophylactic utility of the present invention extends to the treatment of any subject that would benefit from the delivery of a compound moiety or compound into a cell expressing TfR.

For example, in some embodiments, certain methods described herein treat cancer (e.g. liver cancer (e.g. hepatocellular carcinoma) , pancreatic cancer, pancreatic liver metastases, prostate cancer, renal cancer, metastatic cancer, melanoma, castration-resistant prostate cancer, breast cancer, triple negative breast cancer, glioblastoma, ovarian cancer, lung cancer, squamous cell carcinoma (e.g., head, neck, or esophagus) , colorectal cancer, leukemia, acute myeloid leukemia, lymphoma, B cell lymphoma, or multiple myeloma) . For example certain methods herein treat cancer by decreasing or reducing or preventing the occurrence, growth, metastasis, or progression of cancer; or treat cancer by decreasing a symptom of cancer. Symptoms of cancer (e.g. liver cancer (e.g. hepatocellular carcinoma) , pancreatic cancer, pancreatic liver metastases, prostate cancer, renal cancer, metastatic cancer, melanoma, castration-resistant prostate cancer, breast cancer, triple negative breast cancer, glioblastoma, ovarian cancer, lung cancer, squamous cell carcinoma (e.g., head, neck, or esophagus) , colorectal cancer, leukemia, acute myeloid leukemia, lymphoma, B cell lymphoma, or multiple myeloma) would be known or may be determined by a person of ordinary skill in the art.

In some embodiments, the cancer is a liver cancer e.g. hepatocellular carcinoma, pancreatic cancer (e.g. pancreatic ductal adenocarcinoma) with pancreatic liver metastases, or a metastatic cancer.

In some embodiments, the cancer may be a pancreatic cancer, pancreatic liver metastases, pancreatic ductal adenocarcinoma (PDAC) , acinar adenocarcinoma, intraductal papillary mucinous neoplasm (IPMN) , pancreatic neuroendocrine tumors (PNETs) , islet cell tumors, insulinoma, glucagonoma, gastrinoma, somatostatinoma, VIPomas, PPomas or pancreatoblastoma.

In some embodiments, the cancer is a TfR expressing cancer. As used herein, a TfR expressing cancer is one where TfR is expressed on the cancer cell's surface. In some embodiments, TfR is expressed on the cancer cell's surface at a level higher than on non-cancerous cell.

In some embodiments, the cancer is a cancer which would benefit from treatment with a CEBPb inhibitor. A cancer which would benefit from treatment with a CEBPb inhibitor as used herein refers to a cancer sensitive to CEBPb inhibition. A cancer sensitive to CEBPb inhibition may exhibit aberrant CEBPb expression or activity, for example increased CEBPb expression or activity, relative to a non-cancerous cell. Without wishing to be bound by theory, as CEBPb suppresses p53 (Ewing et al, 2008) alongside regulating multiple factors critical to the survival and proliferation of cancer cells (Pal et al, 2009) , by suppressing CEBPb the cancer may be effectively treated.

In some embodiments of these aspects, the ribonucleic acids of the invention find use in therapeutic and prophylactic methods in combination (i.e. simultaneously or sequentially) with one or more therapeutic compounds as defined above.

In particular embodiments, the ribonucleic acids are used in combination with an inhibitor of DNA synthesis. The present invention therefore provides nucleic acid compounds for use in a method of medical treatment or prophylaxis, wherein the method of medical treatment or prophylaxis comprises the step of administering the pharmaceutical composition in combination (i.e. simultaneously or sequentially) with an inhibitor of DNA synthesis. The invention also provides the use of nucleic acid compounds in the manufacture of medicaments for treating or preventing a disease or disorder, wherein the medicament is administered simultaneously or sequentially with an inhibitor of DNA synthesis. The invention described herein also provides methods of treating or preventing a disease or disorder, comprising administering to a subject in need thereof a therapeutically or prophylactically effective amount of a nucleic acid compound and an effective amount of an inhibitor of DNA synthesis.

Where components of a combination, e.g. a nucleic acid compound and an inhibitor of DNA synthesis, are administered together administration may be simultaneous administration. Where components of a combination, e.g. a nucleic acid compound and an inhibitor of DNA synthesis, are administered separately, administration may be simultaneous administration or sequential administration.

Compounds of the invention may find particular utility in combination with an inhibitor of DNA synthesis. This should be taken to encompass all drugs, prodrugs, conjugates, and derivatives thereof. Exemplary inhibitors of DNA synthesis are described in Cozzarelli, Annual Review of Biochemistry, Volume 46, 1977, pp 641-668, which is herein incorporated by reference in its entirety. An inhibitor of DNA synthesis may be a DNA polymerase inhibitor or a ribonucleotide reductase inhibitor. An inhibitor of DNA synthesis may be a nucleoside analogue, e.g. a purine or pyrimidine nucleoside analogue. Exemplary compounds include 6-mercaptopurine, 5-fluorouracil (5-FU) , capecitabine, tegafur, acycloguanosine (Acyclovir) , acycloguanosyl 5'-thymidyltriphosphate, 2'-C-cyano-2'-deoxy-1-β-D-arabino-pentofuranosylcytosine (CNDAC) , sapacitabine (a prodrug of CNDAC) , doxifluridine, floxuridine, azacitidine (5-aza-2'-deoxycytidine) , decitabine, or gemcitabine.

In particular embodiments, the inhibitor of DNA synthesis is gemcitabine (2', 2'-difluoro 2'deoxycytidine) or a derivative or prodrug thereof. EP-A-0 184 365 (which is incorporated by reference in its entirety) discloses the synthesis of a range of novel 2'-deoxy, 2',2'-difluoro nucleoside derivatives useful in the treatment of cancer, among these Gemcitabine is found. Derivatives of gemcitabine include gemcitabine esters or amides in which the 3'- and/or 5'-OH group and/or the N<4>-amino group is derivatised with a C18- and/or C20- saturated or mono-unsaturated acyl group, preferably an acyl group selected from oleoyl, elaidoyl, cis-eicosenoyl and trans-eicosenoyl, for example elaidic acid (5') -gemcitabine ester and elaidic acid (N4) -gemcitabine amide, as detailed in EP0986570B1 (which is incorporated by reference in its entirety) .

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

### EXAMPLE 1 - Improving TfR aptamer passive delivery with cleavable SS linkers

In order to test whether cleavable disulphate bridge (SS) linkers improved TfR aptamer delivery of small RNA, a series of constructs were made by fusing the 22nt TfR binding aptamer (SEQ ID NO:1) to: i. a CEBPalpha small activating RNA (saCEBPa, SEQ ID NO:24) ii. CEBPbeta small interfering RNA (siCEBPb, SEQ ID NO:9) , or iii. siTTR (SEQ ID NO:28) .

For the saCEBPa and saCEBPb constructs, variants were produced where the aptamer and small RNAs were linked through a disulphide linker (SS) .

The linker was introduced through the use of a 5'-Thiol C-6 Disulfide Modifier CED phosphoramidite (according to Formula I) during oligonucleotide synthesis through phosphoramidate chemistry.

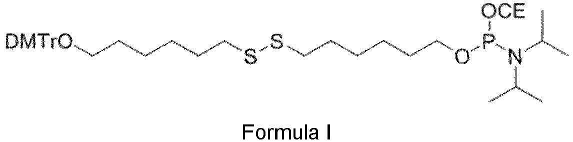

Standard conditions for the synthesis of the oligonucleotides by phosphoramidite chemistry were used, with the exception for the oxidizing reagent, where a 0.02M Iodine in Pyridine-Water, 9:1, v/v, was used instead of 0.05M Iodine. 5'-Thiol C-6 Disulfide Modifier CED phosphoramidite reacts with the 5'-hydroxyl of the support bound oligonucleotide. After the following oxidation, capping and detritylation the 5'-SSR has been introduced.

In order to compare the effects of the SS linker on active and passive delivery, constructs were applied to cells in the presence and absence of transfection agent Lipofectamine^{™} 2000 (L2K, ThermoFisher Scientific).

The effects of the constructs on CEBPA and B mRNA were measured in Panc1 (human pancreatic cancer cell line) cells. Cells were seeded at a density of approximately 50,000 per well in a 24 well plate, in antibiotic free media with no trypsin (Accutase^{®}). Constructs were applied to cells at 10, 500 or 1000 nM concentration, and mRNA levels measured after incubation for 72 hrs. The results are shown in Figure 1.

For the saCEBPA constructs, TfR- saCEBPA 10 nM + L2K showed an increased relative expression of CEBPA, whilst mRNA levels in TfR-SS-saCEBPA 10 nM + L2K remained within 1 standard deviation of untreated samples. CEBPA mRNA levels were lower in all passive delivery experiments (TfR-SS-saCEBPA: 500 nM, 1000 nM; TfR-saCEBPA: 500 nM, 1000 nM) , although this effect was less pronounced for the SS constructs, and least for TfR-SS-saCEBPA 1000 nM, which was within one standard deviation of the control. This suggests that the SS construct upregulated expression in a dose-dependent manner, however it appears that the delivery of saRNA constructs had adverse effects overall.

For the siCEBPB constructs, CEBPB mRNA was severely downregulated in both TfR-siCEBPB and TfR-SS-siCEBPB active delivery experiments (10 nM + L2K) relative to control siRNA against Firefly Luciferace (siFLUC) (10 nM + L2K) , with comparable amounts of downregulation for both the TfR-siCEBPB and TfR-SS-siCEBPB. TfR-SS-siCEBPB reduced CEBPB mRNA in the passive uptake experiment relative to TfR-siTTR (500 nM) . This effect was slightly more pronounced at 500 nM than 1000 nM. This indicates successful delivery of siRNA in both experiments.

### EXAMPLE 2 - In vitro evaluation of TfR aptamer-small RNAs in three cancer cell lines

In order to evaluate the effects of TfR aptamer-small RNAs, experiments were performed in PANC-1, Sup-B15 and CCFR-CEM cell lines.

The TfR-SS-siCEBPB construct as described in Example 1 was selected for these experiments. A TfR aptamer (SEQ ID NO:1) fused via a disulphide bridge (SS) to siRNA for MALATI (TfR-MALATI siR) was generated as a negative control for siCEBPB activity. The siCEBPB (SEQ ID NO:9) without a TfR aptamer delivery system was also included for comparative purposes.

Cells were seeded in plates on Day 0, and the first transfection of TfR aptamer-small RNAs was performed on day 1. The second transfection of TfR aptamer-small RNAs was performed on day 2. Sampling was performed on day 3. Total RNA extraction was performed on samples prior to qRT-PCR quantification of GAPDH, CEBPA, CEBPB, and p21 gene expression. Expression was normalized to GAPDH.

Transfections were performed at 400 nM and 800 nM concentrations.

As can be seen in Figure 2, CEBPbeta gene expression was reduced by the TfR-SS-siCEBPB construct in all three cell types, and this effect was statistically significant. This effect was slightly more pronounced at 800 nM of RNA than for 400 nM. C/EBPA and p21 gene expression was also upregulated in all three tested cells. There was no statistically significant change in CEBPbeta, CEBPalpha or p21 expression in cells treated with TfR-MALATI siR or with siCEBPbeta without a TfR aptamer, at either concentration.

TfR aptamers are therefore capable of and sufficient to effectively deliver CEBPbeta siRNA to a variety of human cancer cells *in vitro.*

### EXAMPLE 3 - TfR-(human) siCEBPB cross-reactivity in primary mouse hepatocytes

In order to verify the effects *in vivo,* it is desirable to test the TFR-siCEBPB constructs in a mouse model system. However, as the mouse CEBPbeta sequence differs from that of humans, it is necessary to first determine the cross reactivity of human siCEBPbeta in mouse.

The human siCEBPbeta sequence was aligned with chromosome 3 of the mouse genome (Accession NC_0344592.1) , in which the CEBPbeta gene is located, using Basic Local Alignment Search Tool (BLAST, https://blast.ncbi.nlm.nih.gov) . This revealed a single base mismatch at position 9 of the human siRNA sequence. To make a human/mouse cross-reactive siRNA, this sequence was substituted in the sense (G to A) and antisense (C to U) strands. The sense and antisense strands of this human/mouse crossreactive (h/m) siCEBPb correspond to SEQ ID NOs: 15 and 16 respectively. TfR-SS-MALATI siRNA was included as an siRNA effector control.

Primary mouse hepatocytes were exposed to TfR-SS-MALATI (100 nM, 500 nM and 1000 nM) or TfR-SS-(h/m) siCEBPb (100 nM, 500 nM and 1000 nM) , or were untreated, and the effects on CEBPB mRNA expression determined as outlined previously.

As can be seen in Figure 3, relative CEBPB mRNA expression was reduced slightly in cells exposed to TfR-SS-(h/m) siCEBPb at 100 nM. This effect was more pronounced for cells exposed to the construct at 500 nM and 1000 nM dosages, although the strongest effect was observed at 500 nM.

The TfR-SS-(h/m) siCEBPb construct therefore is cross-reactive in mouse hepatocytes.

### EXAMPLE 4 - Chemical stabilisation and siCEBPb activity in mouse BNL hepatocyte cells

Whilst "naked" siRNA sequences are functional, modifications to the ribose 2' site can significantly increase stability, activity and efficacy of siRNAs (Hu et al, 2020) .

Previously generated siRNAs, each of which was generated using human or mouse sequences, or the crossreactive human and mouse siRNA described above, were modified through 2'F and 2'-OMe substitutions according to the Enhanced Stabilisation Chemistry (ESC) schema. Modified siRNAs were produced as follows:

**Table 1: ESC modified siRNAs**

| **Name** | **Target (Species)** | **Unmodified ("Naked") sequence** | **ESC modified sequence** |
|---|---|---|---|
| *(h*/*m) siCEBPB* | CEBPB | Sense: SEQ ID NO:7 | Sense: SEQ ID NO:15 |
| | (Human/mouse) | Antisense: SEQ ID NO:8 | Antisense: SEQ ID NO:16 |
| *(m) siCEBPB* | CEBPB (Mouse) | Sense: SEQ ID NO:11 | Sense: SEQ ID NO:17 |
| | | Antisense: SEQ ID NO:12 | Antisense: SEQ ID NO:18 |
| *(h) siCEBPB* | CEBPB (Human) | Sense: SEQ ID NO:9 | Sense: SEQ ID NO:25 |
| | | Antisense: SEQ ID NO:10 | Antisense: SEQ ID NO:26 |

The activity of naked and ESC siRNAs was investigated in mouse BNL hepatocyte cells.

BNL-ME mouse cells were seeded at a density of approximately 20,000 cells per well. The following siRNAs were tested: (m) siCEBPB (Ambion) (SEQ ID NOs:11 and 12) , (m) siCEBPB (Ambs63860) (ESC) (SEQ ID NOs: 17, 18) , (h/m) siCEBPB (SEQ ID NOs:7 and 8) , (h/m) siCEBPB ESC (SEQ ID NOs:15 and 16) , (h) siCEBPB (AP6143-6145) (SEQ ID NOs:9 and 10) , (r) siCEBPB (Hao) (SEQ ID NOs:13 and 14) . Test siRNAs were transfected at concentrations of 1, 5 and 10 nM in the presence of Lipofectamine^{™} 3000 (L3K) . Control treatments (untreated, mock L3K treatment, and siFLUC at 1, 5 and 10 nM) were also performed. The media was changed 24 hours following transfection, and cells lysed after 48hrs. CEBPB mRNA transcript were quantified through qPCR.

As can be seen in Figure 4, the "naked", or non-ESC oligonucleotides affected CEBPB mRNA transcript levels. The largest reductions were seen for (h/m) siCEBPB, where all three concentrations reduced expression below half that of the untreated cells. All tested siRNAs showed a dose-dependent response, with higher concentrations resulting in larger reductions in CEBPB mRNA expression.

Figure 5 compares the ESC modified siRNAs to their naked equivalents. Chemically stabilised (h/m) siCEBPB displayed a similar activity to the unmodified version. As over a short period many stabilisation effects are not apparent, these results demonstrate that the ESC modifications did not reduce activity and, over a longer term, may indeed increase stability.

### EXAMPLE 5 - TfR-siCEBPb in Pancreatic Cancer with Liver Metastasis mouse model

The anti-tumour effects of TfR-siCEBPb was investigated in a mouse model of pancreatic cancer with liver metastasis
Treatment group 1 was administered PBS as a negative control. Treatment group 2 was a TfR-siFLUC construct (SEQ ID NO:32, conjugated to SEQ ID NO:1 ), as described previously. Treatment groups 3 and 4 was administered ESC modified siCEBPb sequences from Example 4 linked to TfR aptamer SEQ ID NO:40, an ESC-modified version of the TfR aptamer laid out in SEQ ID NO:1, via a disulphide bridge as described previously. Group 3 is treated with a ribonucleic acid comprising the human-mouse cross reactive siCEBPb described previously, whilst Group 4 is treated with a ribonucleic acid comprising a mouse siCEBPb. These ribonucleic acids correspond to TfR-SS-siCEBPb seq 1 (SEQ ID NO: 41+50) and TfR-SS-siCEBPb seq 2 (SEQ ID NO:34 and 47) respectively.

The treatment groups and treatment schema are summarised in Table 2.

**Table 2: Treatment groups**

| Group | Treatment | Dose (mg/kg) | Injection route | Injection days |
|---|---|---|---|---|
| 1 | PBS | - | Subcutaneous | 1, 3, 5, 8, 11, 14, 17 |
| 2 | TfR-siFLUC | 15 | Subcutaneous | 1, 3, 5, 8, 11, 14, 17 |
| 3 | TfR-SS-(m) siCEBPb ESC seq 1 (SEQ ID NO: 41+50) | 15 | Subcutaneous | 1, 3, 5, 8, 11, 14, 17 |
| 4 | TfR-SS-(h/m) siCEBPb ESC seq 2 (SEQ ID NO: 34 and 47) | 15 | Subcutaneous | 1, 3, 5, 8, 11, 14, 17 |

Each group consisted of five 6-week-old NOD/SCID mice. Three additional mice were included for baseline.

Fourteen days prior to the first dose, mice underwent tumour challenge by performing intrahepatic tumor implantation by injecting 30 mL of a monocellular suspension (in PBS) containing 1 × 10⁶ PANC-Luc cells into a region in the middle lobe of the livers of 6-week-old female NOD/SCID. The day prior to the first dose, mice underwent bioluminescence tumour imaging (BTL) and were weighed to set the week 0 baseline.

Doses were administered as a 100µL injection on injection days as outlined in Table 2.

Animals for establishing baseline were culled and sampled at day 3.

Blood samples were drawn 1 hour after the first injection on day 1, for PK analysis.

At day 21 (week 3), BTL and body weight were recorded, and blood samples were taken, along with pathological sections of pancreas, tumour and liver for study and cryopreservation. The schema of the experiments is shown as Figure 6.

At week 0, the BTL photon counts and body weights for the five treatment groups at week 0 were comparable, indicating a similar tumour burden in all groups prior to treatment. After 3 weeks (Figure 7A), PBS and TfR-Fluc treated controls show an increase in photon count of around 30%, indicating an increase in tumour size. The increase in photon count is lower for both treatment groups 3 and 4, and this difference is statistically significant, suggesting that both TfR-CEBPb compounds reduced tumour burden. The effect is more marked for treatment group 4. However, no decrease relative to controls is observed for treatment group 5. Figure 7B demonstrates that a body weight gain at 3 weeks in treatment group 4 but not group 3 relative to week 0. Figures 7C and 7D report the effects of the treatment on tumour volume and weight respectively. Tumour volume and weight are comparable between treatment groups 1, 2, and 5. Slightly lower values can be seen in treatment group 3, however statistically significant lower average tumour volumes and weights can be seen for treatment group 4.

The data demonstrate that, whilst both TfR-(m)CEBPb and TfR-(h/m)CEBPb show ability to reduce tumour burden, the effects on IVIS, body weight, tumour volume and weight are all more pronounced for TfR-CEBPb seq 2 than seq-1, suggesting a higher efficacy for this construct. The fact that improvement in tumour burden relative to controls is observed for treatment group 5, corresponding to TfR-CEBPb seq 2 with an albumin tag, possibly suggests that the Tag moiety interfered with cellular uptake. However, albumin tags may never the less be beneficial in improving half-life, for example for intravenous administration, and may be conjugated onto ribonucleic acids in a manner which does not affect uptake.

It is expected that the mice treated with TfR-siCEBPb will have decreased tumor burden at day 21 relative to the PBS and TfR-siFLUC treated mice.

### EXAMPLE 6 - Combination of TfR-siCEBPb with Gemcitabine in Pancreatic Cancer with Liver Metastasis mouse model

The combinatorial anti-tumour effects of TfR-siCEBPb and DNA synthesis inhibitor gemcitabine will be investigated in the mouse model of pancreatic cancer with liver metastasis. The TfR-siCEBPb (ESC) sequences from Example 5 will be used in this study.

The treatment groups will be as follows:

**Table 3: Treatment groups**

| Group | Treatment | Dose (mg/kg) | Injection route | Injection days |
|---|---|---|---|---|
| 1 | PBS | - | Subcutaneous | 1, 3, 8, 11, 14, 17 |
| 2 | TfR-siFLUC | 10 | Subcutaneous | 1, 3, 8, 11, 14, 17 |
| 3 | Gemcitabine | 50 | Intravenous | 1, 3, 8, 11, 14, 17 |
| 4 | TfR-(h/m) siCEBPb seq 2 ESC (SEQ ID NO: 34+47) | 10 | Subcutaneous | 1, 3, 8, 11, 14, 17 |
| 5 | TfR-(h/m) siCEBPb seq 2 ESC (SEQ ID NO: 34+47) | 10 | Subcutaneous | 1, 3, 8, 11, 14, 17 |
| | | 50 | Intravenous | |
| | + gemcitabine | | | |

Fourteen days prior to the first dose, mice will undergo tumour challenge. The day prior to the first dose, mice will undergo bioluminescence tumour imaging (BTL) . Doses will be administered as a 100µL injection on injection days as outlined in Table 3. At day 21, blood samples will be taken, along with pathological sections of pancreas, tumour and liver for study and cryopreservation. The schema of the experiments is shown as Figure 8.

It is expected that the mice treated with TfR-siCEBPb or with gemcitabine will have decreased tumor burden at day 21 relative to the PBS and TfR-siFLUC treated mice. This effect will be even more pronounced for mice treated with a combination of TfR-siCEBPb and gemcitabine.

The known mechanism of action of gemcitabine is inhibition of DNA synthesis (Huang et al, 1991) , and the anti-tumor effects of gemcitabine in pancreatic cancer have been previously determined. As the advanced PDAC is a main mortality of pancreatic cancer, we will investigate the feasibility of gemcitabin in combination with TfR-siCEBPb therapeutics.

### EXAMPLE 7 Combination of TfR-siCEBPb with PD-1 inhibition

The combinatorial anti-tumour effects of TfR-siCEBPb and PD-1 inhibitor pembrolizumab were investigated in the mouse model of pancreatic cancer with liver metastasis. The TfR-siCEBPb (ESC) and TfR-siFLUC sequences from Example 5 were used in this study.

The treatment groups were as follows:

**Table 4: Treatment groups**

| Group | Treatment | Dose (mg/kg) |
|---|---|---|
| 1 | PBS | - |
| 2 (PD1) | Pembrolizumab | 0.25 |
| 3 (P+F) | TfR-siFLUC | 10 |
| | Pembrolizumab | 0.25 |
| 4 (P+C) | TfR-(h/m) siCEBPb seq 2 ESC (SEQ ID NO: 34+47) | 10 |
| | | 0.25 |
| | Pembrolizumab | |

Fourteen days prior to the first dose, mice underwent tumour challenge. The day prior to, and 21 days following the first dose, mice underwent bioluminescence tumour imaging (BTL). The results of this are shown in Figure 10.

All mice created with pembrolizumab (i.e. treatment groups 2-4) all exhibited smaller tumour volumes compared to PBS-treated controls. No significant difference in tumour volume was observed between treatment groups 2 and 3. This was to be expected, as TfR-siFLUC is a control for any therapeutic properties of the TfR aptamer portion. However, interestingly, treatment group 4 (P+C) had the smallest mean tumour size, showing a marked reduction compared to groups treated with pembrolizumab without TfR-SiCEBPb (see Figure 10A). Furthermore, no effect on animal weight was seen for TfR-CEBPb treated mice.

This surprising synergistic effect between TfR-targeted siCEBPb and PD-1 inhibition is expected for further chemotherapeutic agents.

### EXAMPLE 9 - Dose Response for GalNAc-(h/m)CEBPb

Primary mouse hepatocytes were incubated in quadruplicate with increasing dosages of a GaINAc3-(h/m)siCEBPb (XD-30742; SEQ ID NO:44), GaINAc3-FLUC (XD-30743; SEQ ID NO:45), or PBS control, for 48 hours prior to visualising through a branched DNA (bDNA) assay. Dosages were prepared as 5-fold dilutions of an initial 5uM dosage down to a minimum concentration of 15.3 pM. Signal was normalised to GAPDH.

The results of this experiment can be seen in Figure 12. XD-30742 was effective at reducing mRNA (Fig. 11A), and had an IC50 of 0.0069 uM, an IC80 of 0.1637 uM and a maximum KD of 83% (Fig. 11C). No corresponding effect was seen for XC-30732 (Fig. 11) of the PBS control (not shown)

This investigation demonstrates that siCEBPb has an inhibitory effect on CEBPb mRNA, and that this effect is dose-responsive. It also demonstrates that GalNAc is capable of facilitating intracellular transport of siCEBPb into hepatocytes.

### References

A number of publications are cited above in order to more fully describe and disclose the invention and the state of the art to which the invention pertains. Full citations for these references are provided below. The entirety of each of these references is incorporated herein.
1. Ewing SJ, Zhu S, Zhu F, House JS, Smart RC. C/EBPbeta represses p53 to promote cell survival downstream of DNA damage independent of oncogenic Ras and p19(Arf) . Cell Death Differ. 2008 Nov;15(11) :1734-44. doi: 10.1038/cdd.2008.105. Epub 2008 Jul 18. PMID: 18636078; PMCID: PMC3779322.
2. Bo Hu, Liping Zhong, Yuhua Weng, Ling Peng, Yuanyu Huang, Yongxiang Zhao & Xing-Jie Liang. Therapeutic siRNA: state of the art. Signal Transduction and Targeted Therapy (2020) 5:101
3. Pal R, Janz M, Galson DL, Gries M, Li S, Jöhrens K, Anagnostopoulos I, Dörken B, Mapara MY, Borghesi L, Kardava L, Roodman GD, Milcarek C, Lentzsch S. C/EBPbeta regulates transcription factors critical for proliferation and survival of multiple myeloma cells. Blood. 2009 Oct 29;114(18) :3890-8. doi: 10.1182/blood-2009-01-201111. Epub 2009 Aug 28. PMID: 19717648; PMCID: PMC2773489.
4. Huguet, F, Girard, N, Guerche, CS, Hennequin, C, Mornex, F, and Azria, D (2009) . Chemoradiotherapy in the management of locally advanced pancreatic carcinoma: a qualitative systematic review. Journal of clinical oncology: official journal of the American Society of Clinical Oncology 27: 2269-2277.
5. Shaib, YH, Davila, JA, and EI-Serag, HB (2006) . The epidemiology of pancreatic cancer in the United States: changes below the surface. Alimentary pharmacology & therapeutics 24: 87-94.
6. Houg, DS, and Bijlsma, MF (2018) . The hepatic pre-metastatic niche in pancreatic ductal adenocarcinoma. Molecular cancer 17: 95.
7. Mohammad, AA (2018) . Advanced pancreatic cancer: The standard of care and new opportunities. Oncology reviews 12: 370.
8. Paulson, AS, Tran Cao, HS, Tempero, MA, and Lowy, AM (2013) . Therapeutic advances in pancreatic cancer. Gastroenterology 144: 1316-1326.
9. Ma, SJ, Prezzano, KM, Hermann, GM, and Singh, AK (2018) . Dose escalation of radiation therapy with or without induction chemotherapy for unresectable locally advanced pancreatic cancer. Radiation oncology 13: 214.
10. Rosenblum, D, Joshi, N, Tao, W, Karp, JM, and Peer, D (2018) . Progress and challenges towards targeted delivery of cancer therapeutics. Nature communications 9: 1410.
11. Zuker, M (2003) . Mfold web server for nucleic acid folding and hybridization prediction. Nucleic acids research 31: 3406-3415.
12. Zadeh, JN, Steenberg, CD, Bois, JS, Wolfe, BR, Pierce, MB, Khan, AR, et al. (2011) . NUPACK: Analysis and design of nucleic acid systems. Journal of computational chemistry 32: 170-173.
13. Hernandez, FJ, Kalra, N, Wengel, J, and Vester, B (2009) . Aptamers as a model for functional evaluation of LNA and 2'-amino LNA. Bioorganic & medicinal chemistry letters 19: 6585-6587.
14. Soontornworajit, B, Zhou, J, Snipes, MP, Battig, MR, and Wang, Y (2011) . Affinity hydrogels for controlled protein release using nucleic acid aptamers and complementary oligonucleotides. Biomaterials 32: 6839-6849
15. Yoon, S, Huang, KW, Reebye, V, Spalding, D, Przytycka, TM, Wang, Y, et al. (2017) . Aptamer-Drug Conjugates of Active Metabolites of Nucleoside Analogs and Cytotoxic Agents Inhibit Pancreatic Tumor Cell Growth. Molecular therapy Nucleic acids 6: 80-88.
16. Yoon, S, Lee, G, Han, D, Song, JY, Kang, KS, and Lee, YS (2010) . Neutralization of infectivity of porcine circovirus type 2 (PCV2) by capsid-binding 2'F-RNA aptamers. Antiviral research 88: 19-24.
17. Yoon, S, Huang, KW, Reebye, V, Mintz, P, Tien, YW, Lai, HS, etal. (2016). Targeted Delivery of C/EBPalpha -saRNA by Pancreatic Ductal Adenocarcinoma-specific RNA Aptamers Inhibits Tumor Growth In Vivo. Molecular therapy: the journal of the American Society of Gene Therapy 24: 1106-1116.
18. Yamamoto, K, Tateishi, K, Kudo, Y, Sato, T, Yamamoto, S, Miyabayashi, K, et al. (2014) . Loss of histone demethylase KDM6B enhances aggressiveness of pancreatic cancer through downregulation of C/EBPalpha. Carcinogenesis.
19. Kumagai, T, Akagi, T, Desmond, JC, Kawamata, N, Gery, S, Imai, Y, et al. (2009) . Epigenetic regulation and molecular characterization of C/EBPalpha in pancreatic cancer cells. International journal of cancer Journal international du cancer 124: 827-833.
20. Timchenko, NA, Wilde, M, Nakanishi, M, Smith, JR, and Darlington, GJ (1996) . CCAAT/enhancer-binding protein alpha (C/EBP alpha) inhibits cell proliferation through the p21 (WAF-1/CIP-1/SDI-1) protein. Genes & development 10: 804-815.
21. Gragoudas, ES, Adamis, AP, Cunningham, ET, Jr., Feinsod, M, Guyer, DR, and Group, VISiONCT (2004) . Pegaptanib for neovascular age-related macular degeneration. The New England journal of medicine 351: 2805-2816.
22. Loehrer, PJ, Sr., Feng, Y, Cardenes, H, Wagner, L, Brell, JM, Cella, D, et al. (2011) . Gemcitabine alone versus gemcitabine plus radiotherapy in patients with locally advanced pancreatic cancer: an Eastern Cooperative Oncology Group trial. Journal of clinical oncology: official journal of the American Society of Clinical Oncology 29: 4105-4112.
23. Ducreux, M, Seufferlein, T, Van Laethem, JL, Laurent-Puig, P, Smolenschi, C, Malka, D, et al. (2018) . Systemic treatment of pancreatic cancer revisited. Semin Oncol.
24. Blomstrand, H, Scheibling, U, Bratthall, C, Green, H, and Elander, NO (2019) . Real world evidence on gemcitabine and nab-paclitaxel combination chemotherapy in advanced pancreatic cancer. BMC Cancer 19: 40.
25. Akahori, T, Sho, M, Yanagimoto, H, Satoi, S, Nagai, M, Nishiwada, S, et al. (2019) . Phase II Study of the Triple Combination Chemotherapy of SOXIRI (S-1/Oxaliplatin/Irinotecan) in Patients with Unresectable Pancreatic Ductal Adenocarcinoma. Oncologist.
26. Huang, P, Chubb, S, Hertel, LW, Grindey, GB, and Plunkett, W (1991) . Action of 2',2'-difluorodeoxycytidine on DNA synthesis. Cancer research 51: 6110-6117.
27. Tuli, R, Shiao, SL, Nissen, N, Tighiouart, M, Kim, S, Osipov, A, et al. (2019) . A phase 1 study of veliparib, a PARP-1/2 inhibitor, with gemcitabine and radiotherapy in locally advanced pancreatic cancer. EBioMedicine*.*
28. Yi H, Liu S, Kashiwagi Y, et al. Phosphorylated CCAAT/Enhancer Binding Protein β Contributes to Rat HIV-Related Neuropathic Pain: In Vitro and In Vivo Studies. J Neurosci. 2018;38(3):555-574. doi:10.1523/JNEUROSCI.3647-16.2017
29. Nair, Jayaprakash K; Willoughby, Jennifer L. S; Chan, Amy; Charisse, Klaus; Alam, Md. Rowshon; Wang, Qianfan; Hoekstra, Menno; Kandasamy, Pachamuthu; Kel'ln, Alexander V; Milstein, Stuart; Taneja, Nate; o'Shea, Jonathan; Shaikh, Sarfraz; Zhang, Ligang; Van Der Sluis, Ronald J; Jung, Michael E; Akinc, Akin; Hutabarat, Renta; Kuchimanchi, Satya; Fitzgerald, Kevin; Zimmermann, Tracy; Van Berkel, Theo J. C; Maier, Martin A; Rajeev, Kallanthottathil G; Manoharan, Muthiah (2014). "Multivalent N-Acetylgalactosamine-Conjugated siRNA Localizes in Hepatocytes and Elicits Robust RNAi-Mediated Gene Silencing". Journal of the American Chemical Society. 136 (49): 16958-16961. doi:10.1021/ja505986a. PMID 25434769.
30. For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

The following numbered clauses, describing aspects of our proposals, are part of the description:
1. A ribonucleic acid compound, comprising: (i) an aptamer capable of binding to transferrin receptor (TfR), and (ii) a CCAAT/enhancer-binding protein β (C/EBPβ) siRNA.
2. The ribonucleic acid compound of clause 1, wherein the aptamer comprises or consists of an RNA sequence having at least 90% sequence identity to SEQ ID NO:1, wherein said RNA sequence has a length of 29 nucleotides or fewer.
3. The ribonucleic acid compound of clause 2, wherein the RNA sequence has a length of 22 nucleotides of fewer.
4. The ribonucleic acid compound of clause2, wherein the RNA sequence is 22 nucleotides in length.
5. The ribonucleic acid compound according to any previous clause, wherein the RNA sequence has 100% sequence identity to SEQ ID NO:1.
6. The ribonucleic acid compound according to any previous clause, wherein the C/EBPβ siRNA comprises or consists of a sequence selected from SEQ ID NOs: 7, 9, 11 and 13.
7. The ribonucleic acid compound according to any one of clauses 1 to 5, wherein the C/EBPβ siRNA comprises or consists of a sequence selected from SEQ ID NOs: 26 and 27.
8. The ribonucleic acid compound according to any previous clause, wherein the C/EBPβ siRNA comprises one or more nucleotides with a substituted 2' ribose.
9. The ribonucleic acid compound according to any previous clause, wherein the aptamer is capable of binding to TfR on a cell surface.
10. The ribonucleic acid compound according to any previous clause, which is capable of being internalised into a cell.
11. The ribonucleic acid compound according to any previous clause, wherein the aptamer is conjugated to the siRNA via a linker.
12. The ribonucleic acid compound according to clause 11, wherein the linker is a cleavable linker.
13. The ribonucleic acid compound according to clause 12, wherein the cleavable linker is a disulphide bridge.
14. A pharmaceutical composition comprising a ribonucleic acid compound according to any previous clause, optionally comprising a pharmaceutically acceptable excipient.
15. A pharmaceutical composition according to clause 14, further comprising a therapeutic agent, optionally an anticancer agent.
16. A pharmaceutical composition according to clause 15, wherein the therapeutic agent is gemcitabine or a PD-1 axis inhibitor (e.g. pembrolizumab).
17. A ribonucleic acid compound according to any one of clauses 1 to 13, or a pharmaceutical composition according to any one of clauses 14 to 16, for use in a method of treating or preventing a disease or disorder.
18. Use of a ribonucleic acid compound according to any one of clauses 1 to 13, or a pharmaceutical composition according to any one of clauses 14 to 16, in the manufacture of a medicament for use in a method of treating or preventing a disease or disorder.
19. A method of treating or preventing a disease or disorder, the method comprising administering to a subject in need thereof an effective amount of a ribonucleic acid compound according to any one of clauses 1 to 13, or the pharmaceutical composition according to any one of clauses 12 to 16.
20. The ribonucleic acid compound or pharmaceutical composition for use according to clause 17, the use according to clause 18, or the method according to clause 19, wherein the disease or disorder is cancer.
21. The ribonucleic acid compound or pharmaceutical composition for use, the use, or the method according to clause 20, wherein the cancer is selected from pancreatic cancer and liver cancer.
22. The ribonucleic acid compound or pharmaceutical composition for use, the use, or the method according to clause 20 or clause 21, wherein the method further comprises administering an anticancer agent.
23. The ribonucleic acid compound or pharmaceutical composition for use, the use, or the method according to clause 22, wherein the anticancer agent is gemcitabine or a PD-1 axis inhibitor (e.g. pembrolizumab).
24. A method of delivering a CCAAT/enhancer-binding protein β (C/EBPβ) siRNA to a cell, the method comprising:
   i. contacting a cell with the ribonucleic acid compound according to any one of clauses 1 to 13 or a pharmaceutical composition according to clause 14; and
   ii. allowing said ribonucleic acid compound to bind to a transferrin receptor on said cell and pass into said cell thereby delivering said C/EBPβ siRNA into said cell.

## Claims

1. A ribonucleic acid compound comprising: (i) one or more N-Acetylgalactosamine (GaINAc) moieties, and (ii) a CCAAT/enhancer-binding protein beta (C/EBPβ) siRNA.

2. The ribonucleic acid compound of claim 1, wherein the moiety comprises 1, 2, 3, 4, 5, 6, 7, 8, 9, 10 or more GaINAc residues.

3. The ribonucleic acid compound of claim 1 or claim 2, wherein the moiety comprises 3 GalNAc residues.

4. The ribonucleic acid compound according to any one of claims 1 to 3, wherein the C/EBPβ siRNA comprises a sequence selected from SEQ ID NOs: 7, 9, 11, 13, 26 and 27.

5. The ribonucleic acid compound according to any one of claims 1 to 4, wherein the C/EBPβ siRNA comprises one or more nucleotides with a substituted 2' ribose.

6. The ribonucleic acid compound of any one of claims 1 to 5, wherein the one or more GalNAc moieties are conjugated to the siRNA via a linker.

7. The ribonucleic acid compound according to claim 6, wherein the linker is an aminohexyl (NHC6) linker.

8. The ribonucleic acid compound of any one of claims 1 to 7, wherein the ribonucleic acid compound comprises SEQ ID NO:44 or 46.

9. A pharmaceutical composition comprising a ribonucleic acid compound according to any one of claims 1 to 8.

10. The pharmaceutical composition according to claim 9, further comprising a pharmaceutically acceptable excipient.

11. A ribonucleic acid compound according to any one of claims 1 to 8, or a pharmaceutical composition according to claim 9 or claim 10, for use in a method of treating or preventing a disease or disorder.

12. The ribonucleic acid compound or pharmaceutical composition for use according to claim 11, wherein the disease or disorder is selected from a metabolic disease or cancer.

13. The method according to claim 12 wherein the disease or disorder is selected from diabetes or liver cancer.
